# EUROPEAN PATENT SPECIFICATION

(11) **EP 2 215 090 B1**
(45) Date of publication and mention of the grant of the patent: **04.03.2015**
(21) Application number: 08843036.8
(22) Date of filing: 27.10.2008
(51) Int. Cl.: C07D 471/10, C07D 473/34, C07D 498/04, C07D 498/08, C07D 498/10, C07D 519/00, A61K 31/5377, A61K 31/5383, A61K 31/5386, A61K 31/541, A61P 3/00, A61P 5/00, A61P 9/00, A61P 25/00, A61P 35/00

(54) **PURINE DERIVATIVES USEFUL AS PI3 KINASE INHIBITORS**
ALS PI3-KINASE-INHIBITOREN GEEIGNETE PURINDERIVATE
DÉRIVÉS DE PURINE UTILES COMME INHIBITEURS DE PI3 KINASE

(30) Priority: 26.10.2007 GB 0721105; 20.03.2008 GB 0805300; 24.04.2008 GB 0807502
(43) Date of publication of application: 11.08.2010
(73) Proprietor: F. Hoffmann-La Roche AG, 4070 Basel (CH)
(72) Inventor: GOLDSMITH, Paul, Slough, Berkshire SL1 4N1 (GB); HANCOX, Timothy, Colin, Slough, Berkshire SL1 4N1 (GB); HUDSON, Alan, Slough, Bershire SL1 4N1 (GB); PEGG, Neil, Anthony, Slough, Berkshire SL1 4N1 (GB); KULAGOWSKI, Janusz, Josef, Harlow Essex CM19 5TR (GB); NADIN, Alan, John, Harlow, Essex CM19 5TR (GB); PRICE, Stephen, Harlow, Essex CM19 5TR (GB)
(74) Representative: Heiroth, Ulrike Hildegard
(86) International application number: PCT/GB2008/003622
(87) International publication number: WO 2009/053716

(56) References cited:
- EP-A- 1 277 738
- WO-A-2006/046035
- GB-A- 2 431 156

## Description

The present invention relates to indolyl purine compounds and to their use as inhibitors of phosphatidylinositol 3-kinase (PI3K).

Phosphatidylinositol (hereinafter abbreviated as "PI") is one of a number of phospholipids found in cell membranes. In recent years it has become clear that PI plays an important role in intracellular signal transduction. In the late 1980s, a PI3 kinase (PI3K) was found to be an enzyme which phosphorylates the 3-position of the inositol ring of phosphatidylinositol (M.Whitman et al., 1988, Nature, 332, 644-646).

PI3K was originally considered to be a single enzyme, but it has now been clarified that a plurality of subtypes are present in PI3K. Each subtype has its own mechanism for regulating activity. Three major classes of PI3Ks have been identified on the basis of their *in vitro* substrate specificity (B. Vanhaesebroeck et al, 1997, Trends in Biochemical Sciences, 22, 267-272). Substrates for class I PI3Ks are PI, PI 4-phosphate (PI4P) and PI 4,5-biphosphate (PI (4,5)P2). Class I PI3Ks are further divided into two groups, class Ia and class Ib, in terms of their activation mechanism. Class Ia PI3Ks include PI3K p110α, p11β and p110δ subtypes, which transmit signals from tyrosine kinase-coupled receptors. Class Ib PI3K includes a p110γ subtype activated by a G protein-coupled receptor. PI and PI(4)P are known as substrates for class II PI3Ks. Class II PI3Ks include PI3K C2α, C2β and C2γ subtypes, which are characterized by containing C2 domains at the C terminus. The substrate for class III PI3Ks is PI only.

In the PI3K subtypes, the class Ia subtype has been most extensively investigated to date. The three subtypes of class Ia are heterodimers of a catalytic 110 kDa subunit and regulatory subunits of 85 kDa or 55 kDa. The regulatory subunits contain SH2 domains and bind to tyrosine residues phosphorylated by growth factor receptors with a tyrosine kinase activity or oncogene products, thereby inducing the PI3K activity of the p110 catalytic subunit which phosphorylates its lipid substrate. Thus, the class Ia subtypes are considered to be associated with cell proliferation and carcinogenesis, immune disorders and conditions involving inflammation.

WO 01/083456 describes a series of condensed heteroaryl derivatives which have activity as inhibitors of PI3 K and which suppress cancer cell growth.

GB-A-2 431 156 relates to 1-cyclyl-3-substituted-benzenes and-azines as inhibitors of phosphatidylinositol-3-kinase.

It has now been found that a series of novel purine compounds have activity as inhibitors of PI3K. The compounds exhibit selectivity for the p110δ subtype of PI3 kinase, over both other class Ia and class Ib PI3Ks. Accordingly, the present invention provides a compound which is a purine of formula (Ia) or (Ib): 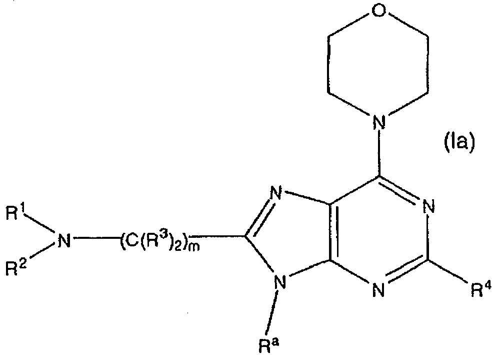 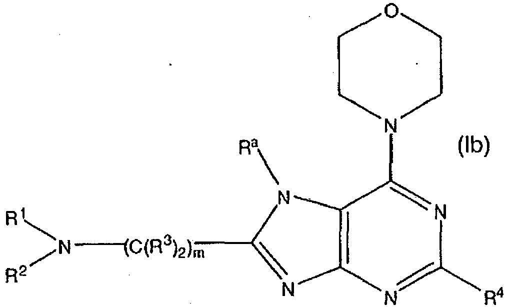 wherein
R¹ and R² form, together with the N atom to which they are attached, a group of the following formula (IIa): 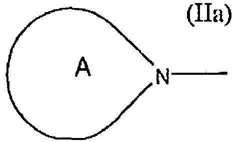
in which A is a group of formula (IIb): 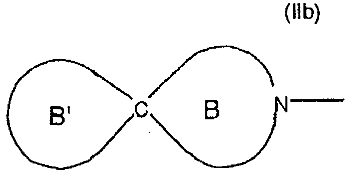 wherein ring B is a 4- to 7-membered saturated N-containing heterocyclic ring which includes 0 or 1 additional heteroatoms selected from N, S and O and ring B' is a 3- to 12- membered saturated carbocyclic ring, a 5- to 7-membered saturated O-containing heterocyclic ring or a 4- to 7-membered saturated N-containing heterocyclic ring as defined above, each of B and B' being unsubstituted or substituted;
m is 0, 1 or 2;
R³ is H or C₁-C₆ alkyl;
R^{a} is selected from R, C(O)OR, C(O)NR₂, halo(C₁-C₆)alkyl, SO₂R, SO₂NR₂, wherein each R is independently H or C₁-C₆ alkyl which is unsubstituted or substituted; and
R⁴ is an indole group which is unsubstituted or substituted;
or a pharmaceutically acceptable salt thereof.

As used herein, the term "fused" indicates that two rings are joined together by a common bond between two adjacent ring atoms. The term "spiro-fused" indicates that two rings are linked through a single common carbon atom. The term "bridgehead" denotes a linking group, of one or more atoms in length, which connects two non-adjacent ring atoms. In each of these three cases a polycyclic (typically a bicyclic) structure is the result.

When any group, ring, group, ring, substituent or moiety defined herein is substituted, it is typically substituted by Z or R⁵ as defined below.

A C₁-C₆ alkyl group is linear or branched. A C₁-C₆ alkyl group is typically a C₁-C₄ alkyl group, for example a methyl, ethyl, n-propyl, i-propyl, n-butyl, sec-butyl or tert-butyl group. A C₁-C₆ alkyl group is unsubstituted or substituted, typically by one or more groups Z or R⁵ as defined below. Typically it is C₁-C₄ alkyl, for example methyl, ethyl, i-propyl, n-propyl, t-butyl, s-butyl or n-butyl.

Z is selected from H, unsubstituted C₁-C₆ alkyl, halo, -OR, -SR, -(C(R⁶)₂)_{q}R,-CH₂OR, -CF₃, -(halo)-C₁-C₆ alkyl, -(C(R⁶)₂)_{q}O-(halo)-C₁-C₆ alkyl, -CO₂R, -(C(R⁶)₂)_{q}CO₂R,-(C(R⁶)₂)_{q}COR, CF₂OH, CH(CF₃)OH, C(CF₃)₂OH, -(CH₂)_{q}OR, -(C(R⁶)₂)_{q}OR, -(CH₂)_{q}NR₂,-(C(R⁶)₂)_{q}NR₂, -C(O)N(R)₂, -(C(R⁶)₂)_{q}CONR₂, -NR₂, -(C(R⁶)₂)_{q}NR₂, -(C(R⁶)₂)_{q}NRC(O)R,-(C(R⁶)₂)_{q}NRC(O)OR, -S(O)ₚR, -S(O)ₚN(R)₂, -(C(R⁶)₂)_{q}S(O)ₚN(R)₂, -OC(O)R,-(C(R⁶)₂)_{q}OC(O)R, -OC(O)N(R)₂, -(C(R⁶)₂)_{q}OC(O)N(R)₂, -NRS(O)ₚR, -(C(R⁶)₂)_{q}NRS(O)ₚR,-NRC(O)N(R)₂, -(C(R⁶)₂)_{q}NRC(O)N(R)₂, CN, -NO₂, =O, a 3- to 12- membered saturated carbocyclic ring which is unsubstituted or substituted, a 5- to 12-membered unsaturated carbocyclic which is unsubstituted or substituted, a 5- to 12-membered unsaturated heterocyclic group which is unsubstituted or substituted and a 4- to 12-membered saturated heterocyclic group which is substituted or unsubstituted, wherein each R is independently selected from H, C₁-C₆ alkyl, C₃-C₁₀ cycloalkyl and a 5- to 12-membered aryl or heteroaryl group, the group being unsubstituted or substituted, or when two groups R are attached to an N atom they form, together with the N atom, a 4- to 7-membered saturated N-containing heterocyclic ring; p is 1 or 2 and q is 0, 1 or 2.

R⁵ is selected from C₁-C₆alkoxy, OR⁶, SR⁶, S(O)ₚR⁶, nitro, CN, halogen, -C(O)R⁶,-CO₂R⁶, -C(O)N(R⁶)₂ and -N(R⁶)₂. R⁶, each of which is the same or different when more than one is present in a given substituent, is selected from H, C₁-C₆ alkyl and C₃-C₁₀ cycloalkyl, and p is 1 or 2.

A halogen or halo group is F, Cl, Br or I. Preferably it is F, Cl or Br. A C₁-C₆ alkyl group substituted by halogen may be denoted by the term "halo-C₁-C₆ alkyl", which means an alkyl group in which one or more hydrogens is replaced by halo. A halo-C₁-C₆ alkyl group preferably contains one, two or three halo groups. A preferred example of such a group is trifluoromethyl.

A C₁-C₆ alkoxy group is linear or branched. It is typically a C₁-C₄ alkoxy group, for example a methoxy, ethoxy, propoxy, i-propoxy, n-propoxy, n-butoxy, sec-butoxy or tert-butoxy group. A C₁-C₆ alkoxy group is unsubstituted or substituted, typically by one or more groups Z or R⁵ as defined above.

A C₃-C₁₀ cycloalkyl group may be, for instance, C₃-C₈ cycloalkyl such as cyclopropyl, cyclobutyl, cyclopentyl, cyclohexyl, or cycloheptyl. Typically it is C₃-C₆ cycloalkyl. A C₃-C₁₀ cycloalkyl group is unsubstituted or substituted, typically by one or more groups Z or R⁵ as defined above.

A 4- to 7-membered saturated N-containing heterocyclic ring typically contains one nitrogen atom and either an additional N atom or an O or S atom, or no additional heteroatoms. It may be, for example, azetidine, pyrrolidine, piperidine, piperazine, morpholine, thiomorpholine or homopiperazine.

A 4- to 7-membered saturated N-containing heterocyclic ring as defined above is unsubstituted or substituted on one or more ring carbon atoms and/or on any additional N atom present in the ring. Examples of suitable substituents include one or more groups Z or R⁵ as defined above, and a C₁-C₆ alkyl group which is unsubstituted or substituted by a group Z or R⁵ as defined above.

Specific examples of a 4- to 7-membered saturated N-containing heterocyclic ring which is substituted as defined above include the following structures:

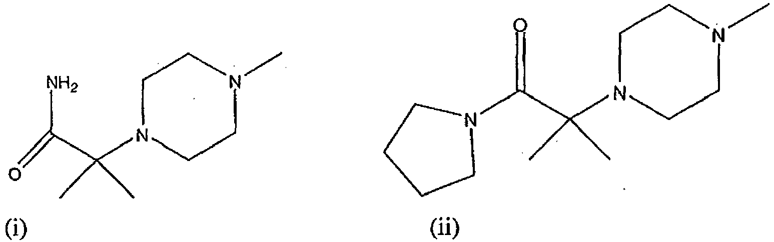

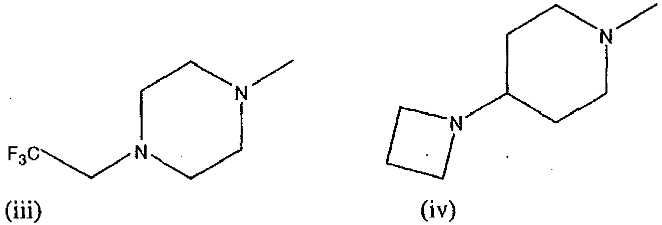

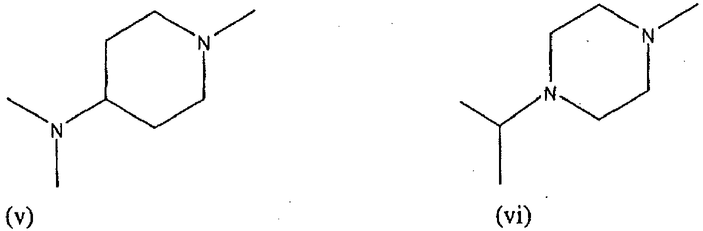

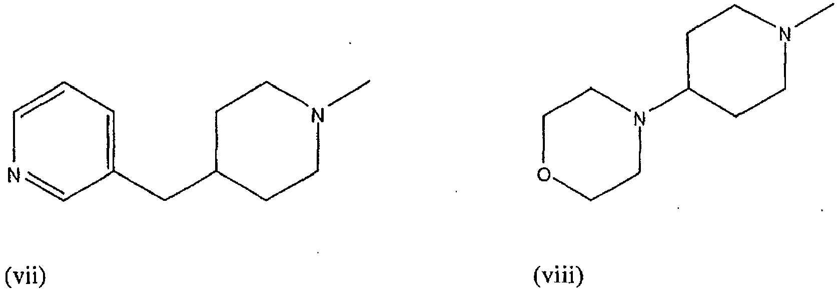

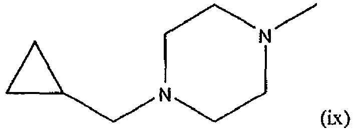

A 5- to 7-membered saturated O-containing heterocyclic ring contains at least one O atom and 0, 1 or 2, typically 0 or 1, additional heteroatoms selected from O, N and S. It is, for instance, tetrahydrofuran, tetrahydropyran, oxetane or morpholine.

A 3- to 12- membered saturated carbocyclic group is a 3-, 4-, 5-, 6-, 7-, 8-, 9-, 10, 11-or 12-membered carbocyclic ring containing only saturated bonds. It is a monocyclic or fused bicyclic ring system. It is, for instance, a 3- to 7-membered saturated carbocyclic ring. Examples include cyclopropane, cyclobutane, cyclopentane, cyclohexane and cycloheptane, and bicyclic ring systems in which two such rings are fused together. Specific examples of a 3- to 12- membered saturated carbocyclic group include the following structures:

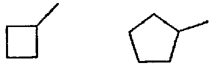

A 5- to 12-membered unsaturated carbocyclic group is a 5-, 6-, 7-, 8-, 9-, 10, 11- or 12-membered carbocyclic ring containing at least one unsaturated bond. It is a monocyclic or fused bicyclic ring system. The group is non-aromatic or aromatic, for instance aryl. Thus, in one embodiment, a 5- to 12-membered unsaturated carbocyclic group is a 5- to 12-membered aryl group. Examples of a 5- to 12-membered unsaturated carbocyclic group include benzene, naphthalene, indane, indene and tetrahydronaphthalene rings, or phenyl, naphthyl, indanyl, indenyl and tetrahydronaphthyl groups. The group is unsubstituted or substituted, typically by one or more groups Z or R⁵ as defined above. Specific examples of a 5- to 12-membered unsaturated carbocyclic group include the following structure:

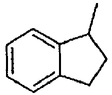

An aryl group is a 5- to 12-membered aromatic carbocyclic group. It is monocyclic or bicyclic. Examples include phenyl and naphthyl groups. The group is unsubstituted or substituted, for instance by a group Z or R⁵ as defined above. Specific examples of an aryl group include the following structures:

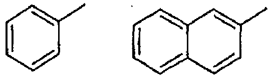

A 5- to 12-membered unsaturated heterocyclic group is a 5-, 6-, 7-, 8-, 9-, 10, 11- or 12- membered heterocyclic ring containing at least one unsaturated bond and at least one heteroatom selected from O, N and S. It is a monocyclic or fused bicyclic ring system. The group is non-aromatic or aromatic, for instance heteroaryl. Thus, in one embodiment a 5- to 12- membered unsaturated heterocyclic group is a 5- to 12-membered heteroaryl group. The 5- to 12-membered unsaturated heterocyclic group may be, for example, furan, thiophene, pyrrole, pyrrolopyrazine, pyrrolopyrimidine, pyrrolopyridine, pyrrolopyridazine, indole, isoindole, pyrazole, pyrazolopyrazine, pyrazolopyrimidine, pyrazolopyridine, pyrazolopyridazine, imidazole, imidazopyrazine, imidazopyrimidine, imidazopyridine, imidazopyridazine, benzimidazole, benzodioxole, benzodioxine, benzoxazole, benzothiophene, benzothiazole, benzofuran, indolizinyl, isoxazole, oxazole, oxadiazole, thiazole, isothiazole, thiadiazole, dihydroimidazole, dihydrobenzofuran, dihydrodioxinopyridine, dihydropyrrolopyridine, dihydrofuranopyridine, dioxolopyridine, pyridine, quinoline, isoquinoline, purine, quinoxaline, tetrahydrobenzofuran, tetrahydroquinoline, tetrahydroisoquinoline, 5,6,7,8-tetrahydro-imidazo[1,5-a]pyrazine, 5,6,7,8-tetrahydro-imidazo[1,2-a]pyrazine, thienopyrazine, pyrimidine, pyridazine, pyrazine, triazine, triazole or tetrazole. The group is unsubstituted or substituted, typically by one or more groups Z or R⁵ as defined above. Specific examples of a 5- to 12- membered unsaturated heterocyclic group include the following structures:

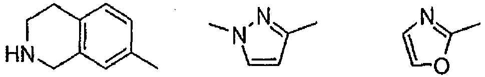

Heteroaryl is a 5- to 12- membered aromatic heterocyclic group which contains 1, 2, 3, or 4 heteroatoms selected from O, N and S. It is monocyclic or bicyclic. Typically it contains one N atom and 0, 1, 2 or 3 additional heteroatoms selected from O, S and N. It may be, for example, a 5- to 7-membered heteroaryl group. Typically it is selected from the heteroaryl groups included in the above list of options for a 5 to 12- membered unsaturated heterocyclic group.

A 4- to 12- membered saturated heterocyclic group is a 4-, 5-, 6-, 7-, 8-, 9-, 10, 11- or 12- membered heterocyclic ring which contains 1, 2, 3, or 4 heteroatoms selected from O, N and S. It is a monocyclic or fused bicyclic ring system. Examples of such heterocyclic rings include, but are not limited to, pyrrolidinyl, tetrahydrofuranyl, tetrahydrothienyl, tetrahydropyranyl, tetrahydrothiopyranyl, piperidino, morpholino, thiomorpholino, thioxanyl, piperazinyl, homopiperazinyl, azetidinyl, oxetanyl, thietanyl, homopiperidinyl, oxepanyl, thiepanyl, oxazepinyl, diazepinyl, thiazepinyl, dithianyl, dithiolanyl, imidazolidinyl, 3-azabicyco[3.1.0]hexanyl, 3-azabicyclo[4.1.0]heptanyl, and azabicyclo[2.2.2]hexanyl. Spiro moieties are also included within the scope of this definition. In one embodiment the saturated 4- to 12-membered saturated heterocyclic group is a 4- to 7-membered saturated N-containing heterocyclic ring as defined above, which is unsubstituted or substituted. The saturated 4- to 12-membered heterocyclic group is unsubstituted or substituted, typically by one or more groups Z or R⁵ as defined above. Specific examples of a 4- to 12-membered saturated heterocyclic group include the following structures:

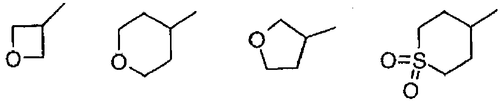

Examples of a 4- to 7-membered saturated N-containing heterocyclic ring which is fused to a second ring as defined above to form a heteropolycyclic ring system include a group selected from azetidine, pyrrolidine, piperidine, piperazine, morpholine, thiomorpholine and homopiperazine, said group being fused to a second ring as defined above. The second ring is typically a 4- to 7-membered saturated N-containing heterocyclic ring as defined above or a 5- to 12-membered unsaturated heterocyclic group. More typically the second ring is a 5-, 6- or 7-membered saturated N-containing heterocyclic ring or a 5- to 7-membered unsaturated heterocyclic ring. Typical examples of the second ring include azetidine, pyrrolidine, piperidine, piperazine, morpholine, thiomorpholine, homopiperazine, pyrrole, imidazole, pyridine, pyridazine, pyrimidine, pyrazine, tetrahydrofuran and tetrahydropyran. Examples of the resulting heteropolycyclic system include octahydro-pyrrolo[1,2-a]pyrazine and octahydro-pyrrolo[3,4-c]pyrrole. Specific examples of the heteropolycyclic system include the following structures:

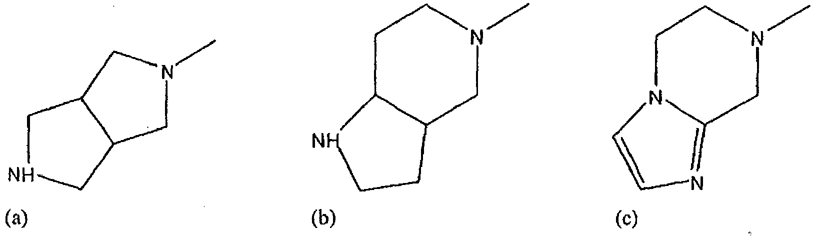

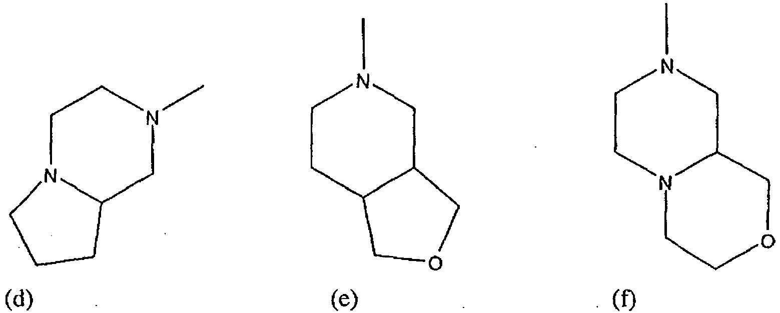

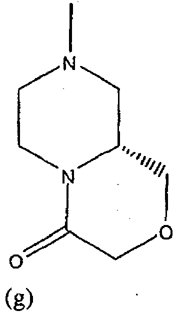

Examples of a 4- to 7-membered saturated N-containing heterocyclic group as defined above which includes a bridgehead group -(CR'₂)ₙ- or -(CR'₂)ᵣ-O-(CR'₂)ₛ- as defined above include 3,8-diaza-bicyclo[3.2.1]octane, 2,5-diaza-bicyclo[2.2.1]heptane, 8-aza-bicyclo[3.2.1]octane, 2-aza-bicyclo[2.2.1]heptane, 3,6-diaza-bicyclo[3.1.1]heptane, 6-aza-bicyclo[3.1.1]heptane, 3,9-diaza-bicyclo[4.2.1]nonane and 3-oxa-7,9-diazabicyclo[3.3.1]nonane.

Specific examples of this group include the following structures:

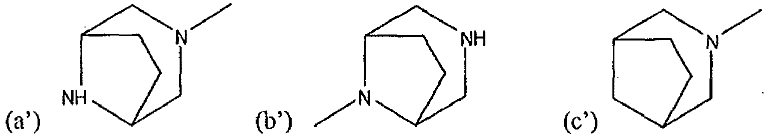

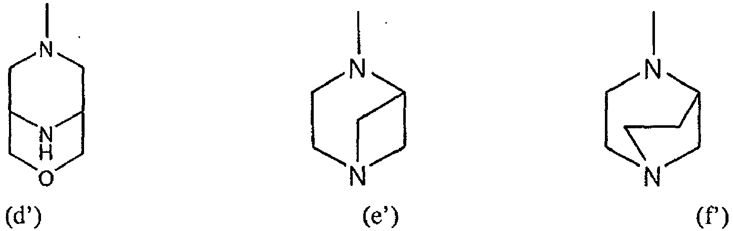

Examples of a group of formula (IIb) as defined above include groups derived from a 4- to 7-membered saturated N-containing heterocyclic group as defined above which is spiro-fused at any available ring carbon atom to a 3 to 12- membered saturated carbocyclic ring, typically to a 3- to 6-membered saturated carbocyclic ring, or to a 4- to 7-membered saturated N-containing heterocyclic group. Examples include a group selected from azetidine, pyrrolidine, piperidine and piperazine which is spiro-fused at a ring carbon atom to a group selected from cyclopropane, cyclobutane, cyclopentane, cyclohexane, azetidine, pyrrolidine, piperidine, piperazine and tetrahydropyran.

The group of formula (IIb) may, for instance, be a group derived from 3,9-diazaspiro[5.5]undecane, 2,7-diazaspiro[3.5]nonane, 2,8-diazaspiro[4.5]decane or 2,7-diazaspiro[4.4]nonane. Specific examples of a group of formula (IIb) include the following structures: 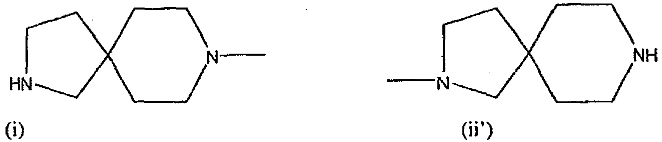 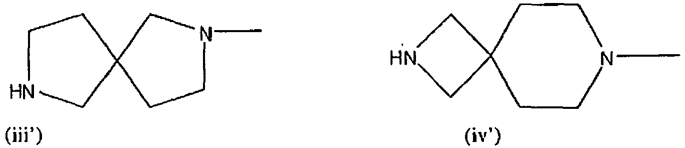 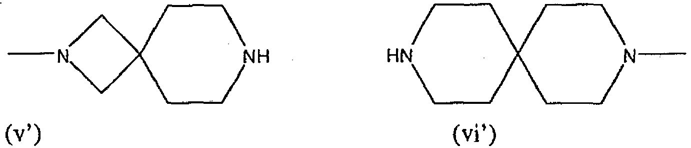 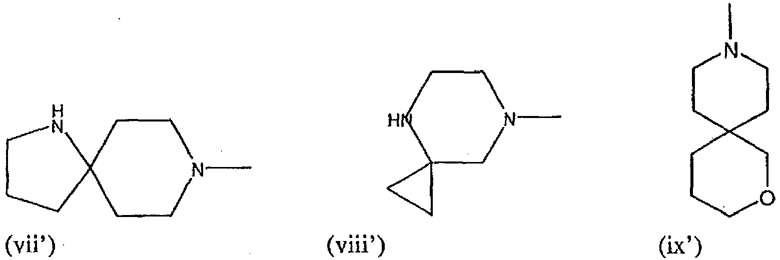 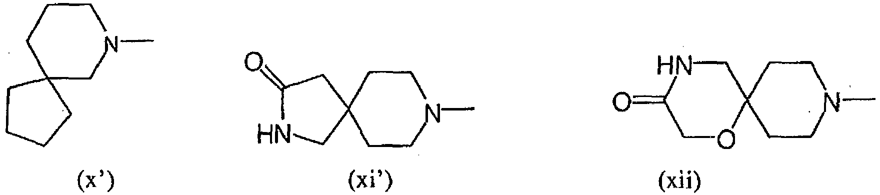 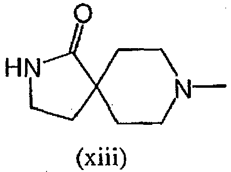 R^{a} is selected from R, -C(O)NR₂, -C(O)OR, halo(C₁-C₆)alky], -SO₂R and -SO₂NR₂, wherein each R is independently H or C₁-C₆ alkyl which is unsubstituted or substituted. When R is C₁-C₆ alkyl which is substituted, it may be substituted by a group Z or R⁵ as defined above. Typically it is substituted by a group selected from CN, halo, -C(O)NR'₂, -NR'C(O)R', -OR', NR'₂, -CF₃, -SO₂R', -SO₂NR'₂, -NR'SO₂R', -OC(O)NR'₂, -NR'C(O)OR' and -NR'C(O)NR'2, wherein each R' is independently H or unsubstituted C₁-C₆ alkyl.

R⁴ is an indolyl group which is unsubstituted or substituted. The indolyl group may be linked to the purine core via any available ring position. It may, for instance, be an indol-4-yl, indol-5-yl, indol-6-yl or indol-7-yl group. Typically it is indol-4-yl or indol-6-yl, more typically an indol-4-yl group.

When substituted, the indolyl may be substituted at one or more available ring positions. Typically it bears a substituent on the benzene moiety of the indole group. For instance, an indol-4-yl group is typically substituted at the 5-, 6- or 7-position, more typically at the 5- or 6-position. An indol-5-yl group is typically substituted at the 4-, 6- or 7- position, more typically at the 4- or 6-position. An indol-6-yl group is typically substituted at the 4-, 5-or 7-position, more typically at the 4- or 5- position. An indol-7-yl group is typically substituted at the 4-, 5- or 6-position, more typically at the 5- or 6-position.

When the indolyl group is substituted it may be substituted by a group Z or R⁵ as defined above. In a typical embodiment the indolyl group is substituted by a group selected from R, -OR, -SR, -S(O)ₚR, CH₂OR, -C(O)R, -CO₂R, CF₃, CF₂OH, CH(CF₃)OH, C(CF₃)₂OH, -(CH₂)_{q}OR, -(CH₂)_{q}NR₂, -C(O)N(R)₂, -NR₂, -N(R)C(O)R, -S(O)ₚN(R)₂, -OC(O)R, OC(O)N(R)₂, -N(R)S(O)ₚR, -NRC(O)N(R)₂, CN, halo, -NO₂ and a 5-membered heteroaryl group containing 1, 2, 3 or 4 heteroatoms selected from O, N and S, wherein R, p and q are as defined above in the definition of Z. In another typical embodiment the indolyl group is substituted by a group selected from C₁ - C₆ alkyl, CN, halo, -C(O)NR₂, halo(C₁-C₆)alkyl such as CF₃, NO₂, OR, SR, NR₂, C(O)R, SOR, SO₂ R, SO₂NR₂, NRC(O)R, CO₂ R and a 5-membered heteroaryl group as defined above. In another more typical embodiment the indolyl group is substituted by a group selected from CN, halo, -C(O)NR₂, halo(C₁-C₆)alkyl such as CF₃, -SO₂R, -SO₂NR₂, and a 5-membered heteroaryl group containing 1, 2, 3 or 4 heteroatoms selected from O, N and S. In the above embodiments R is typically H or C₁-C₆ alkyl.

Typically the substituent on the indolyl group is an electron-withdrawing group. When the substituent is a 5-membered heteroaryl group it may be, for example, furan, thiophene, pyrrole, imidazole, pyrazole, triazole, tetrazole, oxazole, isoxazole, oxadiazole, thiazole, isothiazole, or thiadiazole.

In one embodiment a substituted indolyl group is an indol-4-yl group substituted at the 5- or 6-position, in particular the 5-position, by CN, halo, -C(O)NH₂, -CF₃, -SO₂Me,-SO₂NMe₂ or a 5-membered heteroaryl group as defined above. Typically the indol-4-yl group is substituted at the 5- or 6-position by halo, in particular by F. More typically the indol-4-yl group is substituted at the 5-position by halo, in particular by F.

The parameter m in formulae (Ia) and (Ib) is 0, 1 or 2. Typically m is 1 or 2. More typically m is 1.

In formulae (Ia) and (Ib), a 4- to 12- membered saturated heterocyclic group in the definitions of R¹ and R² may be a 4- to 7-membered saturated N-containing heterocyclic ring which includes 0 or 1 additional heteroatoms selected from N, S and O. A 5- to 12-membered unsaturated heterocyclic group in the definitions of R¹ and R² may be a 5- to 12-membered heteroaryl group. A 5- to 12- membered unsaturated carbocyclic group in the definitions of R¹ and R² may be a 5- to 12- membered aryl group.

In one aspect, the invention provides a compound which is a purine of formula (Ia) or (Ib): 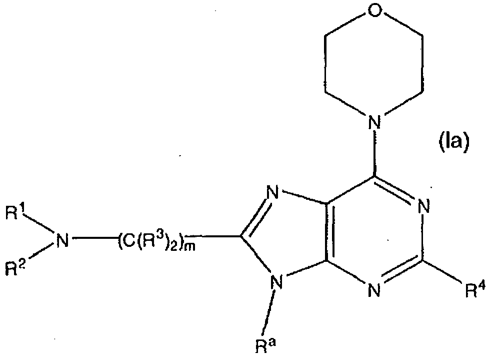 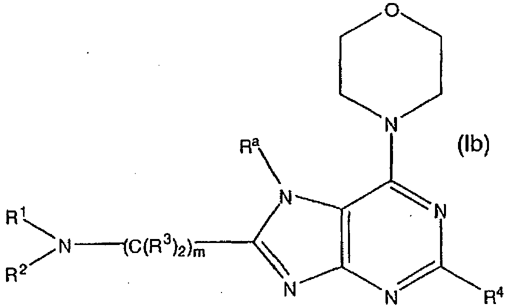 wherein
R¹ and R² form, together with the N atom to which they are attached, a group of the following formula (Ha): 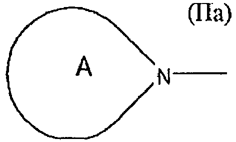
in which A is a group of formula (IIb): 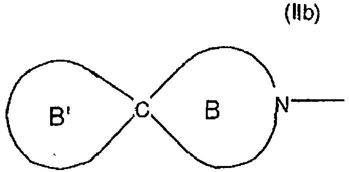 wherein ring B is a 4- to 7-membered saturated N-containing heterocyclic ring which includes 0 or 1 additional heteroatoms selected from N, S and O and ring B' is a 3- to 12- membered saturated carbocyclic ring, a 5- to 7-membered saturated O-containing heterocyclic ring or a 4- to 7-membered saturated N-containing heterocyclic ring as defined above, each of B and B' being unsubstituted or substituted;
m is 0, 1 or 2;
R³ is H or C₁-C₆ alkyl;
R³ is selected from R, C(O)NR₂, halo(C₁-C₆)alkyl, SO₂R, SO₂NR₂, wherein each R is independently H or C₁-C₆ alkyl which is unsubstituted or substituted; and
R⁴ is an indole group which is unsubstituted or substituted;
or a pharmaceutically acceptable salt thereof.

Specific examples of compounds of the invention include those listed in Table 1 below:

**Table 1**

| **Compound No.** | **Structure** | **Name** |
|---|---|---|
| 15 | 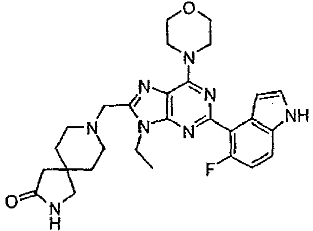 | 8-[9-Ethyl-2-(5-fluoro-1*H*-indol-4-yl)-6-morpholin-4-yl-9*H*-purin-8-ylmethyl]-2,8-diaza-spiro[4.5]decan-3-one |
| 16 | 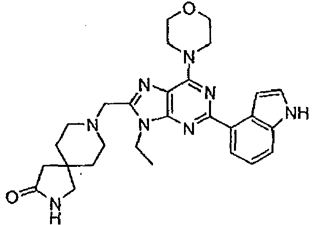 | 8-[9-Ethyl-2-(*H*-indol-4-yl)-6-morpholin-4-yl-9*H*-purin-8-ylmethyl]-2,8-diaza-spiro[4.5]decan-3-one |
| 21 | 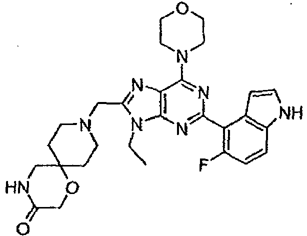 | 9-[9-Ethyl-2-(5-fluoro-1*H*-indol-4-yl)-6-morpholin-4-yl-9*H*-purin-8-ylmethyl]-1-oxa-4,9-diaza-spiro[5.5]undecan-3-one |
| 22 | 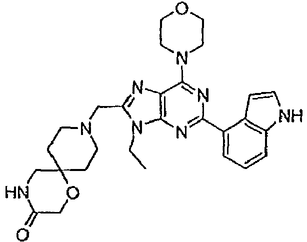 | 9-[9-Ethyl-2-(1*H*-indol-4-yl)-6-morpholin-4-yl-9*H*-purin-8-ylmethyl]-1-oxa-4,9-diaza-spiro[5.5]undecan-3-one |
| 57 | 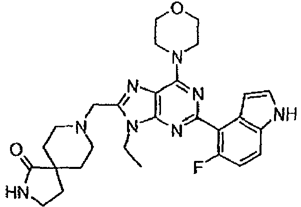 | 8-[9-Ethyl-2-(5-fluoro-1*H*-indol-4-yl)-6-morpholin-4-yl-9*H*-purin-8-ylmethyl]-2,8-diaza-spiro[4.5]decan-1-one |
| 59 | 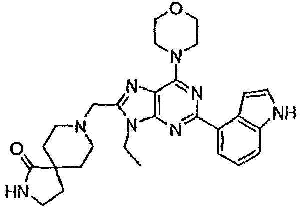 | 8-[9-Ethyl-2-(1*H*-indol-4-yl)-6-morpholin-4-yl-9*H*-purin-8-ylmethyl]-2,8-diaza-spiro[4.5]decan-1-one |

and the pharmaceutically acceptable salts thereof.

A suitable synthetic strategy for producing a purine of formula (Ia) or (Ib) employs the precursor carboxaldehydes of formula (IIIa) and (IIIb):

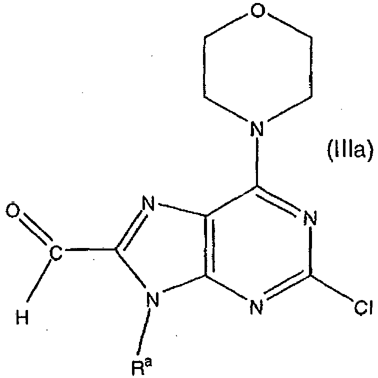

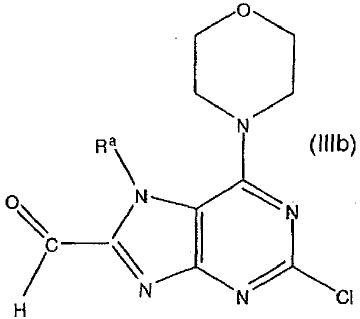

Starting from these precursors the synthesis comprises performing, in either order, a reductive amination and a palladium-mediated (Suzuki-type) cross-coupling reaction.

A compound of the invention may thus be produced by a process which comprises treating a compound of formula (IIIa) or (IIIb): 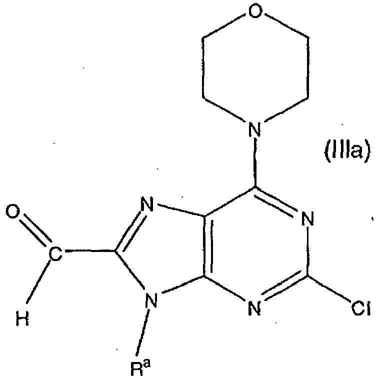 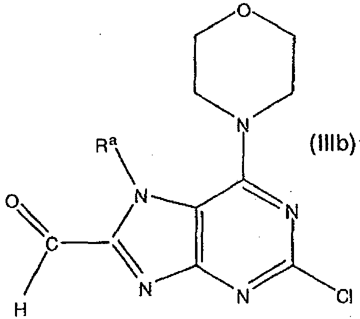 with an amine of formula NHR^{1a}R^{2a} in which R^{1a} and R^{2a} are as defined above for R¹ and R² or R^{1a} and R^{2a} are as defined above for R¹ and R² wherein an N atom is present and is protected by an amine protecting group, in the presence of a suitable reducing agent; and treating the resulting compound of formula (IVa) or (IVb): 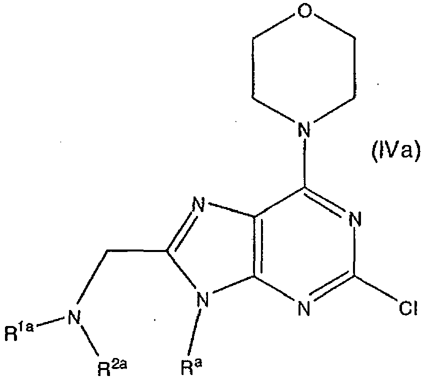 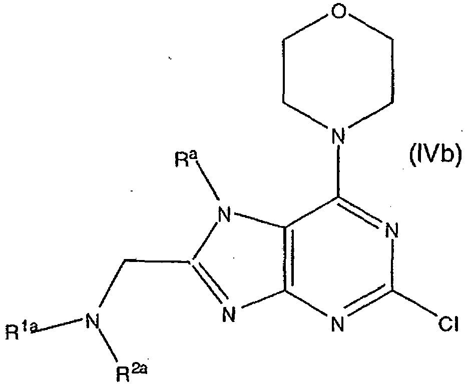 wherein R^{1a} and R^{2a} are as defined above, with a boronic acid or ester thereof of formula R⁴B(OR¹⁵)₂ in which R⁴ is as defined above and each R¹⁵ is H or C₁-C₆ alkyl or the two groups OR¹⁵ form, together with the boron atom to which they are attached, a pinacolato boronate ester group, in the presence of a Pd catalyst; and, if R^{1a} and/or R^{2a} includes an amine protecting group, removing the protecting group. Any suitable amine protecting groups may be used in R^{1a} and/or R^{2a}, for instance a *t*-butoxycarbonyl (BOC) group.

A compound of formula (I) may also be produced by treating a compound of formula (IIIa) or (IIIb): 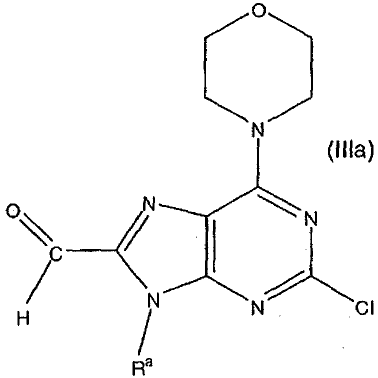 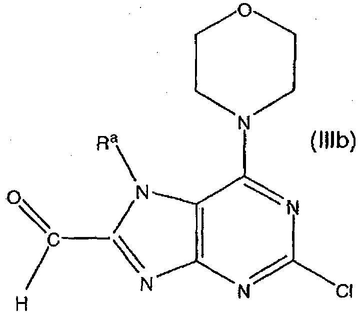 with a boronic acid or ester thereof of formula R⁴B(OR¹⁵)₂ in which R⁴ is as defined above and each R¹⁵ is H or C₁-C₆ alkyl, or the two groups OR¹⁵ form, together with the boron atom to which they are attached, a pinacolato boronate ester group, in the presence of a Pd catalyst; treating the resulting compound of formula (Va) or (Vb): 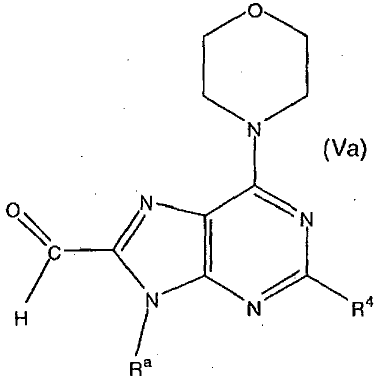 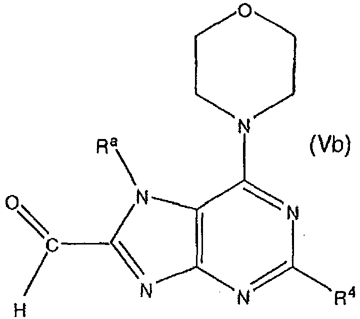 wherein R⁴ is as defined above, with an amine of formula NHR^{1a}R^{2a} in which R^{1a} and R^{2a} are as defined above, in the presence of a suitable reducing agent; and, if R^{1a} and/or R^{2a} includes an amine protecting group, removing the protecting group. In this embodiment of the process the N atom of the indole group R⁴ may, if necessary, be protected before the compound of formula (V) is treated with the amine of formula NHR^{1a}R^{2a}.

Both the reductive amination step and the Pd-mediated cross-coupling step take place under conventional conditions. The palladium catalyst may be any that is typically used for Suzuki-type cross-couplings, such as PdCl₂(PPh₃)₂. The reducing agent in the amination step is typically a borohydride, for instance NaBH(OAc)₃, NaBH₄ or NaCNBH₃, in particular NaBH(OAc)₃.

Intermediate compounds of formulae (IIIa) and (IIIb) are known compounds or may be made by routine synthetic chemical techniques, for instance according to the scheme shown in the Examples which follow or by analogy with such a scheme.

Purines of formula (I) may be converted into pharmaceutically acceptable salts, and salts may be converted into the free compound, by conventional methods. Pharmaceutically acceptable salts include salts of inorganic acids such as hydrochloric acid, hydrobromic acid and sulfuric acid, and salts of organic acids such as acetic acid, oxalic acid, malic acid, methanesulfonic acid, trifluoroacetic acid, benzoic acid, citric acid and tartaric acid. In the case of compounds of the invention bearing a free carboxy substituent, the salts include both the above-mentioned acid addition salts and the salts of sodium, potassium, calcium and ammonum. The latter are prepared by treating the free purine of formula (I), or an acid addition salt thereof, with the corresponding metal base or ammonia.

Compounds of the present invention have been found in biological tests to be inhibitors of PI3 kinase. The compounds are selective for the p110δ isoform, which is a class Ia PI3 kinase, over other class Ia PI3 kinases. They are thus selective for the p110δ isoform over both the p110α isoform and the p110β isoform. In particular they are selective for p110δ over p110β. The compounds are also selective for the p110δ isoform over p110γ, which is a class Ib kinase.

The selectivity exhibited by compounds of the invention for p110δ over other isoforms of PI3 kinase is at least 2-fold. Typically the selectivity is 5-fold, or 10-fold, or 20-fold, or 50-fold, rising to 100-fold or higher in many cases. Thus the compounds may be 2-fold, 5-fold, 10-fold, 20-fold, 50-fold or 100-fold selective for p110δ over p110β. They may also be 2-fold, 5-fold, 10-fold, 20-fold, 50-fold or 100-fold selective for p110δ over p110α or over p110γ.

A compound of the present invention may be used as an inhibitor of PI3 kinase, in particular of a class Ia PI3 kinase. Accordingly, a compound of the present invention can be used to treat a disease or disorder arising from abnormal cell growth, function or behaviour associated with PI3 kinase, in particular the p110δ isoform of PI3 kinase. Examples of such diseases and disorders are discussed by Drees et al in Expert Opin. Ther. Patents (2004) 14(5):703 - 732. These include proliferative disorders such as cancer, immune disorders, cardiovascular disease, viral infection, inflammation, metabolism/endocrine disorders and neurological disorders. Examples of metabolism/endocrine disorders include diabetes and obesity. Examples of cancers which the present compounds can be used to treat include leukaemia, brain tumours, renal cancer, gastric cancer and cancer of the skin, bladder, breast, uterus, lung, colon, prostate, ovary and pancreas.

A compound of the present invention may be used as an inhibitor of PI3 kinase. A human or animal patient suffering from a disease or disorder arising from abnormal cell growth, function or behaviour associated with PI3 kinase, in particular with the p110δ isoform of PI3 kinase such as an immune disorder, cardiovascular disease, viral infection, inflammation, a metabolism/endocrine disorder or a neurological disorder, may thus be treated by a method comprising the administration thereto of a compound of the present invention as defined above. A human or animal patient suffering from cancer may also be treated by a method comprising the administration thereto of a compound of the present invention as defined above. The condition of the patient may thereby be improved or ameliorated.

A compound of the present invention can be administered in a variety of dosage forms, for example orally such as in the form of tablets, capsules, sugar- or film-coated tablets, liquid solutions or suspensions or parenterally, for example intramuscularly, intravenously or subcutaneously. The compound may therefore be given by injection or infusion.

The dosage depends on a variety of factors including the age, weight and condition of the patient and the route of administration. Daily dosages can vary within wide limits and will be adjusted to the individual requirements in each particular case. Typically, however, the dosage adopted for each route of administration when a compound is administered alone to adult humans is 0.0001 to 50 mg/kg, most commonly in the range of 0.001 to 10 mg/kg, body weight, for instance 0.01 to 1 mg/kg. Such a dosage may be given, for example, from 1 to 5 times daily. For intravenous injection a suitable daily dose is from 0.0001 to 1 mg/kg body weight, preferably from 0.0001 to 0.1 mg/kg body weight. A daily dosage can be administered as a single dosage or according to a divided dose schedule.

A compound of the invention is formulated for use as a pharmaceutical or veterinary composition also comprising a pharmaceutically or veterinarily acceptable carrier or diluent. The compositions are typically prepared following conventional methods and are administered in a pharmaceutically or veterinarily suitable form. The compound may be administered in any conventional form, for instance as follows:
A) Orally, for example, as tablets, coated tablets, dragees, troches, lozenges, aqueous or oily suspensions, liquid solutions, dispersible powders or granules, emulsions, hard or soft capsules, or syrups or elixirs. Compositions intended for oral use may be prepared according to any method known in the art for the manufacture of pharmaceutical compositions and such compositions may contain one or more agents selected from the group consisting of sweetening agents, flavouring agents, colouring agents and preserving agents in order to provide pharmaceutically elegant and palatable preparations.

Tablets contain the active ingredient in admixture with non-toxic pharmaceutically acceptable excipients which are suitable for the manufacture of tablets. These excipients may be for example, inert diluents, such as calcium carbonate, sodium carbonate, lactose, dextrose, saccharose, cellulose, corn starch, potato starch, calcium phosphate or sodium phosphate; granulating and disintegrating agents, for example, maize starch, alginic acid, alginates or sodium starch glycolate; binding agents, for example starch, gelatin or acacia; lubricating agents, for example silica, magnesium or calcium stearate, stearic acid or talc; effervescing mixtures; dyestuffs, sweeteners, wetting agents such as lecithin, polysorbates or lauryl sulphate. The tablets may be uncoated or they may be coated by known techniques to delay disintegration and adsorption in the gastrointestinal tract and thereby provide a sustained action over a longer period. For example, a time delay material such as glyceryl monostearate or glyceryl distearate may be employed. Such preparations may be manufactured in a known manner, for example by means of mixing, granulating, tableting, sugar coating or film coating processes.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is present as such, or mixed with water or an oil medium, for example, peanut oil, liquid paraffin, or olive oil.

Aqueous suspensions contain the active materials in admixture with excipients suitable for the manufacture of aqueous suspensions. Such excipients are suspending agents, for example, sodium carboxymethylcellulose, methylcellulose, hydroxypropylmethylcellulose, sodium alginate, polyvinylpyrrolidone gum tragacanth and gum acacia; dispersing or wetting agents may be naturally-occurring phosphatides, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides for example polyoxyethylene sorbitan monooleate.

The said aqueous suspensions may also contain one or more preservatives, for example, ethyl or n-propyl p-hydroxybenzoate, one or more colouring agents, such as sucrose or saccharin.

Oily suspension may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol.

Sweetening agents, such as those set forth above, and flavouring agents may be added to provide a palatable oral preparation. These compositions may be preserved by this addition of an antioxidant such as ascorbic acid. Dispersible powders and granules suitable for preparation of an aqueous suspension by the addition of water provide the active ingredient in admixture with a dispersing or wetting agent, a suspending agent and one or more preservatives. Suitable dispersing or wetting agents and suspending agents are exemplified by those already mentioned above. Additional excipients, for example sweetening, flavouring and colouring agents, may also be present.

The pharmaceutical compositions of the invention may also be in the form of oil-in-water emulsions. The oily phase may be a vegetable oil, for example olive oil or arachis oils, or a mineral oil, for example liquid paraffin or mixtures of these. Suitable emulsifying agents may be naturally-occurring gums, for example gum acacia or gum tragacanth, naturally occuring phosphatides, for example soy bean lecithin, and esters or partial esters derived from fatty acids an hexitol anhydrides, for example sorbitan mono-oleate, and condensation products of the said partial esters with ethylene oxide, for example polyoxyethylene sorbitan monooleate. The emulsion may also contain sweetening and flavouring agents. Syrups and elixirs may be formulated with sweetening agents, for example glycerol, sorbitol or sucrose. In particular a syrup for diabetic patients can contain as carriers only products, for example sorbitol, which do not metabolise to glucose or which only metabolise a very small amount to glucose.

Such formulations may also contain a demulcent, a preservative and flavouring and coloring agents.
B) Parenterally, either subcutaneously, or intravenously, or intramuscularly, or intrasternally, or by infusion techniques, in the form of sterile injectable aqueous or oleaginous suspensions. This suspension may be formulated according to the known art using those suitable dispersing of wetting agents and suspending agents which have been mentioned above. The sterile injectable preparation may also be a sterile injectable solution or suspension in a non-toxic paternally-acceptable diluent or solvent, for example as a solution in 1,3-butane diol.
   Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition fatty acids such as oleic acid find use in the preparation of injectables.
C) By inhalation, in the form of aerosols or solutions for nebulizers.
D) Rectally, in the form of suppositories prepared by mixing the drug with a suitable non-irritating excipient which is solid at ordinary temperature but liquid at the rectal temperature and will therefore melt in the rectum to release the drug. Such materials are cocoa butter and poly-ethylene glycols.
E) Topically, in the form of creams, ointments, jellies, collyriums, solutions or suspesions.

The invention will be further described in the Examples which follow:

### EXAMPLES

### General Synthetic Procedure

The following general scheme depicts the synthetic approach referred to in the Reference Examples and Examples which follow:

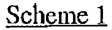

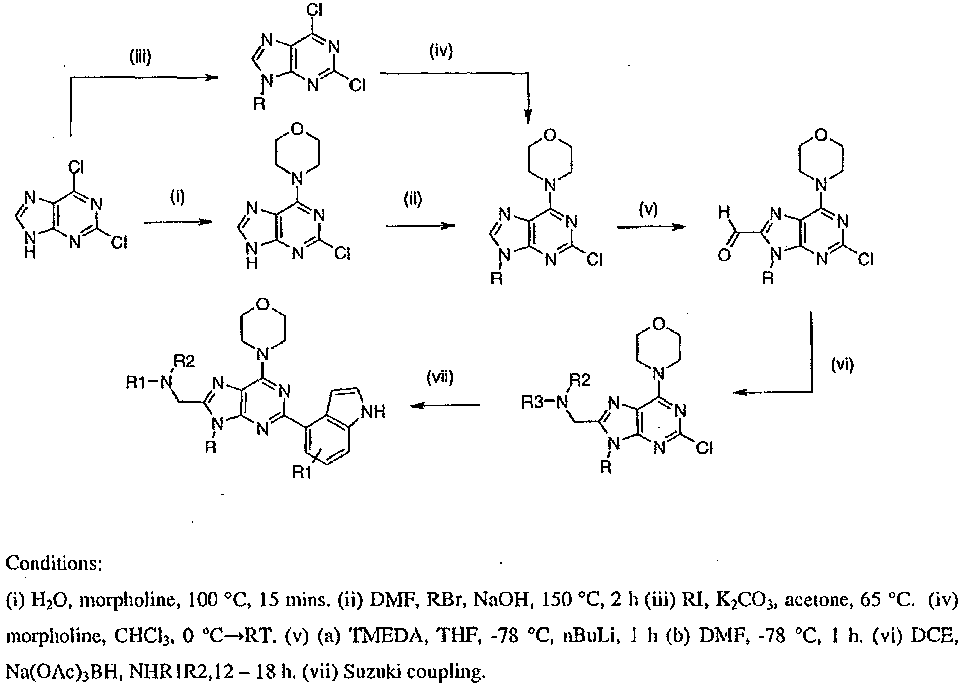

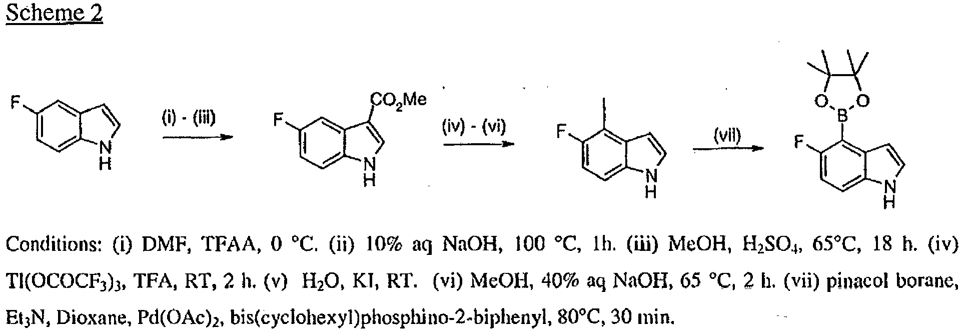

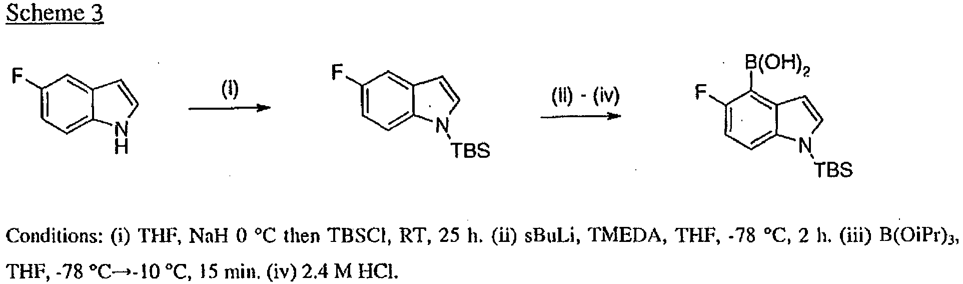

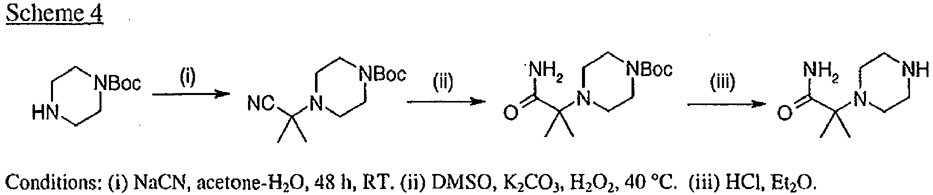

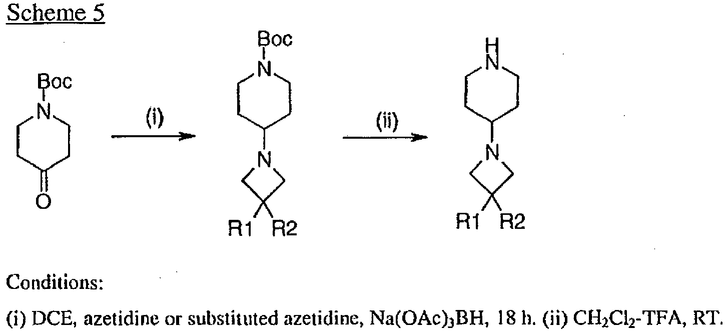

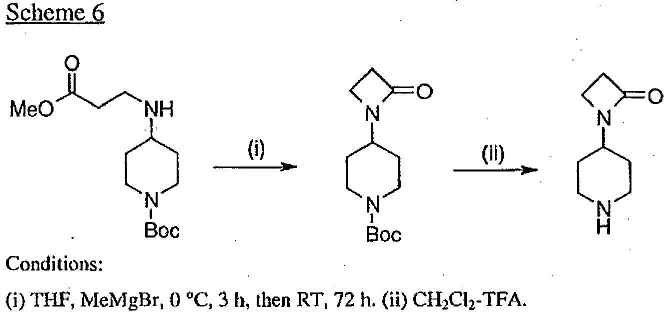

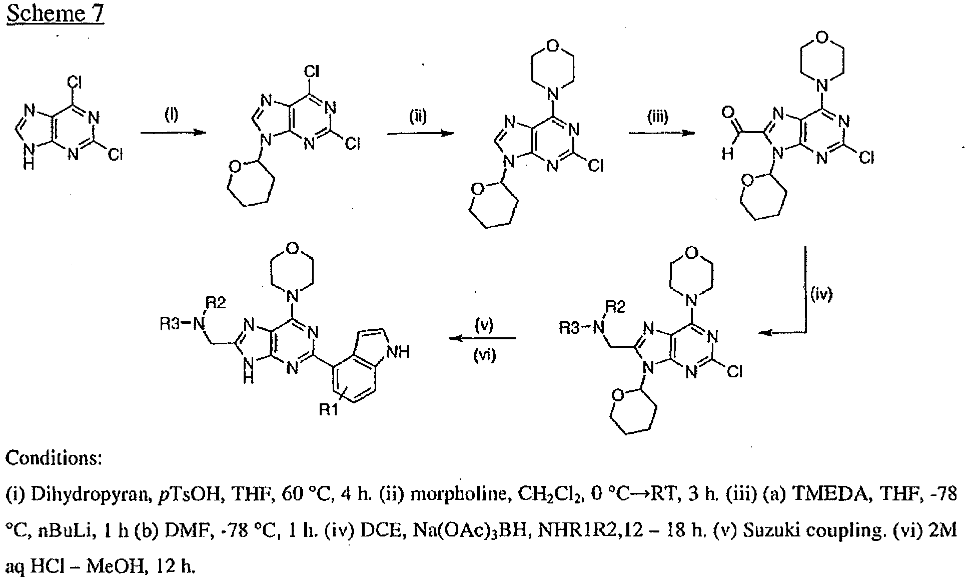

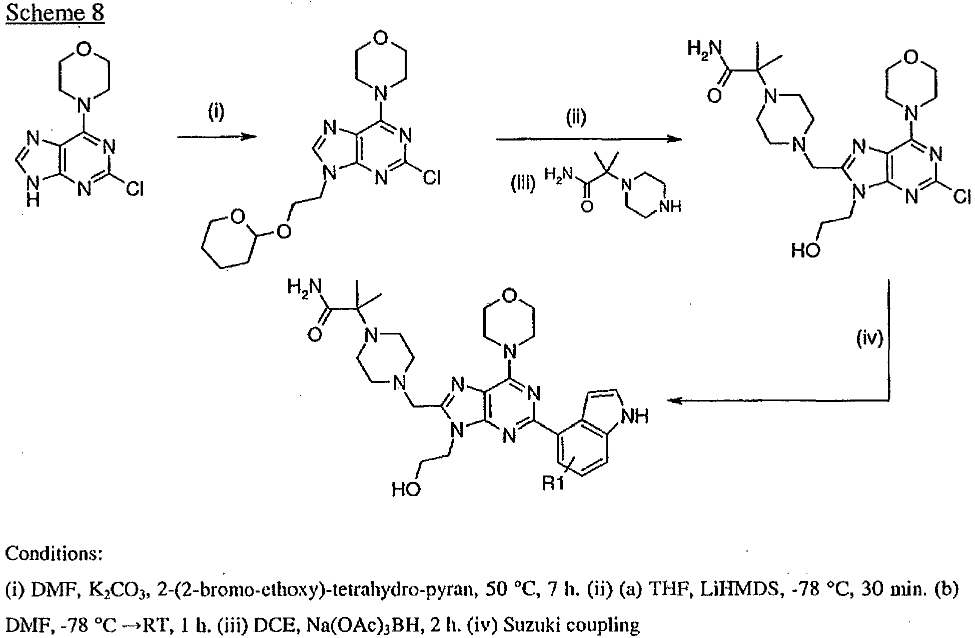

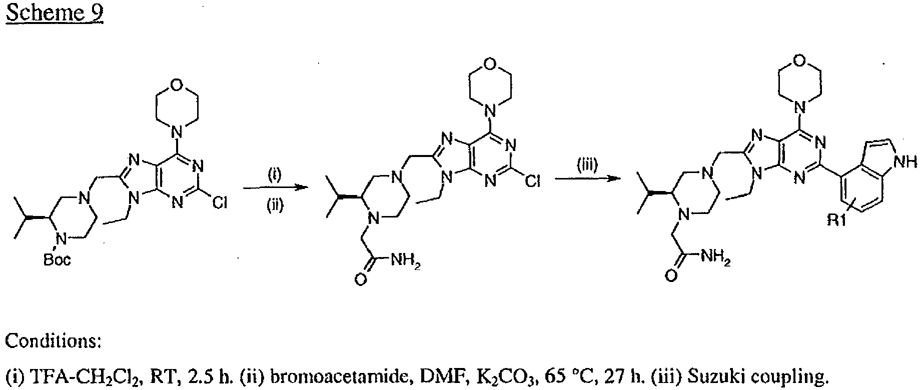

### General Experimental Details:

### NMR spectroscopy

NMR spectra were obtained on a Varian Unity Inova 400 spectrometer with a 5 mm inverse detection triple resonance probe operating at 400MHz or on a Bruker Avance DRX 400 spectrometer with a 5 mm inverse detection triple resonance TXI probe operating at 400 MHz or on a Bruker Avance DPX 300 spectrometer with a standard 5mm dual frequency probe operating at 300 MHz. Shifts are given in ppm relative to tetramethylsilane.

### Purification by column chromatography

Compounds purified by column chromatography were purified using silica gel or Isolute® cartridge or Redisep® cartridge, eluting with gradients from 100-0 to 0-100 % of cyclohexane/EtOAc, or from 100-0 to 0-100 % pentane/EtOAc or from 100-0 to 70-30 % DCM/MeOH (with or without the addition of NH₃ 0.1 %). 'Silica gel' refers to silica gel for chromatography, 0.035 to 0.070 mm (220 to 440 mesh) (e.g. Fluka silica gel 60), and an applied pressure of nitrogen up to 10 p.s.i accelerated column elution. Where thin layer chromatography (TLC) has been used, it refers to silica gel TLC using plates, typically 3 × 6 cm silica gel on aluminium foil plates with a fluorescent indicator (254 nm), (e.g. Fluka 60778).

### Purification by preparative HPLC:

Compounds purified by preparative HPLC were purified using a C18-reverse-phase column (100 × 22.5 mm i.d Genesis column with 7 µm particle size, UV detection at 230 or 254 nm, flow 5-15 mL/min), or a Phenyl-Hexyl column (250 x 21.2 mm i.d. Gemini column with 5 µm particle size, UV detection at 230 or 254 nm, flow 5-20 mL/min), eluting with gradients from 100-0 % to 0-100 % water/acetonitrile or water/MeOH containing 0.1 % TFA or water/acetonitrile containing 0.1 % formic acid. The free base was liberated by partitioning between EtOAc and a sat. solution of sodium bicarbonate. The organic layer was dried (MgSO₄) and concentrated *in vacuo.* Alternatively, the free base was liberated by passing through an Isolute® SCX-2 cartridge, eluting with NH₃ in methanol.

### Microwave Reactions:

Microwave experiments were carried out using a Smith Synthesiser or a Biotage Initiator™, which uses a single-mode resonator and dynamic field tuning, both of which give reproducibility and control. Temperatures from 40-250°C can be achieved and pressures of up to 20bar can be reached.
All solvents and commercial reagents were used as received. Non-commercially available reagents/reactants were prepared according to procedures described in the literature.

### Abbreviations used in the experimental section:

aq. = aqueous
BOC = *t*-Butoxycarbonyl
bs = broad singlet (NMR)
Cs₂CO₃ = cesium carbonate
d = doublet (NMR)
DCE = 1,2-dichloroethane
DCM = dichloromethane
DIPEA = diisopropylethylamine
DMA = dimethylacetamide
DMAP = dimethylaminopyridine
DMF = dimethylformamide
DMSO = dimethylsulfoxide
eq. = equivalents
EtOAc = ethyl acetate
EtOH = ethanol
h = hour(s)
HATU = *O*-(7-Azabenzotriazol-1-yl)-*N*,*N*,*N'*,*N'*-tetramethyluronium hexafluorophosphate
HCl = hydrochloric acid
H₂O = water
HPLC = high pressure liquid chromatography
IMS = industrial methylated spirit
*i*PrOH = isopropanol
LCMS = liquid chromatography mass spectrometry
LiHMDS = lithium bis(trimethylsilyl)amide
M = molar
m = multiplet (NMR)
MeOH = methanol
mg = milligram
MgSO₄ = magnesium sulphate
min = minute(s)
mL = millilitre
Na₂CO₃ = sodium carbonate
NaHCO₃ = sodium hydrogen carbonate
NaOH = sodium hydroxide
Na₂SO₄ = sodium sulfate
NH₄OH = ammonium hydroxide solution
NMR = nuclear magnetic resonance
q = quartet (NMR)
Rt = retention time
RT = room temperature
t = triplet (NMR)
TBAF = tetrabutylammonium fluoride
TBDMS = *tert*-butyldimethylsilyl
TFA = trifluoroacetic acid
THF = tetrahydrofuran
TLC = thin layer chromatography
TMEDA = *N*,*N'*,*N'*,*N'*-tetramethylethylenediamine

### General synthetic strategies

### Reference Example 1 General methods for Suzuki coupling

The Suzuki coupling reaction depicted generally in scheme 10 below were performed using one of the methods set out below.

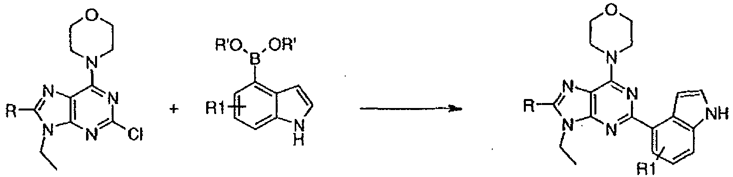

### Method A:

A mixture of the appropriate 2-chloropurine (1 eq.), Na₂CO₃ (2 eq.), the appropriate indole boronate ester (1.5 eq.) and bis(triphenylphosphine)palladium (II) chloride (0.1 eq.) in dioxane/water (2:1) was heated at 125 °C for 20 - 50 min in a microwave reactor. The resulting mixture was diluted with water then extracted with ethyl acetate. The combined organic extracts were dried (Na₂SO₄) filtered and concentrated *in vacuo* then purified by either preparative HPLC or column chromatography to give the desired product. Alternatively, the reaction mixture was loaded onto an Isolute® SCX-2 cartridge which was washed with MeOH then eluted with 2 M NH₃ in MeOH. The resulting residue was then purified by either preparative HPLC or column chromatography to give the desired product.

### Method B

A mixture of the appropriate 2-chloropurine (1 eq.), Cs₂CO₃ (1.5 eq.), the appropriate indole boronate ester (1.2 eq.) and tetrakis(triphenylphosphine)palladium (0.05 eq.) in dioxane/water (3:1) was heated at 125 °C - 140 °C, for 10 - 60 min in a microwave reactor. The resulting mixture was diluted with water then extracted with ethyl acetate. The combined organic extracts were dried (MgSO₄), filtered and concentrated *in vacuo* then purified by either preparative HPLC or column chromatography to give the desired product. Alternatively, the reaction mixture was loaded onto an Isolute® SCX-2 cartridge, washed with MeOH then eluted with 2 M NH₃ in MeOH. The resulting residue was then purified by either preparative HPLC or column chromatography to give the desired product.

### Method C

A mixture of the appropriate 2-chloropurine (1 eq.), Cs₂CO₃ (1.5 eq.), the appropriate indole boronic acid (1.2 eq.) and tetrakis(triphenylphosphine)palladium (0.05 eq.) in dioxane/water (3: 1) was heated at 125 °C - 140 °C, for 10 - 60 min in a microwave reactor. The resulting mixture was diluted with water then extracted with ethyl acetate. The combined organic extracts were dried (MgSO₄), filtered and concentrated *in vacuo* then purified by either preparative HPLC or column chromatography to give the desired product. Alternatively, the reaction mixture was loaded onto an Isolute® SCX-2 cartridge, washed with MeOH then eluted with 2 M NH₃ in MeOH. The resulting residue was then purified by either preparative HPLC or column chromatography to give the desired product.

### Method D

A mixture of the appropriate 2-chloropurine (1 eq.), Cs₂CO₃ (1.5 eq.), the appropriate indole boronic acid (1.2 eq.) and tetrakis(triphenylphosphine)palladium (0.05 eq.) in acetonitrile/water (3:1) was heated at 125 °C - 140 °C , for 10 - 60 min in a microwave reactor. The resulting mixture was diluted with water then extracted with ethyl acetate. The combined organic extracts were dried (MgSO₄) filtered and concentrated *in vacuo* then purified by either preparative HPLC or column chromatography to give the desired product. Alternatively, the reaction mixture was loaded onto an Isolute® SCX-2 cartridge, washed with MeOH then eluted with 2 M NH₃ in MeOH. The resulting residue was then purified by either preparative HPLC or column chromatography to give the desired product.

### Method E

A mixture of the appropriate 2-chloropurine (1 eq.), Cs₂CO₃ (1.5 eq.), the appropriate indole boronate ester (1.2 eq.) and tetrakis(triphenylphosphine)palladium (0.05 eq.) in acetonitrile/water (3:1) was heated at 140 °C, for 10 - 30 min in a microwave reactor. The resulting mixture was diluted with water then extracted with ethyl acetate. The combined organic extracts were dried (MgSO₄), filtered and concentrated *in vacuo* then purified by either preparative HPLC or column chromatography to give the desired product. Alternatively, the reaction mixture was loaded onto an Isolute® SCX-2 cartridge, washed with MeOH then eluted with 2 M NH₃ in MeOH. The resulting residue was then purified by either preparative HPLC or column chromatography to give the desired product.

### Method F

A mixture of the appropriate 2-chloropurine (1 eq.), Na₂CO₃ (1.5 eq.), the appropriate indole boronate ester (1.2 eq.) and tetrakis(triphenylphosphine)palladium (0.1 eq.) in acetonitrile/water (2:1) was heated at 140 °C, for 10 - 30 min in a microwave reactor. The resulting mixture was diluted with water then extracted with ethyl acetate. The combined organic extracts were dried (MgSO₄), filtered and concentrated *in vacuo* then purified by either preparative HPLC or column chromatography to give the desired product. Alternatively, the reaction mixture was loaded onto an Isolute® SCX-2 cartridge, washed with MeOH then eluted with 2 M NH₃ in MeOH. The resulting residue was then purified by either preparative HPLC or column chromatography to give the desired product.

### Method G

A mixture of the appropriate 2-chloropurine (1 eq.), Na₂CO₃ (1.5 eq.), the appropriate indole boronate acid (1.2 eq.) and tetrakis(triphenylphosphine)palladium or bis(triphenylphosphine)palladium (II) chloride (0.5→0.1 eq.) in acetonitrile/water (2:1) was heated at 140 °C, for 10 - 30 min in a microwave reactor. The resulting mixture was diluted with water then extracted with ethyl acetate. The combined organic extracts were dried (MgSO₄), filtered and concentrated *in vacuo* then purified by either preparative HPLC or column chromatography to give the desired product. Alternatively, the reaction mixture was loaded onto an Isolute® SCX-2 cartridge, washed with MeOH then eluted with 2 M NH₃ in MeOH. The resulting residue was then purified by either preparative HPLC or column chromatography to give the desired product.

### Method H

A mixture of the appropriate 2-chloropurine (1 eq.), Cs₂CO₃ (1.5 eq.), indole boronate acid (1.2 eq.) and bis(triphenylphosphine)palladium (II) chloride (0.1 eq.) in dioxane/water (2:1) was heated at 140 °C, for 10 - 60 min in a microwave reactor. The resulting mixture was diluted with water then extracted with ethyl acetate. The combined organic extracts were dried (MgSO₄), filtered and concentrated *in vacuo* then purified by either preparative HPLC or column chromatography to give the desired product. Alternatively, the reaction mixture was loaded onto an Isolute® SCX-2 cartridge, washed with MeOH then eluted with 2 M NH₃ in MeOH. The resulting residue was then purified by either preparative HPLC or column chromatography to give the desired product.

### Method I

A mixture of the appropriate 2-chloropurine (1 eq.), Cs₂CO₃ (1.5 eq.), indole boronate ester (1.2 eq.) and bis(triphenylphosphine)palladium (II) chloride (0.1 eq.) in dioxane/water (2:1) was heated at 140 °C, for 10 - 60 min in a microwave reactor. The resulting mixture was diluted with water then extracted with ethyl acetate. The combined organic extracts were dried (MgSO₄), filtered and concentrated *in vacuo* then purified by either preparative HPLC or column chromatography to give the desired product. Alternatively, the reaction mixture was loaded onto an Isolute® SCX-2 cartridge, the cartridge was then washed with MeOH then eluted with 2 M NH₃ in MeOH. The resulting residue was then purified by either preparative HPLC or column chromatography to give the desired product.

### Reference Example 2 General method for BOC-deprotection

To a solution of the relevant BOC-protected aminopurine in DCM was added TFA and the resulting solution was stirred at RT for 30-180 min. The resulting mixture was diluted with water then extracted with DCM. The combined organic extracts were dried (MgSO₄ or Na₂SO₄), filtered and concentrated *in vacuo,* then purified by either preparative HPLC or column chromatography to give the desired product. Alternatively, the reaction mixture was loaded onto an Isolute® SCX-2 cartridge, washed with MeOH then eluted with 2 M NH₃ in MeOH. The resulting residue was then purified by either preparative HPLC or column chromatography to give the desired product.

### Reference Example 3 General method for TBDMS-deprotection

To a solution of the relevant TBDMS-protected 5-fluoro-1*H*-indol-4-yl-purine in THF was added TBAF and the resulting solution was stirred at RT for 30 min, then concentrated *in vacuo.* The resultant residue was purified by either preparative HPLC or column chromatography to give the desired product.

### Preparation of Intermediates

### Reference Example 4

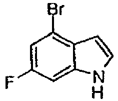

### 4-Bromo-6-fluoro-1H-indole

To a solution of 1-bromo-5-fluoro-2-methyl-3-nitro-benzene (7.49 g, 31.8 mmol) in dioxane (40 mL) was added DMF-DMA (21.0 mL, 158 mmol) and pyrrolidine (2.6 mL, 31.1 mmol). The reaction mixture was heated at 100 °C. The mixture was cooled to RT and evaporated to dryness to give 1-[2-(2-bromo-4-fluoro-6-nitro-phenyl)-1-methyl vinyl]-pyrrolidine as a dark red residue (10.0 g, theoretical yield). To a suspension of the pyrrolidine (10.0 g, 31.7 mmol) and Raney®-Nickel (suspension in H₂O, 15 mL) in MeOH:THF (1:1, 150 mL) was added hydrazine monohydrate (2.3 mL, 47.4 mmol) at 0 °C and the mixture stirred at RT for 5 hours. The reaction mixture was then filtered through Celite and the filter cake washed with EtOAc. The filtrate was evaporated to dryness and the resulting residue to give the title compound as pale oil (2.57 g, 37 %).
NMR δ_{H} (300 MHz, CDCl₃) 6.57 (apparent t, J = 2.7, 1H), 7.04 (dd, J = 2.1. 9.1, 1H), 7.12 (dd, J = 2.1, 9.1, 1H), 7.20-7.25 (m, 1H) and 8.25 (s, 1H).

### Reference Example 5

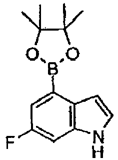

### 6-Fluoro-4-(4,4,5,5-tetramethyl-[1,3,2]dioxaborolan-2-yl)-1H-indole

To a stirring mixture of 4-bromo-6-fluoro-1H-indole (6.76 g, 31.5 mmol), bis(pinacolato)diboron (12.8 g, 94.7 mmol) and potassium acetate (9.3 g, 94.7 mmol) in methyl sulfoxide (100 mL) was added 1,1'-bis(diphenylphosphine)ferrocene-dichloropalladium (1.29 g, 5 mol %). The reaction mixture was flushed out with nitrogen and heated to 100 °C for 16 hours. The mixture was partitioned between ethyl acetate and water and the organic layer washed with brine, dried over MgSO₄ and evaporated down. The crude product was purified by column chromatography followed by triturating to yield the title compound (7.87 g).
δ_{H} (400MHz, CDCl₃) 1.41 (s, 12H), 7.04 (m, 1H), 7.16 (dd, 1H), 7.26 (t, 1H), 7.40 (dd, 1H), 8.14 (br s, 1H).

### Reference Example 6

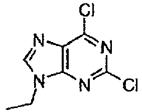

### 2,6-Dichloro-9-ethyl-9H-purine

To a stirred suspension of 2,6-dichloropurine (1.89 g; 10 mmol) and K₂CO₃ (1.73 g; 12.5 mmol) in acetone (25 mL) was added iodoethane (1.0 mL; 12.5 mmol) and the resulting mixture was heated at reflux temperature for 5 h. The reaction mixture was cooled, filtered, the filtrate concentrated and the residue purified by flash chromatography (100 % EtOAc) to obtain the title compound as a colourless crystalline solid (1.38 g; 64 %).
δ_{H} (400 MHz, CDCl₃) 1.61 (t, J = 7.2, 3H), 4.36 (q, J = 7.2, 2H), 8.14 (s, 1H).
[The corresponding isomer 2,6-dichloro-7-ethyl-7*H*-purine was also isolated (0.61 g; 28 %). δ_{H} (400 MHz, CDCl₃) 1.64 (t, J = 7.2, 3H), 4.55 (q, J = 7.2, 2H), 8.27 (s, 1H).]

### Reference Example 7

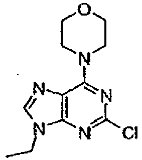

### 2-Chloro-9-ethyl-6-morpholin-4-yl-9H-purine

To a stirred solution of 2,6-dichloro-9-ethyl-9*H*-purine (1.38 g; 6.4 mmol) in CHCl₃ at 0 °C was added morpholine (1.2 mL; 13.8 mmol). The reaction mixture was stirred at 0 °C for 30 min then RT. for 1.5 h. Volatiles were evaporated, the residue was taken up in CH₂Cl₂ (100 mL) and washed with a mixture of H₂O/2M HCl/brine (1:1:1; 150 mL). The organic layer was dried (Na₂SO₄) and evaporated to give the title compound as a white solid (1.65 g; 96 %).
δ_{H} (400 MHz, CDCl₃) 1.52 (t, J = 7.2, 3H), 3.83-3.86 (m, 4H), 4.23 (q, J = 7.2, 2H), 4.32 (br s, 4H), 7.73 (s, 1H).

### Reference Example 8

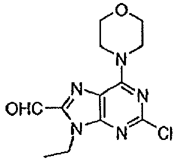

### 2-Chloro-9-ethyl-6-morpholin-4-yl-9H-purine-8-carbaldehyde

To a stirred solution of 2-chloro-9-ethyl-6-morpholin-4-yl-9*H*-purine (1.35 g; 5.0 mmol) and TMEDA (1.1 mL; 7.3 mmol) in anhydrous THF (40 mL) at -78 °C was added *n*-butyllithium (2.8 mL of a 2.5 M hexanes solution; 7.0 mmol). The orange solution was stirred at -78 °C for 30 min. after which time anhydrous DMF (0.7 mL; 9.0 mmol) was added. Stirring was continued at -78 °C for a further 30 min. after which time the reaction was quenched by pouring into cold 0.1. M HCl (500 mL) with vigorous stirring. The resulting solid precipitate was collected by filtration, washed with H₂O and dried to give the title compound as a pale yellow solid (1.23 g; 83 %).
δ_{H} (400 MHz, CDCl₃) 1.40 (t, J = 7.2, 3H), 3.83-3.86 (m, 4H), 4.05 (br s, 2H), 4.60 (q, J = 7.2, 2H), 4.68 (br s, 2H), 9.87 (s, 1H).

### Reference Example 9

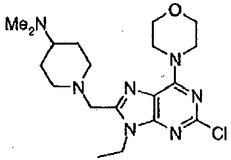

### [1-(2-Chloro-9-ethyl-6-morpholin-4-yl-9H-purin-8-ylmethyl)-piperidin-4-yl]-dimethyl-amine

To a stirred solution of 2-chloro-9-ethyl-6-morpholin-4-yl-9*H*-purine-8-carbaldehyde (250 mg; 0.85 mmol), 4-dimethylaminopiperidine (0.16 g; 1.25 mmol) and AcOH (0.05 ml) in 1,2-dichloroethane (4 mL) was added NaB(OAc)₃H (0.27 g; 1.27 mmol) and the resulting mixture was stirred at RT overnight (16 h). The reaction was quenched with 2 M HCl (10 ml) and stirred vigorously for 30 min. The layers were separated and the aqueous layer was basified to pH 11 with saturated Na₂CO₃ solution. This aqueous mixture was extracted with CH₂Cl₂ (20 mL), the organic layer was separated, dried (Na₂SO₄) and the solvent evaporated to give the title compound as an off-white solid (300 mg; 87 %).
δ_{H} (400 MHz, CDCl₃) 1.44 (t, J = 7.2, 3H), 1.47-1.54 (m, 2H), 1.83-1.86 (m, 2H), 2.10-2.16 (m, 3H), 2.29 (s, 6H), 2.88-2.91 (m, 2H), 3.69 (s, 2H), 3.82-3.85 (m ,4H), 4.30 (br s, 4H) 4.32 (q, J = 7.2, 2H).

### Reference Example 10

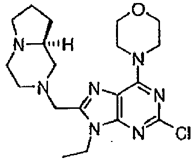

### 2-Chloro-9-ethyl-8-[(S)-1-(hexahydro-pyrrolo[1,2-a]pyrazin-2-yl)methyl]-6-moirpholin-4-yl-9H-purine

Prepared in a similar manner to Reference Example 9 using (*S*)-octahydro-pyrrolo[1,2-a]pyrazine in place of 4-dimethylaminopiperidine.
δ_{H} (400 MHz, CDCl₃) 1.46 (t, J = 7.2, 3H), 1.61-2.41 (m, 9H), 2.77-3.11 (m, 4H), 3.74 (AB doublet, J = 14, 1H), 3.79 (AB doublet, J = 14, 1H), 3.82-3.85 (m, 4H), 4.24 (br s, 4H) overlapping 4.33 (q, J = 7.2, 2H).

### Reference Example 11

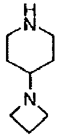

### 4-Azetidin-1-yl-piperidine

To a solution of 4-oxo-piperidine-1-carboxylic acid tert-butyl ester (1.75 g, 8.88 mmol) in dichloroethane (80 mL) was added azetidine (0.6 g, 10.53 mmol) and the mixture was stirred at RT for 30 min. Sodium triacetoxyborohydride (3.9 g, 18.44 mmol) was added and the resulting solution was stirred at RT for 18 h. The reaction mixture was partitioned between water and DCM and the layers separated. The organic layer was extracted further with DCM and the combined aqueous layers were concentrated *in vacuo*. The resultant white semi-solid was suspended in DCM and a saturated aqueous solution of NaHCO3 was added. The layers were thoroughly mixed, the organic layer isolated and the aqueous layer further extracted with DCM. The combined organic layers were washed with brine, dried (Na₂SO₄) and concentrated *in vacuo* to give 4-azetidin-1-yl-piperidine-1-carboxylic acid tert-butyl ester as a white solid (2.0 g, 95 %). BOC-deprotection of 4-azetidin-1-yl-piperidine-1-carboxylic acid tert-butyl ester (400 mg, 1.67 mmol) using TFA:DCM (1:4) gave the title compound as a yellow oil (185 mg, 79 %)
NMR δ_{H} (400 MHz, CDCl₃) 1.04-1.16 (m, 2 H), 1.68 (d, J = 12.8 Hz, 2 H), 1.98-2.08 (m, 3 H), 2.55 (td, J = 12.1, 2.6 Hz, 2 H), 3.06 (dt, J = 12.8, 3.6 Hz, 2 H) and 3.15 (t, J = 6.9 Hz, 4 H).

### Reference Example 12

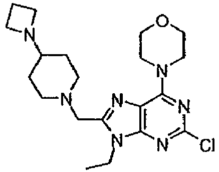

### 8-(4-Azetidin-1-yl-piperidin-1-ylmethyl)-2-chloro-9-ethyl-6-morpholin-4-yl-9H-purine

Prepared in a similar manner to Reference Example 9 using 4-azetidinl-yl-piperazine (Reference Example 11) in place of 4-dimethylaminopiperidine.
δ_{H} (400 MHz, CDCl₃) 1.22-1.38 (m, 2H), 1.42 (t, J = 7.2, 3H), 1.68-1.71 (m, 2H), 1.98-2.20 (m, 5H), 2.78-2.81 (m, 2H), 3.18 (br t, J = 6.8, 4H), 3.69 (s, 2H), 3.82-3.84 (m, 4H), 4.27-4.33 (m, 6H).

### Reference Example 13

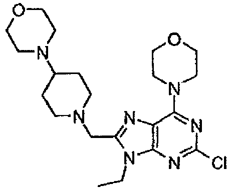

### 2-Chloro-9-ethyl-6-morpholin-4-yl-8-(4-morpholin-4-yl-piperidin-1-ylmethyl)-9H-purine

Prepared in a similar manner to Reference Example 9 using 4-morpholino-piperidine in place of 4-dimethylaminopiperidine.
δ_{H} (400 MHz, CDCl₃) 1.43 (t, J =7.2, 3H), 1.46-1.57 (m, 3H), 1.85-1.88 (m, 2H), 2.11-2.21 (m, 3H), 2.54-2.56 (m, 4H), 2.89-2.92 (m, 2H), 3.69 (s, 2H), 3.73-3.75 (m, 4H), 3.82-3.85 (m, 4H), 4.25-4.35 (m, 6H).

### Reference Example 14

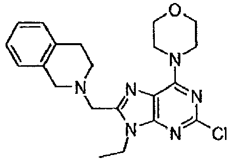

### 2-(2-Chloro-9-ethyl-6-morpholin-4-yl-9H-purin-8-ylmethyl)-1,2,3,4-tetrahydroisoquinoline

Prepared in a similar manner to Reference Example 9 using 1,2,3,4-tetrahydro-isoquinoline in place of 4-dimethylaminopiperidine.
δ_{H} (400 MHz, CDCl₃) 1.31 (t, J = 7.2, 3H), 2.70-2.82 (m, 4H), 3.62 (s, 2H), 3.75-3.77 (m, 4H), 3.81 (s, 2H), 4.24-4.29 (m, 6H), 6.92-6.94 (m, 1H), 7.02-7.10 (m, 3H).

### Reference Example 15

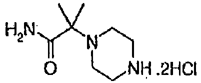

### 2-Piperazine-1-yl-isobutyramide di-hydrochloride

To a solution of *tert*-butyl-1-piperazinecarboxylate (15.0 g) in dichloromethane (150 mL) and methanol (150 ml) at 0 °C was added hydrogen chloride (40 mL; 2M solution in diethyl ether). The mixture was stirred at room temperature for 1.5 hours and reduced *in vacuo* to yield *tert-*butyl-1-piperazinecarboxylate hydrochloride (17.9 g).
To a solution of *tert*-butyl-1-piperazinecarboxylate hydrochloride (17.9 g) in water (200 mL) at room temperature was added sodium cyanide (3.94 g). A solution of acetone (5.9 mL) in water (20 mL) was then added dropwise and stirred at room temperature for 48 hours. The mixture was partitioned between ethyl acetate and water. The combined organic layers were washed with brine, separated, dried (MgSO₄) and reduced *in vacuo* to yield 4-(cyano-dimethyl-methyl)-piperazine-1-carboxylic acid *tert*-butyl ester (17.5 g).

To a solution of 4-(cyano-dimethyl-methyl)-piperazine-1-carboxylic acid *tert*-butyl ester (960 mg) in methyl sulfoxide (20 mL) at 0 °C was added potassium carbonate (104 mg). Hydrogen peroxide (2.0 mL; 27.5 wt % solution in water) was then added dropwise. The resulting mixture was heated to 40 °C overnight. To the cooled mixture was added water and the precipitated solid filtered and dried yielding 4-(1-carbamoyl-2-methyl-ethyl)-piperazine-1-carboxylic acid *tert*-butyl ester (677 mg). The BOC-group was removed using HCl in ether under standard conditions to give 2-piperazine-1-yl-isobutyramide di-hydrochloride (600 mg). [M+H]⁺: 172.

### Reference Example 16

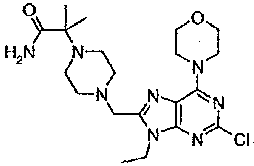

### 2-[4-(2-Chloro-9-ethy)-6-morpholin-4-yl-9H-purin-8-ylmethyl)-piperazin-1-yl]-isobutyramide

Prepared in a similar manner to Reference Example 9 using 2-piperazine-1-yl-isobutyramide di-hydrochloride (Reference Example 15) in place of 4-dimethylaminopiperidine.
δ_{H} (400 MHz, CDCl₃) 1.21 (s, 6H), 1.41 (t, J = 7.2, 3H), 2.52 (br s, 8H), 3.68 (s, 2H), 3.79-3.82 (m, 4H), 4.23-4.32 (m, 6H), 5.17 (br d, J = 4.8, 1H), 7.06 (br d, J = 4.8, 1 H).

### Reference Example 17

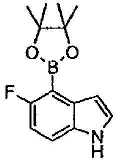

### 5-Fluoro-4-(4,4,5,5-tetramethyl-[1,3,2]dioxaborolan-2-yl)-1H-indole

A solution of 5-fluoroindole (5 g, 37.0 mmol) in DMF (40 mL) was treated at 0 °C with trifluoroacetic anhydride (6.1 mL, 42.6 mmol). After 30 min, the reaction was poured into water and the resulting precipitate collected by filtration, washed with water, then dried *in vacuo*. The solid was then dissolved in 10% aqueous NaOH (200 mL) and heated at reflux for 1 h. The reaction mixture was then cooled, washed with dichloromethane and acidified with aqueous HCl. The resulting white precipitate was collected by filtration, washed with water, taken up in dichloromethane, washed with water, dried (MgSO₄) and evaporated *in vacuo.* The resulting material (5 g, 75%) was dissolved in methanol (80 mL) and treated with concentrated sulfuric acid (2 mL) then heated at reflux overnight. The reaction was cooled and the resulting precipitate collected, washed with water and evaporated *in vacuo* to give 5-fluoro-1*H*-indole-3-carboxylic acid methyl ester as a peach-coloured solid (4.5 g, 83 %).

A solution of thallium tris(trifluoroacetate) (8.45 g, 15.6 mmol) in TFA (35 mL) was added to a solution of 5-fluoro-1*H*-indole-3-carboxylic acid methyl ester (2 g, 10.4 mmol) in TFA (10 mL) at room temperature and stirred for 2 h. The reaction mixture was evaporated *in vacuo* and the resulting residue suspended in water (25 mL) before being treated with a solution of potassium iodide (5.2 g, 31.3 mmol) in water (50 mL). The reaction mixture was treated with dichloromethane (100 mL) and methanol (5 mL) and the resulting precipitate removed by filtration through celite.

The organic layer was separated, washed successively with sodium thiosulfate solution and brine, then dried (MgSO₄) and evaporated *in vacuo.* The resultant material was dissolved in methanol (60 mL) and treated with 40% aqueous NaOH solution (60 mL) then refluxed for 2 h. The reaction mixture was cooled and extracted with DCM/MeOH (ratio 95:5), dried (MgSO₄), filtered and evaporated *in vacuo* to give a crude solid. Purification by column chromatography gave 5-fluoro-4-iodo-1*H*-indole as a pale brown solid (1.05 g, 39 %). NMR δ_{H} (300 MHz, CDCl₃) 6.49-6.52 (m, 1H), 6.95 (apparent dt, J = 0.4, 8.6, 1H), 7.26-7.33 (m, 2H) and 8.35 (s, 1H).

A solution of 5-fluoro-4-iodo-1*H*-indole (261 mg, 1.0 mmol) in dioxane (1 mL) was treated with triethylamine (0.2 mL, 1.4 mmol), palladium acetate (4.5 mg, 0.02 mmol) and bis(cyclohexyl)phosphino-2-biphenyl (28 mg, 0.08 mmol) then heated to 80 °C. A solution of pinacolborane (1 M in THF, 2.66 mL, 2.66 mmol) was added via syringe. After 30 min, the reaction mixture was cooled, then diluted with water (10 mL) and DCM (10 mL). The resulting mixture was passed through a phase separation cartridge, and the dichloromethane layer was evaporated *in vacuo* to obtain the title compound which was used without further purification.

### Reference Example 18

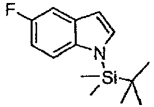

### 1-(tert-Butyl-dimethyl-silanyl)-5-fluoro-1H-indole

To a solution of 5-fluoro-1*H*-indole (30.0 g, 0.222 mol) in anhydrous THF (250 mL) was added sodium hydride (60 % suspension in mineral oil, 10.22 g, 0.255 mol) portionwise and maintaining the solution at 0 °C. The reaction mixture was stirred at 0 °C for 20 min, then a solution of tert-butyl-chloro-dimethyl-silane (40.15 g, 0.266 mol) in anhydrous THF (20 mL) was added and the solution stirred at RT for 25 h. The reaction mixture was poured into H₂O and the layers separated. The aqueous layer was extracted with EtOAc and the combined organic layers were dried (MgSO₄), then concentrated *in vacuo.* The resultant residue was purified by column chromatography (silica gel, cyclohexane:DCM 100 % to 50:50) to provide the title compound was obtained as a colourless oil (41.2 g, 74 %).
¹H NMR (400 MHz, CDCl₃): δ 0.60 (s, 6 H), 0.94 (s, 9 H), 6.58 (dd, J = 3.2, 1.0 Hz, 1 H), 6.87-6.93 (m, 1 H), 7.23 (d, J = 3.2 Hz, 1 H), 7.24-7.29 (m, 1 H) and 7.41 (m, 1 H).

### Reference Example 19

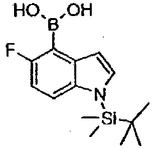

### [1-(tert-Butyl-dimethyl-silanyl)-5-fluoro-1H-indol-4-yl]boronic acid

To a solution of 1-(tert-butyl-dimethyl-silanyl)-5-fluoro-1*H*-indole (30.0 g, 0.12 mol) in anhydrous THF (1000 mL) were added *N,N,N',N*'-tetramethylethylenediamine (36.6 mL, 0.241 mol) and a solution of *s*-butyl lithium (1.4 M in cyclohexane, 172 mL, 0.241 mmol) at-78 °C. The resulting mixture was stirred at -78 °C for 2 h, then triisopropyl borate (37.5 mL, 162.7 mmol) was added dropwise. The resulting solution was stirred at -78 °C for 40 min, then allowed to warm to -20 °C. An aqueous solution of HCl (2.4 M, 250 mL) was added and the resulting mixture was poured into H₂O. The layers were separated and the aqueous layer extracted with EtOAc. The combined organic layers were dried (MgSO₄) and concentrated *in vacuo*. The resultant yellow solid was then crystallised from DCM and cyclohexane to give the title compound as a white solid (25.0 g, 71 %).
¹H NMR (400 MHz, CD₃OD): δ 0.62 (s, 6 H), 0.92 (s, 9 H), 6.51 (d, J = 3.2 Hz, 1 H), 6.79-6.90 (m, 1 H), 7.30-7.36 (m, 1 H) and 7.54 (dd, J = 9.0, 4.6 Hz, 1 H).

### Reference Example 20

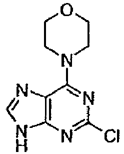

### 2-Chloro-6-morpholin-4-yl-9H-purine

To a solution of 2,6-dichloro-9*H*-purine (5.0 g, 26.46 mmol) in water (100 mL) was added morpholine (6.9 mL, 79.37 mmol). The resulting mixture was heated at reflux for 15 min, and then allowed to cool to RT. The resultant white precipitate was collected by filtration and washed with water, MeOH and EtOH, then dried at 40 °C for 18 h to give the title compound (6.26 g, 99 %).
¹H NMR (400 MHz, DMSO-d₆): δ 3.72 (m, 4 H), 4.18 (m, 4 H), 8.16 (s, 1 H) and 13.24 (bs, 1 H).

### Reference Example 21

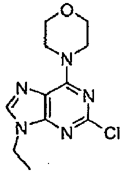

### 2-Chloro-9-ethyl-6-morpholin-4-yl-9H-purine

To a solution of 2-chloro-6-morpholin-4-yl-9*H*-purine (304 mg, 1.27 mmol) in DMF (6 mL) were added bromoethane (284 µL, 3.81 mmol) and sodium hydroxide (152 mg, 3.81 mmol). The reaction mixture was heated at 150 °C for 2 h, then allowed to cool to RT. The reaction mixture was partitioned between water and DCM, the layers separated and the aqueous layer was further extracted with DCM. The combined organic fractions were washed with water, dried (Na₂SO₄) and concentrated *in vacuo* to give the title compound as a yellow solid (210 mg, 80 %).
¹H NMR (400 MHz, CDCl₃): δ 1.50 (t, J = 7.3 Hz, 3 H), 3.79-3.85 (m, 4 H), 4.13-4.32 (m, 6 H) and 7.71 (s, 1 H).

### Reference Example 22

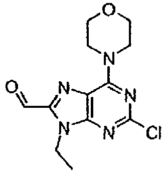

### 2-Chloro-9-ethyl-6-morpholin-4-yl-9H-purine-8-carbaldehyde

To a solution of 2-chloro-9-ethyl-6-morpholin-4-yl-9*H*-purine (7.27 g, 27.0 mmol) and TMEDA (6.1 mL, 40.6 mmol) in anhydrous THF (200 mL) was added a solution of *n-*BuLi in THF (2.5 M, 15.1 mmol, 37.9 mmol) at - 78 °C. The resulting mixture was stirred at -78 °C for 1 h, then anhydrous DMF (3.8 mL, 48.7 mmol) was added drop-wise. The reaction mixture was stirred at -78 °C for 1 h, then partitioned between a cold aqueous solution of HCl (0.1 M) and DCM. The organic layer was separated and dried (Na₂SO₄), then concentrated *in vacuo.* The resultant residue was purified by column chromatography to give the title compound as a cream solid (6.02 g, 75 %).
[M + H]⁺ 296.1

### Reference Example 23

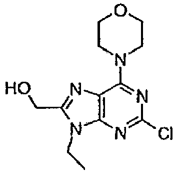

### (2-Chloro-9-ethyl-6-morpholin-4-yl-9H-purin-8-yl)-methanol

To a suspension of 2-chloro-9-ethyl-6-morpholin-4-yl-9*H*-purine-8-carbaldehyde (1.18 g, 4.0 mmol) in IMS (20 mL) and THF (20 mL) was added sodium borohydride (152 mg, 4.0 mmol). The reaction mixture was stirred at RT for 90 min, then concentrated *in vacuo.* The resulting residue was partitioned between EtOAc and a saturated aqueous solution of NaHCO₃. The organic layer was separated and washed with brine, then dried (Na₂SO₄) and concentrated *in vacuo* to give the title compound as a white solid (1.20 g, 99 %).
[M + H]⁺ 298.3

### Reference Example 24

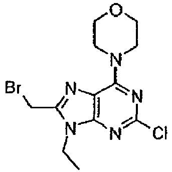

### 8-Bromomethyl-2-chloro-9-ethyl-6-morpholin-4-yl-9H-purine

To a suspension of (2-chloro-9-ethyl-6-morpholin-4-yl-9*H*-purin-8-yl)-methanol (1.20 g, 4.0 mmol) in DCM (50 mL) were added carbon tetrabromide (1.59 g, 4.8 mmol) and triphenylphosphine (1.36 g, 5.2 mmol) at 0 °C. The reaction mixture was stirred at 0 °C for 4 h, then partitioned between DCM and brine. The organic layer was separated and washed with brine, then dried (Na₂SO₄) and concentrated *in vacuo.* The resultant residue was purified by column chromatography to give the title compound as a white solid (1.24 g, 86 %).
[M + H]⁺ 360.2 (⁷⁹Br) and 362.2 (⁸¹Br)

### Reference Example 25

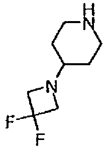

### 4-(3,3-Difluoro-azetidin-1-yl)-piperidine

To a solution of 4-oxo-piperidine-1-carboxylic acid *tert*-butyl ester (1.0 g, 5.0 mmol) in DCE (50 mL) was added 3,3-difluoroazetidine hydrochloride (712 mg, 5.5 mmol). The mixture was stirred at RT for 15 min, then sodium triacetoxyborohydride (1.59 g, 7.5 mmol) was added and stirring was continued for 17 h. The reaction mixture was diluted with brine and extracted with DCM. The organic layer was separated, dried (Na₂SO₄) and concentrated *in vacuo.* The resultant residue was purified by column chromatography to give 4-(3,3-difluoro-azetidin-1-yl)-piperidine-1-carboxylic acid *tert*-butyl ester as a pale yellow solid (1.2 g, 88 %). To a solution of 4-(3,3-difluoro-azetidin-1-yl)-piperidine-1-carboxylic acid *tert-*butyl ester (552 mg, 2.0 mmol) in DCM (4 mL) was added TFA (2 mL) and the resulting mixture was stirred at RT for 45 min. The reaction mixture was loaded onto an Isolute® SCX-2 cartridge, washed with MeOH then eluted with 2 M NH₃ in MeOH to give the title compound as a pale yellow solid (271 mg, 77 %).
¹H NMR (400 MHz, CDCl₃): δ 1.16-1.27 (m, 2 H), 1.63-1.72 (m, 2 H), 2.14-2.23 (m, 1 H), 2.54-2.62 (m, 2 H), 3.09 (dt, J = 12.7, 3.9 Hz, 2 H) and 3.46-3.57 (m, 4 H).

### Reference Example 26

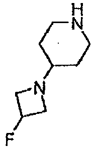

### 4-(3-Fluoro-azetidin-1-yl)-piperidine

Prepared according to the method used in the preparation of 4-(3,3-difluoro-azetidin-1-yl)-piperidine using 3-fluoroazetidine in place of 3,3-difluoroazetidine hydrochloride. The title compound was obtained as a colourless oil (180 mg, 39 %).
¹H NMR (400 MHz, CDCl₃): δ 1.13-1.24 (m, 2 H), 1.64 (dd, J = 12.6,4.3 Hz, 2 H), 2.16-2.23 (m, 1 H), 2.82-2.91 (m, 2 H), 3.01-3.14 (m, 2 H), 3.59-3.67 (m, 2 H), 3.78-3.95 (m, 2 H), 5.10 (m, J = 55.8 Hz, 1 H).

### Reference Example 27

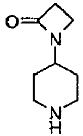

### 1-Piperidin-4-yl-azetidin-2-one

To a solution of 4-(2-methoxycarbonyl-ethylamino)-piperidine-1-carboxylic acid *tert-*butyl ester (3.4 g, 12.0 mmol) in anhydrous THF (75 mL) was slowly added a solution of methyl magnesium bromide in diethyl ether (3 M, 6 mL, 18 mmol) at 0 °C. The reaction mixture was stirred at 0 °C for 3 h, then allowed to warm to RT and stirring was continued for 72 h. The reaction mixture was concentrated *in vacuo* and the resulting residue was partitioned between EtOAc and an aqueous solution of ammonium chloride. The organic layer was separated and washed with brine, then dried (Na₂SO₄) and concentrated *in vacuo.* The resultant residue was purified by column chromatography to give 4-(2-oxo-azetidin-1-yl)-piperidine-1-carboxylic acid *tert*-butyl ester as a pale yellow oil (598 mg, 20 %). To a solution of 4-(2-oxo-azetidin-1-yl)-piperidine-1-carboxylic acid *tert*-butyl ester (595 mg, 2.34 mmol) in DCM (6 mL) was added TFA (2 mL). The resulting mixture was stirred at RT for 1.5 h, then concentrated *in vacuo.* The resultant residue was loaded onto an Isolute® SCX-2 cartridge, washed with MeOH then eluted with 2 M NH₃ in MeOH to give the title compound as a pale yellow solid (325 mg, 90 %).
¹H NMR (400 MHz, CDCl₃): δ 1.48-1.61 (m, 2 H), 1.83 (dd, J = 12.6, 3.7 Hz, 2 H), 2.54-2.67 (m, 2 H), 2.86 (t, J = 4.0 Hz, 2 H), 3.10 (dt, J = 12.6, 3.5 Hz, 2 H), 3.22 (t, J = 4.0 Hz, 2 H) and 3.66 (m, 1 H).

### Reference Example 28

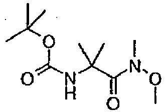

### [1-(Methoxy-methyl-carbamoyl)-1-methyl-ethyl]-carbamic acid tert-butyl ester

To a solution of 2-tert-butoxycarbonylamino-2-methyl-propionic acid (20 g, 98.5 mmol) and bis(2-oxo-3-oxazolidinyl)phosphinic chloride (32.6 g, 128.0 mmol) in DCM (280 mL) were added *N,O*-dimethylhydroxylamine hydrochloride (10.4 g, 107.0 mmol) and triethylamine (40.0 mL, 287.0 mmol) at 0 °C. The resulting suspension was stirred at RT for 70 h, and then partitioned between water and DCM. The organic layer was separated and washed with brine, then dried (MgSO₄) and concentrated *in vacuo*. The resultant residue was purified by column chromatography to give the title compound as a colourless oil (12.2 g, 50 %).
¹H NMR (400 MHz, CDCl₃): δ 1.44 (s, 9 H), 1.54 (s, 6 H), 3.21 (s, 3 H), 3.70 (s, 3 H) and 5.24 (bs, 1 H).

### Reference Example 29

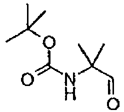

### (1,1-Dimethyl-2-oxo-ethyl)-carbamic acid tert-butyl ester

To a solution of [1-(methoxy-methyl-carbamoyl)-1-methyl-ethyl]-carbamic acid tert-butyl ester (8.4 g, 34.1 mmol) in anhydrous THF (150 mL) was added Super Hydride® dropwise at - 40 °C. The resulting mixture was allowed to warm to RT over 30 min, and then stirred at RT for 1 h. An aqueous solution of citric acid was added, the mixture was concentrated *in vacuo* and the resulting residue partitioned between water and EtOAc. The organic layer was separated and washed with brine, then dried (MgSO₄) and concentrated *in vacuo.* The resultant residue was purified by column chromatography to give the title compound as a white solid (4.36 g, 68 %).
¹H NMR (400 MHz, CDCl₃): δ 1.33 (s, 6 H), 1.44 (s, 9 H), 4.96 (bs, 1 H)and 9.43 (s, 1 H).

### Reference Example 30

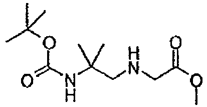

### (2-tert-Butoxycarbonylamino-2-methyl-propylamino)-acetic acid methyl ester

To a solution of (1,1-dimethyl-2-oxo-ethyl)-carbamic acid tert-butyl ester (0.66 g, 3.52 mmol) in DCE (40 mL) was added glycine methyl ester hydrochloride (0.93 g, 7.41 mmol). The resulting suspension was stirred at RT for 30 min, and then sodium cyanoborohydride (0.83 g, 3.92 mmol) was added. The resulting white suspension was stirred at RT for 19 h, and then partitioned between a saturated aqueous solution of NaHCO₃ and EtOAc. The organic layer was separated and washed with brine, then dried (MgSO₄) and concentrated *in vacuo.* The resultant residue was purified by column chromatography to give the title compound as a colourless oil (0.80 g, 88 %).
¹H NMR (400 MHz, CDCl₃): 8 1.29 (s, 6 H), 1.44 (s, 9 H), 1.62 (bs, 1 H), 2.64 (s, 2 H), 3.43 (s, 2 H), 3.73 (s, 3 H) and 5.04 (bs, 1 H).

### Reference Example 31

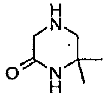

### 6,6-Dimefhyl-piperazin-2-one

A solution of (2-tert-butoxycarbonylamino-2-methyl-propylamino)-acetic acid methyl ester (0.8 g, 3.07 mmol) in DCM (20 mL) and TFA (5 mL) was stirred at RT for 90 min. The reaction mixture was concentrated *in vacuo,* then azeotroped with toluene to give (2-amino-2-methyl-propylamino)-acetic acid methyl ester as a colourless oil. A solution of (2-amino-2-methyl-propylamino)-acetic acid methyl ester in IMS (6 mL) and NH₄OH (2 mL) was stirred at RT for 90 min. The reaction mixture was concentrated *in vacuo,* then azeotroped with toluene. The resultant white solid was used in subsequent reactions without further purification (100 %).
¹H NMR (400 MHz, CDCl₃): δ 1.27 (s, 6 H), 2.78 (s, 2 H), 3.43 (s, 2 H) and 6.76 (bs, 1 H).

### Reference Example 32

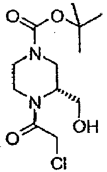

### (R)-4-(2-Chloro-acetyl)-3-hydroxymethyl-piperazine-1-carboxylic acid tert-butyl ester

To a solution of (*R*)-3-hydroxymethyl-piperazine-1-carboxylic acid *tert*-butyl ester (795 mg, 3.68 mmol) in DCM (20 mL) was added triethylamine (1.53 mL, 11.04 mmol). The resulting mixture was cooled to 0 °C before the dropwise addition of chloroacetyl chloride (325 µL, 4.05 mmol). The mixture was warmed to RT and stirred for 5 h. The reaction mixture was partitioned between a saturated aqueous solution of NaHCO₃ and DCM and the aqueous layer extracted with further DCM. The combined organic fractions were dried (Na₂SO₄) and concentrated *in vacuo*. The resulting residue was purified by column chromatography to give the title compound as a colourless oil which was a mixture of rotamers (710 mg, 66%).
¹H NMR (400 MHz, CDCl₃): δ 1.48 (s, 9H), 2.80-2.92 (m, 1H), 2.95-3.10 (m, 2H), 3.34-3.44 (m, ½H), 3.60-3.74 (m, 2½H), 3.96-4.16 (m, 3½H), 4.22-4.30 (m, ½H), 4.32-4.40 (m, ½H) and 4.63 (bs, ½H).

### Reference Example 33

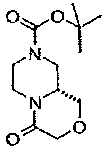

### (R)-4-Oxo-hexahydro-pyrazino[2,1-c][1,4]oxazine-8-carboxylic acid tert-butyl ester

To a solution of (*R*)-4-(2-chloro-acetyl)-3-hydroxymethyl-piperazine-1-carboxylic acid tert-butyl ester (710 mg, 2.45 mmol) in THF (16 mL) at 0 °C was added potassium *tert-*butoxide (326 mg, 2.91 mmol). The resulting mixture was stirred for 75 min before the addition of acetic acid (0.6 mL). The resulting mixture was partitioned between water and DCM and the aqueous layer extracted with further DCM. The combined organic fractions were dried (Na₂SO₄) and concentrated *in vacuo.* The resulting residue was purified by column chromatography to give the title compound as a colourless oil (570 mg, 91%).
¹H NMR (400 MHz, CDCl₃): δ 1.48 (s, 9H), 2.69 (td, J = 12.9, 3.0 Hz, 2H), 2.78-2.89 (m, 1H), 3.48-3.58 (m, 2H), 3.96-4.10 (m, 3H), 4.14 (d, J = 16.2 Hz, 1H), 4.20 (d, J = 16.8 Hz, 1H) and 4.57 (m, 1H).

### Reference Example 34

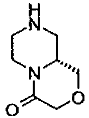

### (R)-Hexahydro-pyrazino[2,1-c][1,4]oxazin-4-one

To a solution of (*R*)-4-oxo-hexahydro-pyrazino[2.1-*c*][1,4]oxazine-8-carboxylic acid *tert*-butyl ester in DCM (5 mL) was added TFA (1 mL). The resulting mixture was stirred at RT for 2 h then concentrated *in vacuo*. The resulting residue was azeotroped with toluene then purified by SCX column to give the title compound as a colourless oil (60 mg, 76%).
¹H NMR (400 MHz, MeOD): δ 2.46-2.55 (m, 1H), 2.59-2.76 (m, 2H), 2.90-3.03 (m, 2H), 3.50-3.60 (m, 2H), 3.94-4.02 (m, 1H), 4.10 (s, 2H) and 4.42-4.48 (m, 1H).

### Reference Example 35

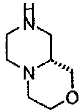

### (R)-Octahydro-pyrazino[2,1-c][1,4]oxazine

To a solution of (*R*)-hexahydro-pyrazino[2,1-*c*][1,4]oxazin-4-one (60 mg, 0.39 mmol) in dioxane (5 mL) was added LiAlH₄ (1.5 mL, 1M solution in THF). The resulting mixture was heated at 80 °C for 2.5 h before the addition of *ⁱ*PrOH (0.5 mL) followed by a saturated aqueous solution of Na₂SO₄ (3 mL). Further Na₂SO₄ was added and the resulting mixture filtered through Celite, washing with EtOAc. The resulting residue was purified by NH₂ colunm to give the title compound as an oil (40 mg, 73%).
¹H NMR (400 MHz, CDCl₃): δ 2.17-2.30 (m, 2H), 2.36-2.47 (m, 2H), 2.61 (d, J = 11.9 Hz, 1H), 2.69-2.79 (m, 2H), 2.92-2.99 (m, 2H), 3.23 (t, J = 10.3 Hz, 1H), 3.61-3.75 (m, 2H) and 3.79-3.87 (m, 1H).

### Reference Example 36

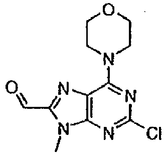

### 2-Chloro-9-methyl-6-morpholin-4-yl-9H-purine-8-carbaldehyde

To a solution of 2-chloro-9-methyl-6-morpholin-4-yl-9*H*-purine (1.0 g, 3.9 mmol) in anhydrous THF (25 mL) was added dropwise a solution of LiHMDS in THF (1.0 M, 5.9 mL, 5.9 mmol) at -78 °C. The resulting mixture was stirred at -78 °C for 1 h, then anhydrous DMF (2.3 mL, 30.3 mmol) was added drop-wise. The reaction mixture was stirred at -78 °C for 45 min, then 30 min at RT. The resulting mixture was cooled down to 0°C and quenched with water, partitioned between a cold aqueous solution of HCl (1.0 M) and DCM. The organic layer was separated and dried (Na₂SO₄), then concentrated *in vacuo* to give the title compound as an orange solid (1.12 g, 99 %).
[M + H]⁺ 282.0

### Reference Example 37

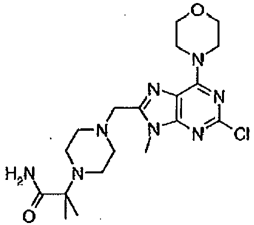

### 2-[4-(2-Chloro-9-methyl-6-morpholin-4-yl-9H-purin-8-ylmethyl)-piperazin-1-yl]-isobutyramide

Prepared according to the method used in the preparation of 8-(2-chloro-9-ethyl-6-morpholin-4-yl-9*H*-purin-8-ylmethyl)-2,8-diazaspiro[4.5]decan-1-one using 2-chloro-9-methyl-6-morpholin-4-yl-9*H*-purine-8-carbaldehyde instead of 2-chloro-9-ethyl-6-morpholin-4-yl-9*H*-purine-8-carbaldehyde and 2-piperazin-1-yl-isobutyramide in place of 2,8-diaza-spiro[4.5]decan-1-one. The title compound was obtained as a yellow solid (384 mg, 83 %)
[M+H]⁺ 437.2

### Reference Example 38

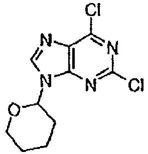

### 2,6-Dichloro-9-(tetrahydro-pyran-2-yl)-9H-purine

A solution of 2,6-dichloropurine (3.1 g), 3,4-dihydro-2H-pyran (2.76 g) and *p-*toluenesulfonic acid (0.31 g) in THF (35 mL) was heated at 60 °C for 4 h. The solvent was evaporated *in vacuo* and the residue purified by column chromatography (50% ethyl acetate-petrol) to give the title compound as a white solid (3.62 g).
δH (400 MHz, CDCl₃) 1.69 - 2.21 (m, 6H), 3.80 (m, 1H), 4.21 (m, 1H), 5.78 (dd, J = 2.5, 10.6, 1H), 8.34 (s, 1H).

### Reference Example 39

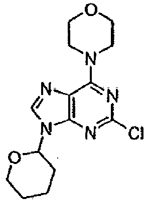

### 2-Chloro-6-morpholin-4-yl-9-(tetrahydro-pyran-2-yl)-9H-purine

To a solution of 2,6-dichloro-9-(tetrahydro-pyran-2-yl)-9*H*-purine (1.0 g) in CH₂Cl₂ (25 mL) at 0 °C was added morpholine (0.687 g) and the mixture stirred for 0.5 h. The mixture was then stirred at RT for 2.5 h. The solvent was evaporated and the residue dissolved in CH₂Cl₂ and washed successively with 1M HCl, brine and then dried (MgSO₄). The solvent was evaporated to yield a white solid (0.97 g).
8H (400 MHz, CDCl₃) 1.64 - 2.19 (m, 6H), 3.79 (m, 1H), 3.84 (t, J = 4.8, 4H), 4.17 (m, 1H), 4.32 (br s, 4H), 5.44 (dd, J = 2.3, 10.7, 1H), 7.93 (s, 1H).

### Reference Example 40

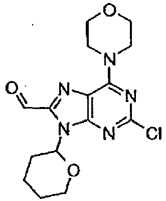

### 2-Chloro-6-morpholin-4-yl-9-(tetrahydro-pyran-2-yl)-9H-purine-8-carbaldehyde

A solution of 2-chloro-6-morpholin-4-yl-9-(tetrahydropyran-2-yl)-9H-purine (716 mg, 2.21 mmol) and TMEDA (0.5 mL, 3.32 mmol) was dissolved in THF (20mL) and the solution was cooled to -78°C under a nitrogen atmosphere, n-Butyllithium (2.5M, 1.2 mL, 3.1 mmol) was added dropwise and the resulting yellow solution was stirred at -78°C for 40 min. DMF (0.31 mL, 3.98mmol) was added and the solution was stirred for a further 1 h then quenched with water before the mixture was allowed to warm to room temperature. The mixture was diluted with water and neutralised with HCl (1M) and extracted with EtOAc (x3). The organics were dried (MgSO₄) and the solvent was removed *in vacuo.* The resulting residue was purified on silica using 0-30% EtOAc in pentane as eluant, to afford the product as a yellow solid (509 mg, 65%).
¹H NMR (400 MHz, CDCl₃): δ 1.55-1.92 (m, 4 H), 2.01-2.12 (m, 1 H), 2.76 (dq, J = 12, 4 Hz, 1 H), 3.76 (dt, J = 12,4 Hz, 1H), 3.85 (t, J = 4.6Hz, 4H), 3.94-4.20 (m, 3H), 4.52-4.80 (m, 2H), 6.25 (dd, J = 12, 2 Hz, 1H), 9.95 (s, 1 H).

### Reference Example 41

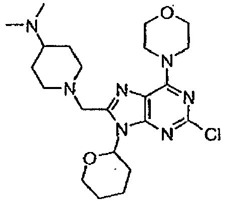

### {1-[2-Chloro-6-morpholin-4-yl-9-(tetrahydro-pyran-2-yl)-9H-purin-8-ylmethyl]-piperidin-4-yl}-dimethyl-amine

Prepared from 2-chloro-6-morpholin-4-yl-9-(tetrahydro-pyran-2-yl)-9*H*-purine-8-carbaldehyde (330 mg) using the method described for [1-{2-chloro-9-ethyl-6-morpholin-4-yl-9*H*-purin-8-ylmethyl)-piperidin-4-yl]-dimethyl-amine. The title compound was isolated as a clear oil (361 mg).
δH(400 MHz, CDCl₃) 1.47 - 2.21 (m, 12H), 2.32 (s, 6H), 2.86 (m, 1H), 2.98 (m, 2H), 3.67-3.72 (m, 2H), 3.82 (t, J = 4.8, 4H), 3.90 (d, J = 13.6, 1H), 4.18 (m, 1H), 4.29 (br s, 4H), 5.86 (dd, J = 2.43, 11.12 1H).

### Reference Example 42

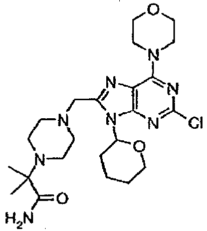

### 2-{4-[2-Chloro-6-morpholin-4-yl-9-(tetrahydro-pyran-2-yl)-9H-purin-8-ylmethyl]-piperazin-1-yl}-isobutyramide

2-Chloro-6-morpholin-4-yl-9-(tetrahydro-pyran-2-yl)-9*H*-purine-8-carbaldehyde (250 mg, 0.71 mmol) and 2-piperazin-1-yl-isobutyramide were suspended in DCE (10mL) and the mixture was stirred for 1 h. Sodium triacetoxyborohydride (226 mg, 1.07 mmol) was added and the resulting mixture was stirred overnight. The mixture was diluted with water and the phases were separated using a phase separating cartridge. The organic phase was concentrated *in vacuo* and the residue was purified by chromatography on silica using 0-5% MeOH in EtOAc to elute the product (298 mg, 83%).
[M + H]⁺ 507.4

### Reference Example 43

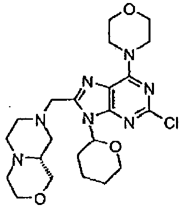

### (R)-8-[2-Chloro-6-morpholin-4-yl-9-(tetrahydro-pyran-2-yl)-9H-purin-8-ylmethyl]-octahydro-pyrazino[2,1-c][1,4]oxazine

2-Chloro-6-morpholin-4-yl-9-(tetrahydro-pyran-2-yl)-9*H*-purine-8-carbaldehyde (259 mg, 0.74 mmol) and (*R*)-octahydropyrazino[2,1-*c*][1,4]oxazine (126 mg, 0.88 mmol) were suspended in DCE (10mL) and the mixture was stirred for 1 h. Sodium triacetoxyborohydride (234 mg, 1.10 mmol) was added and the resulting mixture was stirred overnight. The mixture was diluted with water and the phases were separated using a phase separating cartridge. The organic phase was concentrated *in vacuo* and the residue was purified by chromatography on silica using 0-5% MeOH in EtOAc to elute the product (322 mg, 91 %).
[M + H]⁺ 478.4

### Reference Example 44

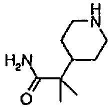

### 2-Piperidin-4-yl-isobutyramide

To a suspension of sodium hydride (60 % suspension in mineral oil, 0.52 g, 13.0 mmol) in anhydrous 1,2-dimethoxyethane (5 mL) was added triethyl 2-phosphonopropionate (2.63 g, 11.0 mmol) at 0 °C. The resulting mixture was stirred for 15 min, then 4-oxo-piperidine-1-carboxylic acid tert-butyl ester (2.00 g, 10.0 mmol) was added. The reaction mixture was stirred at 0 °C for 45 min, allowed to warm to RT, stirred for 1 h and then heated at 70 °C for a further 17 h. The reaction mixture was allowed to cool, then partitioned between water and DCM. The organic layer was separated, passed through a hydrophobic frit and concentrated *in vacuo.* The resultant residue was purified by column chromatography to give 4-(1-ethoxycarbonyl-ethylidene)-piperidine-1-carboxylic acid tert-butyl ester as a colourless oil (1.05 g, 37 %).

To a solution of 4-(1-ethoxycarbonyl-ethylidene)-piperidine-1-carboxylic acid tert-butyl ester (1.05 g, 3.71 mmol) in IMS (15 mL) was added palladium (10 wt. % on carbon, 110 mg) and ammonium formate (saturated solution in IMS, 5 mL). The mixture was stirred at RT for 36 h, filtered through Celite and the filtrate evaporated. The resulting residue was partitioned between DCM and water, the organic layer separated, passed through a hydrophobic frit and concentrated *in vacuo* to give 4-(1-ethoxycarbonyl-ethyl)-piperidine-1-carboxylic acid tert-butylester as a colourless oil (1.04 g, 98 %). To a solution of 4-(1-ethoxycarbonyl-ethyl)-piperidine-1-carboxylic acid tert-butylester (1.04 g, 3.64 mmol) in anhydrous THF (15 mL) was added a solution of lithium diisopropylamide (1.8 M solution in THF/heptane/ethylbenzene, 4.05 mL, 7.29 mmol) at - 78 °C. The resulting mixture was allowed to warm to 0 °C and stirred for 30 min. Methyl iodide (5.17 g, 36.44 mmol) was added drop-wise, the mixture stirred for 2 h and then allowed to warm to RT. The reaction mixture was quenched with water and extracted with DCM. The organic layer was separated, passed through a hydrophobic frit and concentrated *in vacuo*. To a solution of the resulting residue in IMS (10 mL) was added an aqueous solution of NaOH (1 M, 20 mL). The resulting mixture was heated at 70 °C for 65 h, then allowed to cool to RT, diluted with H₂O and washed with DCM. The aqueous layer was then acidified and extracted with DCM. The organic layer was separated, passed through a hydrophobic frit and concentrated *in vacuo* to give 4-(1-carboxy-1-methyl-ethyl)-piperidine-1-carboxylic acid tert-butyl ester as an orange solid (777 mg, 79 % over 2 steps). To a solution of 4-(1-carboxy-1-methyl-ethyl)-piperidine-1-carboxylic acid tert-butyl ester (777 mg, 2.86 mmol) in DMF (10 mL) was added triethylamine (2.61 g, 25.77 mmol), ammonium chloride (766 mg, 14.32 mmol) and HATU (1.20 g, 3.15 mmol). The resulting mixture was stirred at RT for 18 h, then partitioned between EtOAc and an aqueous solution of HCl (1 M). The organic layer was separated and dried (MgSO₄), then concentrated *in vacuo.* To a solution of the resultant residue in DCM (5 mL) was added TFA (3 mL) and the resulting mixture stirred at RT for 3 h. The reaction mixture was loaded onto an Isolute® SCX-2 cartridge, washed with MeOH then eluted with 2 M NH₃ in MeOH to give the title compound as a pale orange solid (122 mg, 25 % over 2 steps).
¹H NMR (400 MHz, CDCl₃): δ 1.14 (s, 6 H), 1.27-1.39 (m, 2 H), 1.63 (m, 2 H), 1.70-1.79 (m, 1 H), 1.99 (bs, 1 H), 2.59-2.67 (m, 2 H), 3.18 (m, 2 H), 5.44-5.67 (bs, 2 H).

### Reference Example 45

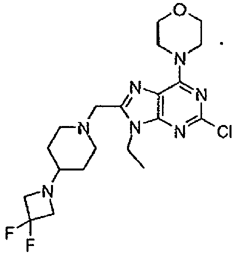

### 2-Chloro-8-[4-(3,3-difluoro-azetidin-1-yl)-piperidin-1-ylmethyl]-9-ethyl-6-morpholin-4-yl-9H-purine

To a solution of 2-chloro-9-ethyl-6-morpholin-4-yl-9*H*-purine-8-carbaldehyde (180 mg, 0.61 mmol) in DCE (15 mL) was added 4-(3,3-difluoro-azetidin-1-yl)-piperidine (130 mg, 0.74 mmol). The mixture was stirred at RT for 1 h then sodium triacetoxyborohydride (200 mg, 0.92 mmol) was added and stirring was continued for 17 h. The reaction mixture was added to water and loaded onto an Isolute® SCX-2 cartridge. The cartridge was washed with MeOH before the product was eluted with 2 M NH₃ in MeOH. The resultant residue was purified by column chromatography to give the title compound as a yellow oil (270 mg, 97 %).
[M + H]⁺ 456.4

### Reference Example 46

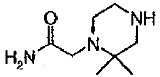

### 2-(2,2-Dimethyl-piperazin-1-yl)-acetamide

To a solution of 3,3-dimethyl-piperazine-1-carboxylic acid tert-butyl ester (0.55 g, 2.57 mmol) in acetonitrile (10 mL) were added 2-bromoacetamide (0.42 g, 3.08 mmol), potassium carbonate (1.06 g, 7.70 mmol) and tetrabutylammonium iodide (95 mg, 0.257 mmol). The reaction mixture was heated at 60 °C for 18 h, then concentrated *in vacuo.* The resulting residue was partitioned between DCM and water. The organic layer was separated, dried (Na₂SO₄), and concentrated *in vacuo* to give 4-carbamoylmethyl-3,3-dimethylpiperazine-1-carboxylic acid tert-butyl ester as a cream solid. 4-Carbamoylmethyl-3,3-dimethyl-piperazine-1-carboxylic acid tert-butyl ester was subsequently BOC-deprotected by using the general method to give the title compound as a white solid (0.43 g, 97 %).
[M + H]⁺ 172.1

### Reference Example 47

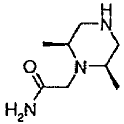

### 2-((cis)-2,6-Dimethyl-piperazin-1-yl)-acetamide

Prepared according to the method used in the preparation of 2-(2,2-dimethyl-piperazin-1-yl)-acetamide using (*cis*)-3,5-dimethyl-piperazine-1-carboxylic acid tert-butyl ester in place of 3,3-dimethyl-piperazine-1-carboxylic acid tert-butyl ester. The title compound was obtained as a white solid (283 mg, 67 %).
¹H NMR (400 MHz, CDCl₃): δ 0.99 (d, J = 5.3 Hz, 6 H), 2.35 (bs, 1 H), 2.39-2.50 (m, 4 H), 2.83-2.93 (m, 2 H), 3.07 (s, 2 H), 6.47 (bs, 1 H) and 7.29 (bs, 1 H).

### Reference Example 48

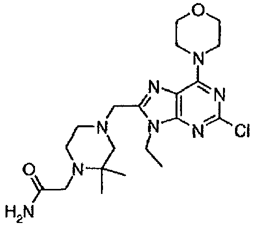

### 2-[4-(2-Chloro-9-ethyl-6-morpholin-4-yl-9H-purin-8-ylmethyl)-2,2-dimethyl-piperazin-1-yl]-acetamide

Prepared according to the method used in the preparation of 2-chloro-8-[4-(3,3-difluoro-azetidin-1-yl)-piperidin-1-ylmethyl]-9-ethyl-6-morpholin-4-yl-9*H*-purine using 2-(2,2-dimethyl-piperazin-1-yl)-acetamide in place of 4-(3,3-difluoro-azetidin-1-yl)-piperidine. The title compound was obtained as a yellow solid (290 mg, 95 %).
[M + H]⁺ 451.2

### Reference Example 49

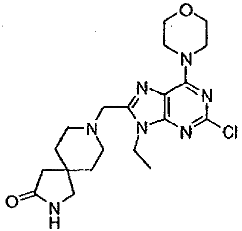

### 8-(2-Chloro-9-ethyl-6-morpholin-4-yl-9H-purin-8-ylmethyl)-2,8-diaza-spiro[4.5]decan-3-one

Prepared according to the method used in the preparation of 2-chloro-8-[4-(3,3-difluoro-azetidin-1-yl)-piperidin-1-ylmethyl]-9-ethyl-6-morpholin-4-yl-9*H*-purine using 2,8-diaza-spiro[4.5]decan-3-one in place of 4-(3,3-difluoro-azetidin-1-yl)-piperidine. The title compound was obtained as a white solid (217 mg, 74 %).
[M + H]⁺ 434.4

### Reference Example 50

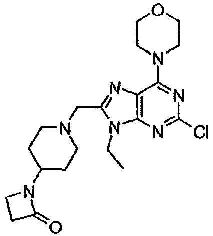

### 1-[1-(2-Chloro-9-ethyl-6-morpholin-4-yl-9H-purin-8-ylmethyl)-piperidin-4-yl]-azetidin-2-one

Prepared according to the method used in the preparation of 2-chloro-8-[4-(3,3-difluoro-azetidin-1-yl)-piperidin-1-ylmethyl]-9-ethyl-6-morpholin-4-yl-9*H*-purine using 1-piperidin-4-yl-azetidin-2-one in place of 4-(3,3-difluoro-azetidin-1-yl)-piperidine. The title compound was obtained as a pale yellow oil (281 mg, 93 %).
[M + H]⁺ 434.3

### Reference Example 51

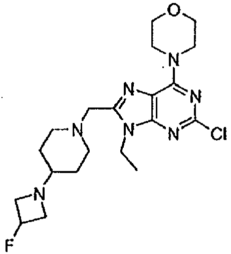

### 2-Chloro-9-ethyl-8-[4-(3-fluoro-azetidin-1-yl)-piperidin-1-ylmethyl]-6-morpholin-4-yl-9H-purine

Prepared according to the method used in the preparation of 2-chloro-8-[4-(3,3-difluoro-azetidin-1-yl)-piperidin-1-ylmethyl]-9-ethyl-6-morpholin-4-yl-9*H*-purine using 4-(3-fluoro-azetidin-1-yl)-piperidine in place of 4-(3,3-difluoro-azetidin-1-yl)-piperidine. The title compound was obtained as a white solid (260 mg, 85 %).
[M + H]⁺ 438.4

### Reference Example 52

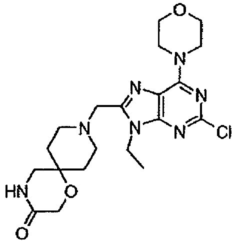

### 9-(2-Chloro-9-ethyl-6-morpholin-4-yl-9H-purin-8-ylmethyl)-1-oxa-4,9-diaza-spiro[5.5]undecan-3-one

Prepared according to the method used in the preparation of 2-chloro-8-[4-(3,3-difluoro-azetidin-1-yl)-piperidin-1-ylmethyl]-9-ethyl-6-morpholin-4-yl-9*H*-purine using 1-oxa-4,9-diaza-spiro[5.5]undecan-3-one in place of 4-(3,3-difluoro-azetidin-1-yl)-piperidine. The title compound was obtained as a white solid (280 mg, 89 %).
¹H NMR (400 MHz, CDCl₃): δ 1.39 (t, J = 7.1 Hz, 3 H), 1.59 (m, 2 H), 1.91 (m, 2 H), 2.48 (m, 2 H), 2.58 (m, 2 H), 3.22 (s, 2 H), 3.69 (s, 2 H), 3.79 (t, J = 4.7 Hz, 4 H), 4.14 (s, 2 H), 4.22-4.31 (m, 6 H) and 5.87 (bs, 1 H).

### Reference Example 53

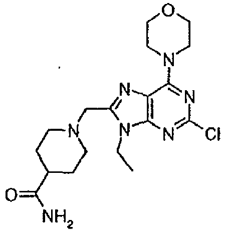

### 1-(2-Chloro-9-ethyl-6-morpholin-4-yl-9H-purin-8-ylmethyl)-piperidine-4-carboxylic acid amide

Prepared according to the method used in the preparation of 2-chloro-8-[4-(3,3-difluoro-azetidin-1-yl)-piperidin-1-ylmethyl]-9-ethyl-6-morpholin-4-yl-9*H*-purine using piperidine-4-carboxylic acid amide in place of 4-(3,3-difluoro-azetidin-1-yl)-piperidine. The title compound was obtained as a white solid (102 mg, 74 %).
[M + H]⁺ 408.4

### Reference Example 54

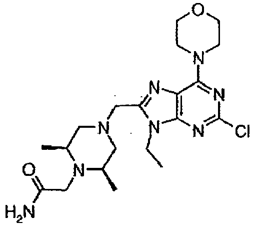

### 2-[(cis)-4-(2-Chloro-9-ethyl-6-morpholin-4-yl-9H-purin-8-ylmethyl)-2,6-dimethyl-piperazin-1-yl]-acetamide

Prepared according to the method used in the preparation of 2-[4-(2-chloro-9-ethyl-6-morpholin-4-yl-9*H*-purin-8-ylmethyl)-piperazin-1-yl]-isobutyramide using 2-((*cis*)-2,6-dimethyl-piperazin-1-yl)-acetamide in place of 2-piperazin-1-yl-isobutyramide. The title compound was obtained as a white foam (300 mg, 76 %).
[M + H]⁺ 451.2

### Reference Example 55

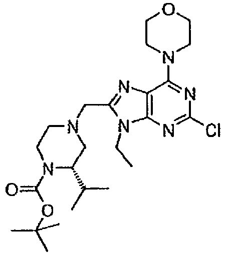

### (S)-4-(2-Chloro-9-ethyl-6-morpholin-4-yl-9H-purin-8-ylmethyl)-2-isopropyl-piperazine-1-carboxylic acid tert-butyl ester

Prepared according to the method used in the preparation of 2-[4-(2-chloro-9-ethyl-6-morpholin-4-yl-9*H*-purin-8-ylmethyl)-piperazin-1-yl]-isobutyramide using (*S*)-2-isopropyl-piperazine-1-carboxylic acid tert-butyl ester in place of 2-piperazin-1-yl-isobutyramide. The title compound was obtained as a yellow foam (340 mg, 97 %).
[M -56 + H]⁺ 451.4

### Reference Example 56

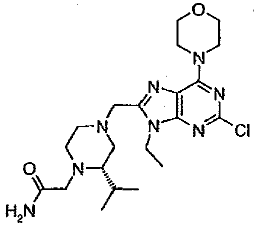

### 2-[(S)-4-(2-Chloro-9-ethyl-6-morpholin-4-yl-9H-purin-8-ylmethyl)-2-isopropyl-piperazin-1-yl]-acetamide

To a solution of (*S*)-4-(2-chloro-9-ethyl-6-morpholin-4-yl-9*H*-purin-8-ylmethyl)-2-isopropyl-piperazine-1-carboxylic acid tert-butyl ester (340 mg, 0.67 mmol) in DCM (25 mL) was added TFA (5 mL) and the solution was stirred at RT for 2.5 h. The reaction mixture was loaded onto an Isolute® SCX-2 cartridge, washed with MeOH then eluted with 2 M NH₃ in MeOH to give 2-chloro-9-ethyl-8-((*S*)-3-isopropyl-piperazin-1-ylmethyl)-6-morpholin-4-yl-9H-purine as a yellow oil. To a solution of 2-chloro-9-ethyl-8-((*S*)-3-isopropyl-piperazin-1-ylmethyl)-6-morpholin-4-yl-9*H*-purine in DMF (25 mL) were added 2-bromoacetamide (277 mg, 2.0 mmol) and potassium carbonate (277 mg, 2.0 mmol). The resulting mixture was heated at 65 °C for 27 h, then allowed to cool to RT. The reaction mixture was partitioned between DCM and water. The organic layer was separated, washed with water and dried (Na₂SO₄), then concentrated *in vacuo* to give the title compound as a yellow oil (236 mg, 76 %).
¹H NMR (400 MHz, CDCl₃): δ 0.86 (d, J = 6.7 Hz, 3 H), 0.93 (d, J = 6.7 Hz, 3 H), 1.42 (t, J = 7.2 Hz, 3 H), 2.10 (m, 1 H), 2.14-2.36 (m, 3 H), 2.38-2.52 (m, 1 H), 2.64 (m, 2 H), 2.83 (d, J = 16.9 Hz, 1 H), 2.86-2.98 (m, 1 H), 3.45 (d, J = 16.9 Hz, 1 H), 3.60-3.73 (m, 2 H), 3.81 (t, J = 4.7 Hz, 4 H), 4.22-4.37 (m, 6 H), 5.68 (bs, 1 H) and 7.11 (bs, 1 H).

### Reference Example 57

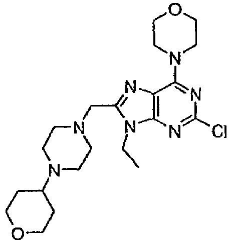

### 2-Chloro-9-ethyl-6-morpliolin-4-yl-8-[4-(tetrahydro-pyran-4-yl)-piperazin-1-ylmethyl]-9H-purine

Prepared according to the method used in the preparation of 2-[4-(2-chloro-9-ethyl-6-morpholin-4-yl-9*H*-purin-8-ylmethyl)-piperazin-1-yl]-isobutyramide using 1-(tetrahydro-pyran-4-yl)-piperazine in place of 2-piperazin-1-yl-isobutyramide. The title compound was obtained as a white foam (568 mg, 96 %).
[M + H]⁺ 450.2

### Reference Example 58

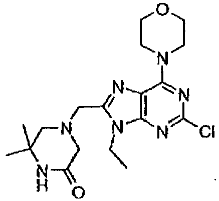

### 4-(2-Chloro-9-ethyl-6-morpholin-4-yl-9H-purin-8-ylmethyl)-6,6-dimethyl-piperazin-2-one

Prepared according to the method used in the preparation of 2-[4-(2-chloro-9-ethyl-6-morpholin-4-yl-9*H*-purin-8-ylmethyl)-piperazin-1-yl]-isobutyramide using 6,6-dimethyl-piperazin-2-one in place of 2-piperazin-1-yl-isobutyramide. The title compound was obtained as a white foam (110 mg, 40 %).
[M + H]⁺ 408.2

### Reference Example 59

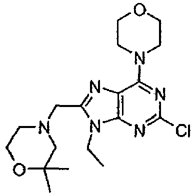

### 2-Chloro-8-(2,2-dimethyl-morpholin-4-ylmethyl)-9-ethyl-6-morpholin-4-yl-9H-purine

Prepared according to the method used in the preparation of 2-[4-(2-chloro-9-ethyl-6-morpholin-4-yl-9*H*-purin-8-ylmethyl)-piperazin-1-yl]-isobutyramide using 2,2-dimethyl-morpholine in place of 2-piperazin-1-yl-isobutyramide. The title compound was obtained as a white foam (232 mg, 87 %).
¹H NMR (400 MHz, CDCl₃): δ 1.21 (s, 6 H), 1.42 (t, J = 7.2 Hz, 3 H), 2.27 (s, 2 H), 2.38 (m, 2 H), 3.61 (s, 2 H), 3.67-3.72 (m, 2 H), 3.76-3.81 (m, 4 H), 4.25 (m, 4 H) and 4.33 (q, J = 7.2 Hz, 2 H).

### Reference Example 60

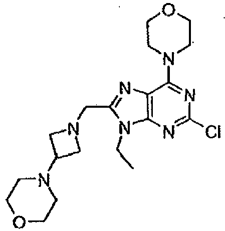

### 2-Chloro-9-ethyl-6-morpholin-4-yl-8-(3-morpholin-4-yl-azetidin-1-ylmethyl)-9H-purine

Prepared according to the method used in the preparation of 2-chloro-8-[4-(3,3-difluoro-azetidin-1-yl)-piperidin-1-ylmethyl]-9-ethyl-6-morpholin-4-yl-9*H*-purine using 4-azetidin-3-yl-morpholine in place of 4-(3,3-difluoro-azetidin-1-yl)-piperidine. The title compound was obtained as a colourless oil (179 mg, 59 %).
[M + H]⁺ 422.4

### Reference Example 61

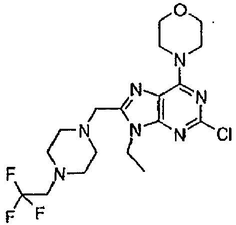

### 2-Chloro-9-ethyl-6-morpholin-4-yl-8-[4-(2,2,2-trifluoro-ethyl)-piperazin-1-ylmethyl]-9H-purine

Prepared according to the method used in the preparation of 2-chloro-8-[4-(3,3-difluoro-azetidin-1-yl)-piperidin-1-ylmethyl]-9-ethyl-6-morpholin-4-yl-9*H*-purine using 1-(2,2,2-trifluoro-ethyl)-piperazine in place of 4-(3,3-difluoro-azetidin-1-yl)-piperidine. The title compound was obtained as a white solid (420 mg, 93 %).
[M + H]⁺ 448.4

### Reference Example 62

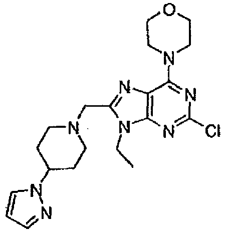

### 2-Chloro-9-ethyl-6-morpholin-4-yl-8-(4-pyrazol-1-yl-piperidin-1-ylmethyl)-9H-purine

Prepared according to the method used in the preparation of 2-chloro-8-[4-(3,3-difluoro-azetidin-1-yl)-piperidin-1-ylmethyl]-9-ethyl-6-morpholin-4-yl-9*H*-purine using 4-pyrazol-1-yl-piperidine in place of 4-(3,3-difluoro-azetidin-1-yl)-piperidine. The title compound was obtained as a white solid (407 mg, 94 %).
[M + H]⁺ 431.4

### Reference Example 63

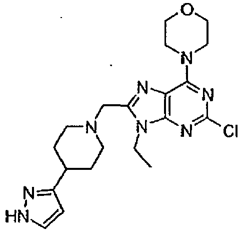

### 2-Chloro-9-ethyl-6-morpholin-4-yl-8-[4-(1H-pyrazol-3-yl)-piperidin-1-ylmethyl]-9H-purine

Prepared according to the method used in the preparation of 2-chloro-8-[4-(3,3-difluoro-azetidin-1-yl)-piperidin-1-ylmethyl]-9-ethyl-6-morpholin-4-yl-9*H*-purine using 4-(1*H*-pyrazol-3-yl)-piperidine in place of 4-(3,3-difluoro-azetidin-1-yl)-piperidine. The title compound was obtained as a white solid (215 mg, 59 %).
[M + H]⁺ 431.4

### Reference Example 64

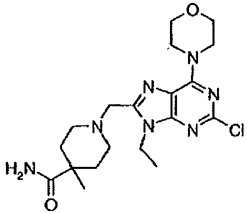

### 1-(2-Chloro-9-ethyl-6-morpholin-4-yl-9H-purin-8-ylmethyl)-4-methyl-piperidine-4-carboxylic acid amide

Prepared according to the method used in the preparation of 2-chloro-8-[4-(3,3-difluoro-azetidin-1-yl)-piperidin-1-ylmethyl]-9-ethyl-6-morpholin-4-yl-9*H*-purine using 4-methyl-piperidine-4-carboxylic acid amide in place of 4-(3,3-difluoro-azetidin-1-yl)-piperidine. The title compound was obtained as a colourless oil (74 mg, 44 %).
[M + H]⁺ 422.4

### Reference Example 65

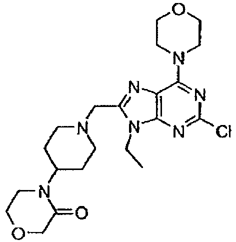

### 4-[1-(2-Chloro-9-ethyl-6-morpholin-4-yl-9H-purin-8-ylmethyl)-piperidin-4-yl]-morpholin-3-one

Prepared according to the method used in the preparation of 2-chloro-8-[4-(3,3-difluoro-azetidin-1-yl)-piperidin-1-ylmethyl]-9-ethyl-6-morpholin-4-yl-9*H*-purine using 4-piperidin-4-yl-morpholin-3-one in place of 4-(3,3-difluoro-azetidin-1-yl)-piperidine. The title compound was obtained as a colourless oil (161 mg, 47 %).
[M + H]⁺ 464.4

### Reference Example 66

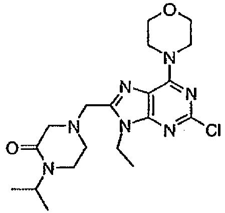

### 4-(2-Chloro-9-ethyl-6-morpholin-4-yl-9H-purin-8-ylmethyl)-1-isopropyl-piperazin-2-one

Prepared according to the method used in the preparation of 2-chloro-8-[4-(3,3-difluoro-azetidin-1-yl)-piperidin-1-ylmethyl]-9-ethyl-6-morpholin-4-yl-9*H*-purine using 1-isopropyl-piperazin-2-one in place of 4-(3,3-difluoro-azetidin-1-yl)-piperidine. The title compound was obtained as a colourless oil (370 mg, 79 %).
[M + H]⁺ 422.3

### Reference Example 67

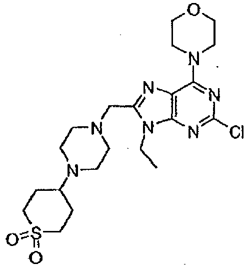

### 2-Chloro-8-[4-(1,1-dioxo-thiopyran-4-yl)-piperazin-1-ylmethyl]-9-ethyl-6-morpholin-4-yl-9H-purine

Prepared according to the method used in the preparation of 2-[4-(2-chloro-9-ethyl-6-morpholin-4-yl-9*H*-purin-8-ylmethyl)-piperazin-1-yl]-isobutyramide using 1-(1,1-dioxo-thiopyran-4-yl)-piperazine in place of 2-piperazin-1-yl-isobutyramide. The title compound was obtained as a white solid (291 mg, 85 %).
[M + H]⁺ 498.3

### Reference Example 68

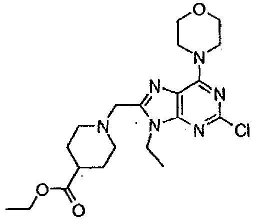

### 1-(2-Chloro-9-ethyl-6-morpholin-4-yl-9H-purin-8-ylmethyl)-piperidine-4-carboxylic acid ethyl ester

Prepared according to the method used in the preparation of 2-[4-(2-chloro-9-ethyl-6-morpholin-4-yl-9*H*-purin-8-ylmethyl)-piperazin-1-yl]-isobutyramide using piperidine-4-carboxylic acid ethyl ester in place of 2-piperazin-1-yl-isobutyramide. The title compound was obtained as a white solid (129 mg, 66 %).
[M + H]⁺ 437.3

### Reference Example 69

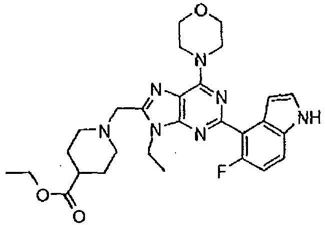

### 1-[9-Ethyl-2-(5-fluoro-1H-indol-4-yl)-6-morpholin-4-yl-9H-purin-8-ylmethyl]-piperidine-4-carboxylic acid ethyl ester

Prepared by using Suzuki coupling method G. The title compound was obtained as a yellow oil (30 mg, 19 %).
[M + H]⁺ 536.4

### Reference Example 70

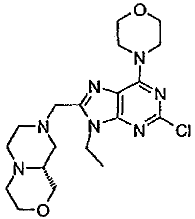

### (R)-8-(2-Chloro-9-ethyl-6-morpholin-4-yl-9H-purin-8-ylmethyl)-octahydro-pyrazino[2,1-c][1,4]oxazine

Prepared according to the method used in the preparation of 2-[4-(2-chloro-9-ethyl-6-morpholin-4-yl-9*H*-purin-8-ylmethyl)-piperazin-1-yl]-isobutyramide using (*R*)-octahydro-pyrazino[2,1-*c*][1,4]oxazine in place of 2-piperazin-1-yl-isobutyramide. The title compound was obtained as a cream solid (315 mg, 98 %).
[M + H]⁺ 422.2

### Reference Example 71

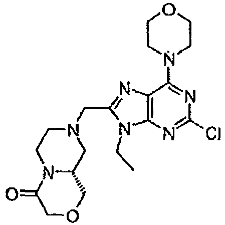

### (R)-8-(2-Chloro-9-ethyl-6-morpholin-4-yl-9H-purin-8-ylmethyl)-hexahydro-pyrazino[2,1-c][1,4]oxazin-4-one

Prepared according to the method used in the preparation of 2-[4-(2-chloro-9-ethyl-6-morpholin-4-yl-9*H*-purin-8-ylmethyl)-piperazin-1-yl]-isobutyramide using (*R*)-hexahydro-pyrazino[2,1-*c*][1,4]oxazin-4-one in place of 2-piperazin-1-yl-isobutyramide. The title compound was obtained as a cream solid (162 mg, 76 %).
[M + H]⁺ 436.4

### Reference Example 72

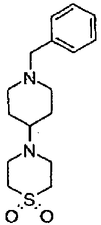

### 4-(1-Benzyl-piperidin-4-yl)-thiomorpholine 1,1-dioxide

To a refluxing solution of 1-benzylpiperidin-4-ylamine (3.98 g, 20.92 mmol) in *iso-*propyl alcohol (15 mL) was added a solution of ethenesulfonyl-ethene (2.47 g, 20.92 mmol) in *iso*-propyl alcohol (15 mL) dropwise. The resulting solution was heated at reflux for 3.5 h, then allowed to cool to RT and concentrated *in vacuo.* The resultant residue was purified by column chromatography to give the title compound as a pale yellow solid (2.93 g, 45 %).
¹H NMR (400 MHz, CDCl₃): δ 1.51-1.72 (m, 4 H), 1.96 (td, J = 11.6, 2.5 Hz, 2 H), 2.43-2.52 (m, 1 H), 2.95 (d, J = 11.6 Hz, 2 H), 3.04 (m, 8 H), 3.49 (s, 2 H) and 7.23-7.34 (m, 5 H).

### Reference Example 73

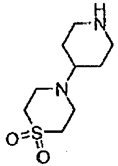

### 4-Piperidin-4-yl-thiomorpholine 1,1-dioxide

To a solution of 4-(1-benzyl-piperidin-4-yl)-thiomorpholine 1,1-dioxide (676 mg, 2.19 mmol) in a mixture of glacial acetic acid (5 mL) and IMS (25 mL) was added palladium hydroxide on carbon (500 mg) under an nitrogen atmosphere. The system was evacuated and back-filled with hydrogen, then stirred at RT for 3 h under an hydrogen atmosphere. The reaction mixture was filtered through Celite and concentrated *in vacuo*. The resultant residue was loaded onto an Isolute® SCX-2 cartridge, washed with MeOH then eluted with 2 M NH₃ in MeOH to give the title compound as white solid (391 mg, 82 %).
[M + H]⁺ 219.2

### Reference Example 74

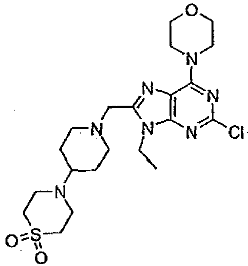

### 2-Chloro-8-[4-(1,1-dioxothiomorpholin-4-yl)-piperidin-1-ylmethyl]-9-ethyl-6-morpholin-4-yl-9H-purine

Prepared according to the method used in the preparation of 2-[4-(2-chloro-9-ethyl-6-morpholin-4-yl-9*H*-purin-8-ylmethyl)-piperazin-1-yl]-isobutyramide using 4-piperidin-4-yl-thiomorpholine 1,1-dioxide in place of 2-piperazin-1-yl-isobutyramide. The title compound was obtained as a white solid (665 mg, 82 %).
[M + H]⁺ 498.3

### Reference Example 75

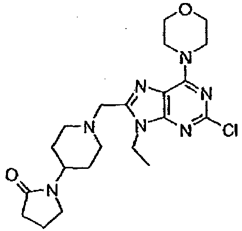

### 1-[1-(2-Chloro-9-ethyl-6-morpholin-4-yl-9H-purin-8-ylmethyl)-piperidin-4-yl]-pyrrolidin-2-one

Prepared according to the method used in the preparation of 2-chloro-8-[4-(3,3-difluoro-azetidin-1-yl)-piperidin-1-ylmethyl]-9-ethyl-6-morpholin-4-yl-9*H*-purine using 1-piperidin-4-yl-pyrrolidin-2-one in place of 4-(3,3-difluoro-azetidin-1-yl)-piperidine. The title compound was obtained as a colourless oil (220 mg, 81 %).
[M + H]⁺ 448.5

### Reference Example 76

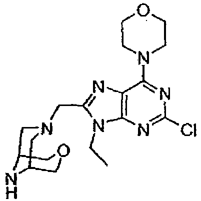

### 7-(2-Chloro-9-ethyl-6-morpholin-4-yl-9H-purin-8-ylmethyl)-3-oxa-7,9-diaza-bicyclo[3.3.1]nonane

To a solution of 2-chloro-9-ethyl-6-morpholin-4-yl-9*H*-purine-8-carbaldehyde (159 mg, 0.54 mmol) in DCE (17 mL) was added 3-oxa-7,9-diaza-bicyclo[3.3.1]nonane (130 mg, 0.65 mmol). The mixture was stirred at RT for 1 h then sodium triacetoxyborohydride (176 mg, 0.83 mmol) was added and stirring was continued for 17 h. The reaction mixture was added to water and loaded onto an Isolute® SCX-2 cartridge. The cartridge was washed with MeOH, and the product then eluted with 2 M NH₃ in MeOH. The resultant residue was purified by column chromatography to give the title compound as a white solid (50 mg, 23 %).
[M + H]⁺ 408.4

### Reference Example 77

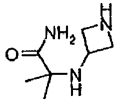

### 2-(Azetidin-3-ylamino)-2-methyl-propionamide

To a solution of 3-amino-azetidine-1-carboxylic acid tert-butyl ester (1.0 g, 5.8 mmol) in dioxane (2 mL) was added 4 M HCl in dioxane (1.5 mL, 5.8 mml) at 0 °C. The mixture was stirred at 0 °C for 5 min, then concentrated *in vacuo.* To a solution of the resulting residue in water (15 mL) was added acetone (506 mg, 8.7 mmol) and NaCN.(285 mg, 5.8 mmol). The reaction mixture was stirred at RT for 20 h, then partitioned between DCM and water. The organic layer was passed through a hydrophobic frit and concentrated *in vacuo* to give 3-[(cyano-dimethyl-methyl)-amino]-azetidine-1-carboxylic acid tert-butyl ester (1.3 g, 94 %) as a colourless oil. To a solution of 3-[(cyano-dimethyl-methyl)-amino]-azetidine-1-carboxylic acid tert-butyl ester (1.3 g, 5.4 mmol) in DMSO (25 mL) was added K₂CO₃ (150 mg, 1.1 mmol) and hydrogen peroxide solution (30 wt. % in H₂O, 3 mL). The reaction mixture was heated at 45 °C for 3 days, then allowed to cool and partitioned between EtOAc and water. The organic layer was separated, dried (MgSO₄) and concentrated *in vacuo* to give 3-(1-carbamoyl-1-methyl-ethylamino)-azetidine-1-carboxylic acid tert-butyl ester (1.24 g, 89 %) as a colourless oil. To a solution of 3-(1-carbamoyl-1-methyl-ethylamino)-azetidine-1-carboxylic acid tert-butyl ester (606 mg, 2.4 mmol) in DCM (5 mL) was added TFA (3 mL) and the resulting mixture was stirred at RT for 4 h. The reaction mixture was loaded onto an Isolute® SCX-2 cartridge, washed with MeOH then eluted with 2 M NH₃ in MeOH to give the title compound as a colourless oil (190 mg, 51 %).
¹H NMR (400 MHz, CDCl₃): δ 1.22 (s, 6 H), 3.26-3.39 (m, 3 H), 3.61-3.72 (m, 3 H), 6.06 (bs, 1 H) and 7.00 (bs, 1 H).

### Reference Example 78

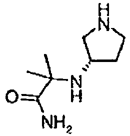

### 2-Methyl-2-((S)-pyrrolidin-3-ylamino)-propionamide

Prepared according to the method used in the preparation of 2-(azetidin-3-ylamino)-2-methyl-propionamide using (*S*)-3-amino-pyrrolidine-1-carboxylic acid tert-butyl ester in place of 3-amino-azetidine-1-carboxylic acid tert-butyl ester. The title compound was obtained as a colourless oil (180 mg, 44 %). [M+H]⁺ 172.2

### Reference Example 79

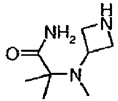

### 2-(Azetidin-3-yl-methyl-amino)-2-methyl-propionamide

To a solution of 3-(1-carbamoyl-1-methyl-ethylamino)-azetidine-1-carboxylic acid tert-butyl ester) in DCE (15 mL) was added formaldehyde (37 wt. % in H₂O, 0.29 mL). The mixture was stirred at RT for 1 h then sodium triacetoxyborohydride (620 mg, 2.9 mmol) was added and stirring was continued for 16 h. The reaction mixture was loaded onto an Isolute® SCX-2 cartridge, washed with MeOH then eluted with 2 M NH₃ in MeOH to give 3-[(1-carbamoyl-1-methyl-ethyl)-methyl-amino]-azetidine-1-carboxylic acid tert-butyl ester (340 mg, 65 %). To a solution of 3-[(1-carbamoyl-1-methyl-ethyl)-methyl-amino]-azetidine-1-carboxylic acid tert-butyl ester (340 mg, 1.3 mmol) in DCM (5 mL) was added TFA (3 mL) and the resulting mixture was stirred at RT for 2 h. The reaction mixture was loaded onto an Isolute® SCX-2 cartridge, washed with MeOH then eluted with 2 M NH₃ in MeOH to give the title compound as a colourless oil (170 mg, 79 %).
¹H NMR (400 MHz, CDCl₃): δ 1.13 (s, 6 H), 2.20 (s, 3 H), 3.42-3.57 (m, 3 H), 3.67-3.77 (m, 3 H), 5.87 (bs, 1 H) and 6.98 (bs, 1 H).

### Reference Example 80

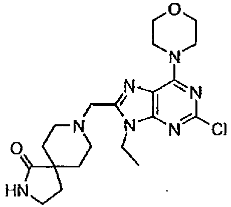

### 8-(2-Chloro-9-ethyl-6-morpholin-4-yl-9H-purin-8-ylmethyl)-2,8-diazaspiro[4.5]decan-1-one

To a solution of 2,8-diaza-spiro[4.5]decan-1-one (120 mg, 0.78 mmol) in DCE (15 mL) was added 2-chloro-9-ethyl-6-morpholin-4-yl-9*H*-purine-8-carbaldehyde (180 mg, 0.61 mmol). The mixture was stirred at RT for 2 h then sodium triacetoxyborohydride (190 mg, 0.90 mmol) was added and stirring was continued for 22 h. The reaction mixture was loaded onto an Isolute® SCX-2 cartridge, washed with MeOH then eluted with 2 M NH₃ in MeOH. The resultant residue was purified by column chromatography to give the title compound as a white solid (175 mg, 52 %).
[M+H]⁺ 434.4

### Reference Example 81

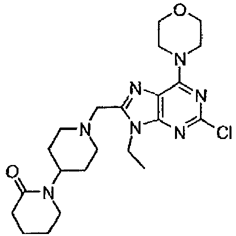

### 1'-(2-Chloro-9-ethyl-6-morpholin-4-yl-9H-purin-8-ylmethyl)-[1,4']bipiperidinyl-2-one

Prepared according to the method used in the preparation of 8-(2-chloro-9-ethyl-6-morpholin-4-yl-9*H*-purin-8-ylmethyl)-2,8-diazaspiro[4.5]decan-1-one using [1,4']bipiperidiny3-2-one in place of 2,8-diaza-spiro[4.5]decan-1-one. The title compound was obtained as a colourless oil (260 mg, 83 %).
[M+H]⁺ 462.4

### Reference Example 82

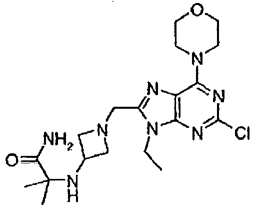

### 2-[1-(2-Chloro-9-ethyl-6-morpholin-4-yl-9H-purin-8-ylmethyl)-azetidin-3-ylamino]-2-methyl-propionamide

Prepared according to the method used in the preparation of 8-(2-chloro-9-ethyl-6-morpholin-4-yl-9*H*-purin-8-ylmethyl)-2,8-diazaspiro[4.5]decan-1-one using 2-(azetidin-3-ylamino)-2-methyl-propionamide in place of 2,8-diaza-spiro[4.5]decan-1-one. The title compound was obtained as a colourless oil (240 mg, 68 %)
[M+H]⁺ 437.3

### Reference Example 83

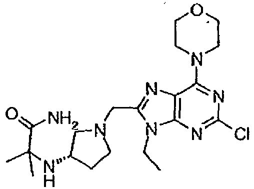

### 2-[(S)-1-(2-Chloro-9-ethyl-6-morpholin-4-yl-9H-purin-8-ylmethyl)-pyrrolidin-3-ylamino]-2-methyl-propionamide

Prepared according to the method used in the preparation of 8-(2-chloro-9-ethyl-6-morpholin-4-yl-9*H*-purin-8-ylmethyl)-2,8-diazaspiro[4.5]decan-1-one using 2-methyl-2-((S)-pyrrolidin-3-ylamino)-propionamide in place of 2,8-diaza-spiro[4.5]decan-1-one. The title compound was obtained as a red oil (148 mg, 45 %)
[M+H]⁺ 451.3

### Reference Example 84

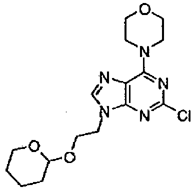

### 2-Chloro-6-morpholin-4-yl-9-[2-(tetrahydro-pyran-2-yloxy)-ethyl]-9H-purine

To a solution of 2-chloro-6-morpholin-4-yl-9*H*-purine (1.00 g, 4.17 mmol) in DMF (30 mL) were added 2-(2-bromo-ethoxy)-tetrahydro-pyran (1.26 mL, 8.34 mmol) and potassium carbonate (1.73 g, 12.52 mmol). The reaction mixture was heated at 50 °C for 7 h, then allowed to cool to RT and quenched with water (60 mL). The resultant white precipitate was collected by filtration and washed with water and diethyl ether, then dried under vacuum to give the title compound (1.26 g, 82 %).
[M + H]⁺ 368.4

### Reference Example 85

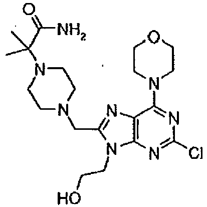

### 2-{4-[2-Chloro-9-(2-hydroxy-ethyl)-6-morpholin-4-yl-9H-purin-8-ylmethyl]-piperazin-1-yl}-isobutyramide

To a solution of 2-chloro-6-morpholin-4-yl-9-[2-(tetrahydro-pyran-2-yloxy)-ethyl]-9*H*-purine (100 mg, 0.27 mmol) in anhydrous THF (5 mL) was added a solution of lithium hexamethyldisilazide in THF (1 M, 0.41 mL, 0.41 mmol) at - 78 °C. The resulting mixture was stirred for 30 min, and then anhydrous DMF (0.15 mL, 1.94 mmol) was added dropwise. The reaction mixture was stirred for 30 min, then allowed to warm to RT and stirred for a further 30 min. The reaction mixture was cooled to -78 °C, quenched with an aqueous solution of ammonium chloride (1 M, 10 mL), and then partitioned between water and DCM. The organic layer was separated, passed through a hydrophobic frit and concentrated *in vacuo*. The resultant residue was dissolved in DCE (15 mL) and 2-piperazin-1-yl-isobutyramide (46 mg, 0.27 mmol) added before the resulting mixture was stirred at RT. Sodium triacetoxyborohydride (111 mg, 0.52 mmol) was added after 1 h and stirring was continued for a further 2 h. The reaction mixture was loaded onto an Isolute® SCX-2 cartridge, washed with MeOH then eluted with 2 M NH₃ in MeOH. The resultant residue was purified by column chromatography to give the title compound as a colourless oil (96 mg, 76 % over 2 steps).
[M + H]⁺ 467.5

### Reference Example 86

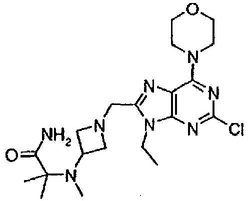

### 2-{[1-(2-Chloro-9-ethyl-6-morpholin-4-yl-9H-purin-8-ylmethyl)-azetidin-3-yl]-methyl-amino}-2-methyl-propionamide

To a solution of 2-chloro-9-ethyl-6-morpholin-4-yl-9*H*-purine-8-carbaldehyde (230 mg, 0.78 mmol) in DCE (15 mL) was added 2-(azetidin-3-yl-methyl-amino)-2-methyl-propionamide (170 mg, 0.99 mmol). The mixture was stirred at RT for 45 min then sodium triacetoxyborohydride (250 mg, 1.17 mmol) was added and stirring was continued for 60 h. The reaction mixture was loaded onto an Isolute® SCX-2 cartridge, washed with MeOH then eluted with 2 M NH₃ in MeOH. The resultant residue was purified by column chromatography to give the title compound as a colourless oil (280 mg, 80 %).
[M + H]⁺ 451.4

### Reference Example 87

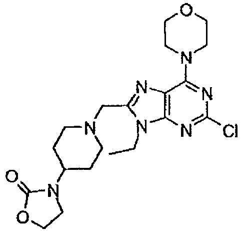

### 3-[1-(2-Chloro-9-ethyl-6-morpholin-4-yl-9H-purin-8-ylmethyl)-piperidin-4-yl]-oxazolidin-2-one

To a solution of *N*-Boc-4-aminopiperidine (1.0 g) in acetonitrile was added potassium carbonate (1.72 g) followed by 2-chloroethyl chloroformate (0.892 g) dropwise and the mixture stirred at RT for 1 h and then heated under reflux for 12 h. The mixture was cooled, partitioned between water and CH₂Cl₂, the organic layers separated and dried (MgSO₄). Purification by column chromatography eluting with 5%→10% CH₂Cl₂-MeOH + NH₃ yielded 4-(2-oxo-oxazolidin-3-yl)-piperidine-1-carboxylic acid *tert*-butyl ester as a colourless solid (535 mg). A mixture of this and 4 M HCl in dioxane (5 mL) in CH₂Cl₂ (2 mL) was stirred at RT for 12 h and the solvent evaporated. The crude product was passed through an SCX-2 cartridge to yield 1-piperidin-4-yl-imidazolidin-2-one (340 mg).

2-Chloro-9-ethyl-6-morpholin-4-yl-9*H*-purine-8-carbaldehyde (270 mg) and 1-piperidin-4-yl-imidazolidin-2-one (203 mg) were reacted together using the standard reductive amination conditions. The title compound was isolated as a white solid (275 mg). δ_{H}(400 MHz, CDCl₃) 1.45 (t, 3H), 1.69 (m, 2H), 1.81 (m, 2H), 2.27 (m, 2H), 2.95 (m, 2H), 3.54 (dd, J = 7.3, 8.7, 2H), 3.71 (s, 2H), 3.77 (1H, m)3.84 (t, J = 4.9, 4H), 4.29 - 4.37 (m, 6H).

### Reference Example 88

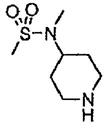

### N-Methyl-N-piperidin-4-yl-methanesulfonamide

To a solution of 1-BOC-4-piperidone (1.0 g) in methanol (10 mL) was added a solution of freshly prepared methylamine in methanol (1.0 mL). The reaction mixture was stirred for 1 hour and then sodium cyanoborohydride (0.315 g) was added. After stirring for 24 hours the reaction mixture was then diluted with dichloromethane, washed with sodium bicarbonate solution, dried (MgSO₄) and the solvent removed *in vacuo*. The residue was purified by flash chromatography to yield 4-methylamino-piperidine-1-carboxylic acid *tert-*butyl ester (0.60g).

To a solution of 4-methylamino-piperidine-1-carboxylic acid *tert*-butyl ester (0.59g) in dichloromethane (10 mL) was added triethylamine (0.42 mL) followed by methane sulfonyl chloride (0.23 mL). After stirring for 3 hours the reaction mixture was then diluted with dichloromethane, washed with sodium bicarbonate solution, dried (MgSO₄) and the solvent removed *in vacuo.* The residue was purified by flash chromatography to yield 4-(methanesulfonyl-methyl-amino)-piperidine-1-carboxylic acid *tert*-butyl ester (0.738 g). Treatment of this compound with HCl in dichloromethane/methanol gave the title compound, which was isolated as the hydrochloride salt (0.57 g).
δ_{H} (400 MHz, d₆-dmso) 1.78 (m, 2H), 1.95 (m, 2H), 2.70 (s, 3H), 2.94 (s, 3H), 2.97 (m, 2H), 3.34 (m, 2H), 3.89 (m, 1H), 8.74 (br s, 2H).

### Reference Example 89

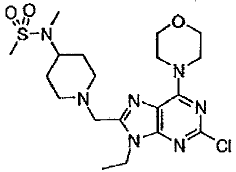

### N-[1-(2-Chloro-9-ethyl-6-morpholin-4-yl-9H-purin-8-ylmethyl)-piperidin-4-yl]-N-methyl-methanesulfonamide

The title compound was prepared under the standard reductive-amination conditions to give a pale yellow solid (0.21g).
δ_{H} (400 MHz, CDCl₃) 1.18 (t, 3H), 1.66 (m, 4H), 2.15 (m, 2H), 2.71 (s, 3H), 2.75 (s, 3H), 2.84 (m, 2H), 3.60 (s, 2H), 3.71 (m, 5H), 4.21 (m, 6H).

### Reference Example 90

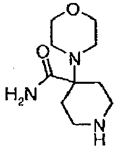

### 4-Morpholin-4-yl-piperidine-4-carboxylic acid amide

To a mixture of 1-BOC-4-piperidone (1.0 g, 5.01 mmol) and morpholine (0.43 mL, 4.93 mmol) in chloroform (5 mL) under nitrogen cooled down to 0 °C was added dropwise trimethylsilylcyanide (0.73 mL, 5.47 mmol). The mixture was warmed to room temperature and stirred overnight. The mixture was partitioned between dichloromethane and water. The combined organic layers were washed with brine, separated and dried (MgSO₄) to yield 4-cyano-4-morpholin-4-yl-piperidine-1-carboxylic acid *tert*-butyl ester (1.19 g). The crude material was stirred in methanol (10 mL) and 1 M sodium hydroxide solution (4.25 mL) was added followed by the dropwise addition of hydrogen peroxide, 30 wt.% solution in water (2.3 mL) at room temperature. The mixture was stirred overnight and then evaporated *in vacuo.* The crude product was purified by column chromatography to yield 4-carbamoyl-4-morpholin-4-yl-piperidine-1-carboxylic acid *tert*-butyl ester (0.54 g).
Treatment of this compound with HCl in dichloromethane/methanol gave the title compound, which was isolated as the dihydrochloride salt (0.49 g).
δ_{H} (400 MHz, d₆-dmso) 2.12 (br m, 4H), 2.87 (br m, 4H), 3.36 (br m, 4H), 3.72 (br m, 4H), 8.76 (br s, 2H).

### Reference Example 91

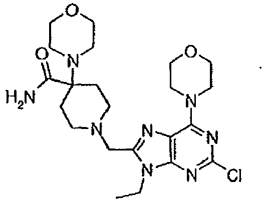

### 1-(2-Chloro-9-ethyl-6-morpholin-4-yl-9H-purin-8-ylmethyl)-4-morpholin-4-yl-piperidine-4-carboxylic acid amide

The title compound was prepared under the standard reductive-amination conditions to give a pale yellow solid (0.21g).
δ_{H}(400 MHz, CDCl₃) 1.44 (t, 3H), 1.83 (m, 4H), 2.58 (m, 6H), 2.76 (m, 2H), 3.71 (m, 6H), 3.83 (t, 4H), 4.31 (m, 6H), 5.22 (br s, 1H), 6.43 (br s, 1H).

### Synthesis of Compounds of formula (I)

### Example 1: {1-[9-Ethyl-2-(1H-indol-4-yl)-6-morpholin-4-yl-9H-purin-8-ylmethyl]-piperidin-4-yl}-dimethyl-amine

A stirred mixture of (1-(2-chloro-9-ethyl-6-morpholin-4-yl-9*H*-purin-8-ylmethyl)-piperidin-4-yl]-dimethyl-amine (Reference Example 9; 82 mg; 0.20 mmol), indole-4-boronic acid (45 mg; 0.28 mmol), PdCl₂(PCy₃)₂ (7.4 mg; 0.01 mmol), K₃PO₄ (0.5 mL of a 1.27 M aqueous solution; 0.64 mmol) and dioxane (1.0 mL) was heated at 125 °C in a microwave for 1.5 h. The product was purified by catch-and-release using an Isolute SCX-2 cartridge followed by flash chromatography (85:15:1 to 80:20:1 CH₂Cl₂/MeOH/NH₄OH as eluent) to afford the title compound as an off-white solid (90 mg; 92 %).
δ_{H} (400 MHz, CDCl₃) 1.57 (t, J = 7.2, 3H), 1.88-1.91 (m, 2H), 2.14-2.20 (m, 3H), 2.35 (br s, 6H), 2.96-2.99 (m, 2H), 3.76 (s, 2H), 3.90-3.92 (m, 4H), 4.41-4.45 (m, 4H), 4.48 (q, J = 7.2, 2H), 7.29-7.35 (m, 2H), 7.50 (d, J = 8.0, 1H), 7.64 (s, 1H), 8.25-8.27 (m, 2H).
[M+H]⁺: 489. This compound is not covered by the claims.

The compounds of Examples 2 to 10 were prepared using an analogues method to that described in Example 1, using the appropriately substituted chloropurine and indole-4-boronic acid starting compounds:

### Example 2 {1-[9-Ethyl-2-(5-fluoro-1H-indol-4-yl)-6-morpholin-4-yl-9H-purin-8-ylmethyl]-piperidin-4-yl}-dimethyl-amine

Suzuki coupling of [1-(2-chloro-9-ethyl-6-morpholin-4-yl-9*H*-purin-8-ylmethyl)-piperidin-4-yl]-dimethyl-amine (Reference Example 9) and 1-(*tert*-butyl-dimethyl-silanyl)-5-fluoro-4-(4,4,5,5-tetramethyl-[1,3,2]dioxaborolan-2-yl)-1*H*-indole (Reference Example 1) afforded the title compound as an off-white solid (17 mg).
δ_{H} (400 MHz, CDCl₃) 1.53 (t, J = 7.2, 3H), 1.53-1.68 (m, 4H), 1.85-1.88 (m, 2H), 2.14-2.20 (m, 3H), 2.31 (s, 6H), 2.96 (br d, J = 11.6), 3.76 (s, 2H), 3.86-3.88 (m, 4H), 4.39 (br s, 4H) overlapping 4.43 (q, J = 7.2, 2H), 6.98 (br s, 1H), 7.07 (dd, J = 10.8 and 8.8, 1H), 7.28-7.30 (m, 1H), 7.37 (dd, J = 8.8 and 4.0, 1H), 8.22 (br s, 1H).
[M+H]⁺: 507. This compound is not covered by the claims.

### Example 3 9-Ethyl-2-(5-fluoro-1H-indol-4-yl)-8-[(S)-1-(hexahydro-pyrrolo[1,2-a]pyrazin-2-yl)methyl]-6-morpholin-4-yl-9H-purine

Suzuki coupling of 2-chloro-9-ethyl-8-[(*S*)-1-(hexahydro-pyrrolo[1,2-a]pyrazin-2-yl)methyl]-6-morpholin-4-yl-9*H*-purine (Reference Example 10) and 1-(*tert*-butyl-dimethyl-silanyl)-5-fluoro-4-(4,4,5,5-tetramethyl-[1,3,2]dioxaborolan-2-yl)-1*H*-indole (Reference Example 3) afforded the title compound as a pale yellow solid (61 mg).
δ_{H} (400 MHz, CDCl₃) 1.39-1.88 (m, 4H) overlapping 1.54 (t, J = 7.2, 3H), 2.01-2.50 (m, 5H), 2.84-3.15 (m, 4H), 3.79-3.89 (m, 6H), 4.38 (br s, 4H) overlapping 4.43 (q, J = 7.2, 2H), 6.98 (br s, 1H), 7.07 (dd, J = 10.8 and 8.8, 1H), 7.28-7.30 (m, 1H), 7.37 (dd, J = 8.8 and 4.0, 1H), 8.22 (br s, 1H).
[M+H]⁺: 505. This compound is not covered by the claims.

### Example 4 9-Ethyl-8-[(S)-1-(hexahydro-pyrrolo[1,2-a]pyrazin-2-yl)methyl]-2-(1H-indol-4-yl)-6-morpholin-4-yl-9H-purine

Suzuki coupling of 2-chloro-9-ethyl-8-[(*S*)-1-(hexahydro-pyrrolo[1,2-a]pyrazin-2-yl)methyl]-6-morpholin-4-yl-9*H*-purine (Reference Example 10) and indole-4-boronic acid afforded the title compound as a pale yellow solid (97 mg).
δ_{H} (400 MHz, CDCl₃) 1.37-1.48 (m, 1H), 1.57 (t, J = 7.2, 3H), 1.67-1.89 (m, 3H), 2.04-2.49 (m, 5H), 3.79-3.93 (m, 6H), 4.42-4.45 (m, 4H) overlapping 4.48 (q, J = 7.2, 3H), 7.30-7.35 (m, 2H), 7.49 (d, J = 8.0, 1H), 7.64 (br s, 1H), 8.26 (d, J = 8.0, 1H) overlapping 8.27 (br s, 1 H).
[M+H]⁺: 487. This compound is not covered by the claims.

### Example 5 8-(4-Azetidin-1-yl-piperidin-1-ylmethyl)-9-ethyl-2-(5-fluoro-1H-indol-4-yl)-6-morpholin-4-yl-9H-purine

Suzuki coupling of 8-(4-azetidin-1-yl-piperidin-1-ylmethyl)-2-chloro-9-ethyl-6-morpholin-4-yl-9*H*-purine (Reference Example 12) and 1-(*tert*)-butyl-dimethyl-silanyl)-5-fluoro-4-(4,4,5,5-tetramethyl-[1,3,2]dioxaborolan-2-yl)-1*H*-indole (Reference Example 3) afforded the title compound as a white solid (5 mg).
δ_{H} (400 MHz, CDCl₃) 1.25-1.35 (m, 2H), 1.50 (t, J = 7.2, 3H), 1.51-1.74 (m, 4H), 1.95-2.23 (m, 5H), 2.83-2.86 (m, 2H), 3.18 (t J = 6.8, 2H), 3.75 (s, 2H), 3.84-3.87 (m, 4H), 4.36 (m, 4H) overlapping 4.39 (q, J = 7.2, 2H), 6.96-6.97 (m, 1H), 7.05 (dd, J = 10.8 and 8.8, 1H), 7.28-7.32 (m, 1H), 7.35 (dd, J = 8.8 and 4.0, 1H), 8.18 (br s, 1H).
[M+H]⁺: 519. This compound is not covered by the claims.

### Example 6 9-Ethyl-2-(5-fluoro-1H-indol-4-yl)-6-morpholin-4-yl-8-(4-morpholin-4-yl-piperidin-1-ylmethyl)-9H-purine

Suzuki coupling of 2-chloro-9-ethyl-6-morpholin-4-yl-8-(4-morpholin-4-yl-piperidin-1-ylmethyl)-9*H*-purine (Reference Example 13) and 1-(*tert*-butyl-dimethyl-silanyl)-5-fluoro-4-(4,4,5,5-tetramethyl-[1,3,2]dioxaborolan-2-yl)-1*H*-indole (Reference Example 3) afforded the title compound as a white solid (49 mg).
δ_{H} (400 MHz, CDCl₃) 1.45-1.53 (m, 5H), 1.86 (br d, J = 12.0, 2H), 2.10-2.25 (m, 4H), 2.54 (br s, 4H), 2.94 (br d, J = 12.0, 2H), 3.70-3.73 (m, 6H), 3.82-3.95 (m, 4H), 4.35 (br s, 4H) overlapping 4.39 (q, J = 7.2, 2H), 6.94 (s, 1H), 7.03 (dd, J = 10.0 and 8.8, 1H), 7.24-7.26 (m, 1 H), 7.33 (dd, J = 8.8 and 3.6, 1H), 8.18 (br s, 1 H).
[M+H]⁺: 549. This compound is not covered by the claims.

### Example 7 9-Ethyl-2-(1H-indol-4-yl)-6-morpholin-4-yl-8-(4-morpholin-4-yl-piperidin-1-ylmethyl)-9H-purine

Suzuki coupling of 2-chloro-9-ethyl-6-morpholin-4-yl-8-(4-morpholin-4-yl-piperidin-1-ylmethyl)-9*H*-purine (Reference Example 13) and indole-4-boronic acid afforded the title compound as a pale yellow solid (93 mg).
δ_{H} (400 MHz, CDCl₃) 1.48-1.58 (m, 5H), 1.87-1.90 (m, 2H), 2.13-2.23 (m, 4H), 2.55-2.58 (m, 4H), 2.95-2.98 (m 2H), 3.73-3.76 (m, 6H), 3.89-3.92 (m, 4H), 4.41-4.44 (m, 4H), 4.48 (q, J = 7.2,2H), 7.30-7.35 (m, 2H), 7.49 (d, J = 8.0, 1H), 7.64 (s, 1H), 8.26 (d, J = 8.0, 1H), 8.28 (br s, 1H).
[M+H]⁺: 531. This compound is not covered by the claims.

### Example 8 2-[9-Ethyl-2-(5-fluoro-1H-indol-4-yl)-6-morpholin-4-yl-9H-purin-8-ylmethyl]-1,2,3,4-tetrahydro-isoquinoline

Suzuki coupling of 2-(2-chloro-9-ethyl-6-morpholin-4-yl-9*H*-purin-8-ylmethyl)-1,2,3,4-tetrahydro-isoquinoline (Reference Example 14) and 1-(*tert*-butyl-dimethyl-silanyl)-5-fluoro-4-(4,4,5,5-tetramethyl-[1,3,2]dioxaborolan-2-yl)-1*H*-indole (Reference Example 3) afforded the title compound as a white solid (31 mg).
δ_{H} (400 MHz, CDCl₃) 1.48 (t, J = 7.2, 3H), 2.84-2.93 (m, 4H), 3.77 (s, 2H), 3.88-3.91 (m, 4H), 3.97 (s, 2H), 4.41 (br s, 4H), 4.46 (q, J = 7.2, 2H), 6.98 (s, 1H), 7.04-7.18 (m, 5H), 7.28-7.29 (m, 1H), 7.37 (dd, J = 8.8 and 4.0, 1H), 8.20 (br s, 1H).
[M+H]⁺: 512. This compound is not covered by the claims.

### Ex ample 9 2-[9-Ethyl-2-(1H-indol-4-yl)-6-morpholin-4-yl-9H-purin-8-ylmethyl]-1,2,3,4-tetrahydro-isoquinoline

Suzuki coupling of 2-(2-chloro-9-ethyl-6-morpholin-4-yl-9*H*-purin-8-ylmethyl)-1,2,3,4-tetrahydro-isoquinoline (Reference Example 14) and indole-4-boronic acid afforded the title compound as an off-white solid (64 mg).
δ_{H} (400 MHz, CDCl₃) 1.53 (t, J = 7.2, 3H), 2.83-2.94 (m, 4H), 3.77 (s, 2H), 3.92-3.94 (m, 4H), 3.97 (s, 2H), 4.44-4.50 (m, 4H), 4.51 (q, J = 7.2, 2H), 7.01-7.20 (m, 3H), 7.28-7.34 (m, 3H), 7.49 (d, J = 8.0, 1H), 7.63 (s, 1H), 8.26 (d, J = 8.0, 1H) overlapping 8.27 (br s, 2H).
[M+H]⁺: 494. This compound is not covered by the claims.

### Example 10 2-{4-[9-Ethyl-2-(1H-indol-4-yl)-6-morpholin-4-yl-9H-purin-8-ylmethyl]-piperazin-1-yl}-isobutyramide

Suzuki coupling of 2-[4-(2-chloro-9-ethyl-6-morpholin-4-yl-9H-purin-8-ylmethyl)-piperazin-1-yl]-isobutyramide (Reference Example 16) and indole-4-boronic acid afforded the title compound as a white solid (58 mg).
δ_{H} (400 MHz, CDCl₃) 1.25 (s, 6H), 1.57 (t, J = 7.2, 3H), 2.59 (br s, 8H), 3.79 (s, 2H), 3.90-3.93 (m, 4H), 4.41-4.44 (m, 4H), 4.48 (q, J = 7.2, 2H), 5.20 (br d, J = 5.2, 1 H), 7.13 (br d, J = 5.2, 1H), 7.28-7.36 (m, 3H), 7.50 (d, J = 8.0, 1H), 7.64 (s, 1H), 8.26 (d, J = 8.0, 1H) overlapping 8.27 (br s, 1 H).
[M+H]⁺: 532. This compound is not covered by the claims.

### Example 11 8-[4-(3,3-Difluoro-azetidin-1-yl)-piperidin-1-ylmethyl]-9-ethyl-2-(5-fluoro-1H-indol-4-yl)-6-morpholin-4-yl-9H-purine

Prepared by using Suzuki coupling method C. The title compound was obtained as a colourless oil (70 mg, 53 %).
[M + H]⁺ 555.2
¹H NMR (400 MHz, CDCl₃): δ 1.41 (m, 2 H), 1.48 (t, J = 7.1 Hz, 3 H), 1.66 (m, 3 H), 2.19 (m, 3 H), 2.86 (m, 2 H), 3.55 (t, J = 11.8 Hz, 4 H), 3.76 (m, 1 H), 3.84 (t, J = 4.7 Hz, 4 H), 4.31-4.42 (m, 6 H), 6.94 (m, 1 H), 6.99-7.07 (m, 1 H), 7.27 (m, 1 H), 7.33 (m, 1 H) and 8.28 (bs, 1 H). This compound is not covered by the claims.

### Example 12 8-[4-(3,3-Difluoro-azetidin-1-yl)-piperidin-1-ylmethyl]-9-ethyl-2-(1H-indol-4-yl)-6-morpholin-4-yl-9H-purine

Prepared by using Suzuki coupling method B. The title compound was obtained as a colourless oil (61 mg, 36 %).
[M + H]⁺ 537.2
¹H NMR (400 MHz, CDCl₃): δ 1.34-1.45 (m, 2 H), 1.52 (t, J = 7.1 Hz, 3 H), 1.68 (m, 2 H), 2.12-2.22 (m, 3 H), 2.84 (m, 2 H), 3.53 (t, J = 11.8 Hz, 4 H), 3.74 (s, 2 H), 3.87 (t, J = 4.7 Hz, 4 H), 4.35-4.46 (m, 6 H), 7.24-7.31 (m, 2 H), 7.44 (dt, J = 8.1, 1.0 Hz, 1 H), 7.59 (m, 1 H), 8.22 (dd, J = 7.6, 1.0 Hz, 1 H) and 8.33 (bs, 1 H). This compound is not covered by the claims.

### Example 13 2-{4-[9-Ethyl-2-(5-fluoro-1H-indol-4-yl)-6-morpholin-4-yl-9H-purin-8-ylmethyl]-2,2-dimethyl-piperazin-1-yl}-acetamide

Prepared by using Suzuki coupling method C. The title compound was obtained as a pale yellow oil (105 mg, 80 %).
[M + H]⁺ 550.3
¹H NMR (400 MHz, CDCl₃): δ 1.06 (s, 6 H), 1.50 (t, J = 7.1 Hz, 3 H), 2.32 (m, 2 H), 2.51 (m, 2 H), 2.61 (m, 2 H), 2.99 (m, 2 H), 3.70 (s, 2 H), 3.83 (t, J = 4.7 Hz, 4 H), 4.34 (s, 4 H), 4.42 (q, J = 7.1 Hz, 2 H), 5.51 (m, 1 H), 6.93 (m, 1 H), 6.97-7.04 (m, 1 H), 7.23-7.28 (m, 2 H), 7.32 (ddd, J = 8.9, 3.9, 0.8 Hz, 1 H) and 8.34 (bs, 1 H). This compound is not covered by the claims.

### Example 14 2-{4-[9-Ethyl-2-(1H-indol-4-yl)-6-morpholin-4-yl-9H-purin-8-ylmethyl]-2,2-dimethyl-piperazin-1-yl}-acetamide

Prepared by using Suzuki coupling method B. The title compound was obtained as a colourless oil (96 mg, 96 %).
[M + H]⁺ 532.3
¹H NMR (400 MHz, CDCl₃): δ 1.08 (s, 6 H), 1.57 (t, J = 7.1 Hz, 3 H), 2.35 (bs, 2 H), 2.54 (bs, 2 H), 2.64 (m, 2 H), 3.02 (bs, 2 H), 3.72 (s, 2 H), 3.90 (t, J = 4.7 Hz, 4 H), 4.41 (t, J = 4.7 Hz, 4 H), 4.50 (q, J = 7.1 Hz, 2 H), 5.47 (d, J = 5.5 Hz, 1 H), 7.28-7.36 (m, 3 H), 7.48 (dt, J = 8.0, 1.0 Hz, 1 H), 7.61 (m, 1 H), 8.24 (dd, J = 7.6, 1.0 Hz, 1 H) and 8.33 (bs, 1 H). This compound is not covered by the claims.

### Example 15 8-[9-Ethyl-2-(5-fluoro-1H-indol-4-yl)-6-morpholin-4-yl-9H-purin-8-ylmethyl]-2,8-diaza-spiro[4.5]decan-3-one

Prepared by using Suzuki coupling method C. The title compound was obtained as a colourless oil (97 mg, 65 %).
[M + H]⁺ 533.2
¹H NMR (400 MHz, CDCl₃): δ 1.48 (t, J = 7.1 Hz, 3 H), 1.68 (m, 4 H), 2.21 (s, 2 H), 2.50 (m, 4 H), 3.18 (s, 2 H), 3.74 (s, 2 H), 3.83 (t, J = 4.7 Hz, 4 H), 4.30-4.44 (m, 6 H), 5.68 (bs, 1 H), 6.92 (m, 1 H), 6.96-7.05 (m, 1 H), 7.26 (m, 1 H), 7.32 (ddd, J = 8.8, 3.9, 0.8 Hz, 1 H) and 8.32 (bs, 1 H).

### Example 16 8-[9-Ethyl-2-(1H-indol-4-yl)-6-morpholin-4-yl-9H-purin-8-ylmethyl]-2,8-diaza-spiro[4.5]decan-3-one

Prepared by using Suzuki coupling method B. The title compound was obtained as a colourless oil (97 mg, 80 %).
[M + H]⁺ 515.2
¹H NMR (400 MHz, CDCl₃): δ 1.50 (t, J = 7.1 Hz, 3 H), 1.65 (m, 4 H), 2.18 (s, 2 H), 2.47 (m, 4 H), 3.14 (s, 2 H), 3.72 (s, 2 H), 3.85 (t, J = 4.7 Hz, 4 H), 4.33-4.44 (m, 6 H), 5.87 (bs, 1 H), 7.23-7.29 (m, 2 H), 7.39-7.45 (m, 1 H), 7.56 (t, J = 2.5 Hz, 1 H), 8.19 (dd, J = 7.5, 1.0 Hz, 1 H) and 8.38 (bs, 1 H).

### Example 17 1-{1-[9-Ethyl-2-(1H-indol-4-yl)-6-morpholin-4-yl-9H-purin-8-ylmethyl]-piperidin-4-yl}-azetidin-2-one

Prepared by using Suzuki coupling method F. The title compound was obtained as a cream solid (122 mg, 77 %).
[M + H]⁺ 515.2
¹H NMR (400 MHz, CDCl₃): δ 1.53 (t, J = 7.2 Hz, 3 H), 1.68 (m, 2 H), 1.85 (m, 2 H), 2.23 (m, 2 H), 2.84-2.92 (m, 4 H), 3.21 (t, J = 4.0 Hz, 2 H), 3.59 (m, 1 H), 3.73 (s, 2 H), 3.88 (t, J = 4.7 Hz, 4 H), 4.35-4.46 (m, 6 H), 7.26-7.33 (m, 2 H), 7.46 (d, J = 8.0 Hz, 1 H), 7.59 (s, 1 H), 8.20-8.23 (m, 1 H) and 8.28 (bs, 1 H). This compound is not covered by the claims.

### Example 18 1-{1-[9-Ethyl-2-(5-fluoro-1H-indol-4-yl)-6-morpholin-4-yl-9H-purin-8-ylmethyl]-piperidin-4-yl}-azetidin-2-one

Prepared by using Suzuki coupling method G. The title compound was obtained as a pale yellow solid (56 mg, 34 %).
[M + H]⁺ 533.2
¹H NMR (400 MHz, CDCl₃): δ 1.48 (t, J = 7.1 Hz, 3 H), 1.68 (m, 2 H), 1.85 (m, 2 H), 2.22 (m, 2 H), 2.83-2.92 (m, 4 H), 3.21 (m, 2 H), 3.59 (m, 1 H), 3.75 (s, 2 H), 3.77-3.86 (m, 4 H), 4.29-4.44 (m, 6 H), 6.92-6.94 (m, 1 H), 7.03 (dd, J = 10.9, 8.7 Hz, 1 H), 7.26 (t, J = 2.8 Hz, 1 H), 7.33 (dd, J = 8.7, 3.8 Hz, 1 H) and 8.23 (bs, 1 H). This compound is not covered by the claims.

### Example 19 9-Ethyl-8-[4-(3-fluoro-azetidin-1-yl)-piperidin-1-ylmethyl]-2-(5-fluoro-1H-indol-4-yl)-6-morpholin-4-yl-9H-purine

Prepared by using Suzuki coupling method G. The title compound was obtained as a cream solid (100 mg, 62 %).
[M + H]⁺ 537.3
¹H NMR (400 MHz, CDCl₃): δ 1.39 (m, 2 H), 1.47 (t, J = 7.1 Hz, 3 H), 1.73 (m, 3 H), 2.17 (m, 3 H), 2.81-2.94 (m, 2 H), 3.17 (m, 2 H), 3.74 (m, 3 H), 3.83 (t, J = 4.7 Hz, 4 H), 4.31-4.42 (m, 6 H), 5.13 (d, J = 57.4 Hz, 1 H), 6.93 (m, 1 H), 7.03 (dd, J = 10.9, 8.7 Hz, 1 H), 7.26 (t, J = 2.8 Hz, I H), 7.33 (ddd, J = 8.7, 3.8, 0.9 Hz, 1 H) and 8.23 (bs, 1 H). This compound is not covered by the claims.

### Example 20 9-Ethyl-8-[4-(3-fluoro-azetidin-1-yl)-piperidin-1-ylmethyl]-2-(1H-indol-4-yl)-6-morpholin-4-yl-9H-purine

Prepared by using Suzuki coupling method F. The title compound was obtained as a white solid (80 mg, 53 %).
[M+H]⁺ 519
¹H NMR (400 MHz, CDCl₃): δ 1.34 (m, 2 H), 1.52 (t, J = 7.1 Hz, 3 H), 1.71 (m, 2 H), 2.16 (m, 3 H), 2.84 (m, 2 H), 3.12 (m, 2 H), 3.66 (m, 2 H), 3.73 (s, 2 H), 3.79-3.90 (m, 4 H), 4.36-4.46 (m, 6 H), 5.00-5.21 (m, 1 H), 7.25-7.32 (m, 2 H), 7.45 (d, J = 8.0 Hz, 1 H), 7.59 (m, 1 H), 8.22 (dd, J = 7.5, 1.0 Hz, 1 H) and 8.26 (bs, 1 H). This compound is not covered by the claims.

### Example 21 9-[9-Ethyl-2-(5-fluoro-1H-indol-4-yl)-6-morpholin-4-yl-9H-purin-8-ylmethyl]-1-oxa-4,9-diaza-spiro[5.5]undecan-3-one

Prepared by using Suzuki coupling method G. The title compound was obtained as a yellow solid (69 mg, 42 %).
[M + H]⁺ 549.2
¹H NMR (400 MHz, CDCl₃): δ 1.44-1.56 (m, 3 H), 1.62 (m, 2 H), 1.94 (m, 2 H), 2.52 (m, 2 H), 2.64 (m, 2 H), 3.24 (d, J = 2.6 Hz, 2 H), 3.75-3.88 (m, 6 H), 4.10-4.21 (m, 2 H), 4.29-4.44 (m, 6 H), 6.11 (bs, 1 H), 6.92 (m, 1 H), 7.03 (dd, J = 10.9, 8.7 Hz, 1 H), 7.26 (m, 1 H), 7.30-7.35 (m, 1 H) and 8.29 (bs, 1 H).

### Example 22 9-[9-Ethyl-2-(1H-indol-4-yl)-6-morpholin-4-yl-9H-purin-8-ylmethyl]-1-oxa-4,9-diaza-spiro[5.5]undecan-3-one

Prepared by using Suzuki coupling method F. The title compound was obtained as a white solid (103 mg, 82 %).
[M + H]⁺ 531.2
¹H NMR (400 MHz, CDCl₃): δ 1.53 (t, J = 7.1 Hz, 3 H), 1.61 (m, 2 H), 1.94 (m, 2 H), 2.52 (m, 2 H), 2.64 (m, 2 H), 3.24 (d, J = 2.6 Hz, 2 H), 3.78 (m, 2 H), 3.87 (t, J = 4.7 Hz, 4 H), 4.16 (s, 2 H), 4.35-4.46 (m, 6 H), 6.06 (bs, 1 H), 7.25-7.32 (m, 2 H), 7.46 (d, J = 8.0 Hz, I H), 7.58 (t, J = 2.5 Hz, 1 H), 8.22 (dd, J = 7.5, 1.0 Hz, 1 H) and 8.29 (bs, 1 H).

### Example 23 1-[9-Ethyl-2-(5-fluoro-1H-indol-4-yl)-6-morpholin-4-yl-9H-purin-8-ylmethyl]-piperidine-4-carboxylic acid amide

Prepared by using Suzuki coupling method C. The title compound was obtained as a white solid (38 mg, 31 %).
[M + H]⁺ 507.2
¹H NMR (400 MHz, CDCl₃): δ 1.47 (t, J = 7.1 Hz, 3 H), 1.69-1.83 (m, 4 H), 2.13-2.29 (m, 3 H), 2.97 (d, J = 11.3 Hz, 2 H), 3.78 (s, 2 H), 3.81 (t, J = 4.7 Hz, 4 H), 4.30 (t, J = 4.7 Hz, 4 H), 4.39 (q, J = 7.1 Hz, 2 H), 6.67 (m, 1 H), 6.96 (dd, J = 10.9, 8.8 Hz, 1 H), 7.31 (d, J = 3.1 Hz, 1 H) and 7.38-7.43 (m, 1 H). This compound is not covered by the claims.

### Example 24 2-{4-[9-Ethyl-2-(5-fluoro-1H-indol-4-yl)-6-morpholin-4-yl-9H-purin-8-ylmethyl]-piperazin-1-yl}-isobutyramide

Prepared by using Suzuki coupling method C. The title compound was obtained as a yellow solid (51 mg, 23 %).
[M + H]⁺ 550.2
¹H NMR (400 MHz, DMSO-d₆): δ 1.07 (s, 6 H), 1.40 (t, J = 7.1 Hz, 3 H), 2.42 (m, 4 H), 2.51 (m, 4 H), 3.70-3.78 (m, 6 H), 4.16-4.34 (m, 6 H), 6.70 (m, 1 H), 6.92-7.01 (m, 2 H), 7.05 (m, 1 H), 7.41-7.45 (m, 2 H) and 11.20 (bs, 1 H). This compound is not covered by the claims.

### Example 25 2-{(cis)-4-[9-Ethyl-2-(5-fluoro-1H-indol-4-yl)-6-morpholin-4-yl-9H-purin-8-ylmethyl]-2,6-dimethyl-piperazin-1-yl}-acetamide

Prepared by using Suzuki coupling method C. The title compound was obtained as a white solid (140 mg, 80 %).
[M + H]⁺ 550.2
¹H NMR (400 MHz, CDCl₃): δ 1.03 (d, J = 6.2 Hz, 6 H), 1.48 (t, J = 7.1 Hz, 3 H), 1.99 (t, J = 10.7 Hz, 2 H), 2.62 (m, 2 H), 2.75 (d, J = 10.7 Hz, 2 H), 3.09 (s, 2 H), 3.69 (s, 2 H), 3.84 (t, J = 4.7 Hz, 4 H), 4.31-4.43 (m, 6 H), 5.51 (bs, 1 H), 6.92 (s, 1 H), 6.97-7.06 (m, 1 H), 7.22-7.28 (m, 2 H), 7.33 (dd, J = 8.8, 3.5 Hz, 1 H) and 8.32 (bs, 1 H). This compound is not covered by the claims.

### Example 26 2-{(cis)-4-[9-Ethyl-2-(1H-indol-4-yl)-6-morpholin-4-yl-9H-purin-8-ylmethyl]-2,6-dimethyl-piperazin-1-yl}-acetamide

Prepared by using Suzuki coupling method B. The title compound was obtained as a yellow solid (154 mg, 85 %).
[M + H]⁺ 532.3
¹H NMR (400 MHz, CDCl₃): δ 1.03 (d, J = 6.2 Hz, 6 H), 1.53 (t, J = 7.1 Hz, 3 H), 1.94-2.04 (m, 2 H), 2.62 (m, 2 H), 2.75 (d, J = 11.3 Hz, 2 H), 3.10 (s, 2 H), 3.69 (s, 2 H), 3.88 (t, J = 4.7 Hz, 4 H), 4.36-4.48 (m, 6 H), 5.41 (d, J = 5.5 Hz, 1 H), 7.25-7.35 (m, 3 H), 7.47 (d, J = 8.2 Hz, 1 H), 7.59 (m, 1 H), 8.22 (dd, J = 7.5, 1.0 Hz, 1 H) and 8.27 (bs, 1 H). This compound is not covered by the claims.

### Example 27 2-{(S)-4-[9-Ethyl-2-(5-fluoro-1H-indol-4-yl)-6-morpholin-4-yl-9H-purin-8-ylmethyl]-2-isopropyl-piperazin-1-yl}-acetamide

Prepared by using Suzuki coupling method C. The title compound was obtained as a yellow oil (32 mg, 23 %).
[M + H]⁺ 564.2
¹H NMR (400 MHz, CDCl₃): δ 0.87 (d, J = 6.8 Hz, 3 H), 0.94 (d, J = 6.8 Hz, 3 H), 1.48 (t, J = 7.1 Hz, 3 H), 2.03-2.14 (m, 1 H), 2.20-2.37 (m, 3 H), 2.46 (ddd, J = 12.2, 9.3, 2.7 Hz, 1 H), 2.63 (d, J = 11.0 Hz, 1 H), 2.73 (d, J = 11.0 Hz, 1 H), 2.83 (d, J = 16.8 Hz, 1 H), 2.90-2.96 (m, 1 H), 3.45 (d, J = 16.8 Hz, 1 H), 3.64-3.79 (m, 2 H), 3.83 (t, J = 4.7 Hz, 4 H), 4.32-4.42 (m, 6 H), 5.49 (d, J = 5.3 Hz, 1 H), 6.93 (m, 1 H), 6.96-7.05 (m, 1 H), 7.11 (d, J = 5.3 Hz, 1 H), 7.26 (s, 1 H), 7.33 (ddd, J = 8.8, 3.8, 0.9 Hz, 1 H) and 8.30 (bs, 1 H). This compound is not covered by the claims.

### Example 28 2-{(S)-4-[9-Ethyl-2-(1H-indol-4-yl)-6-morpholin-4-yl-9H-purin-8-ylmethyl]-2-isopropyl-piperazin-1-yl}-acetamide

Prepared by using Suzuki coupling method B. The title compound was obtained as a yellow oil (31 mg, 23 %).
[M + H]⁺ 546.2
¹H NMR (400 MHz, CDCl₃): δ 0.86 (d, J = 6.8 Hz, 3 H), 0.93 (d, J = 6.8 Hz, 3 H), 1.53 (t, J = 7.1 Hz, 3 H), 2.04-2.14 (m, 1 H), 2.20-2.36 (m, 3 H), 2.46 (ddd, J = 12.2,9.3, 2.8 Hz, 1 H), 2.63 (d, J = 11.1 Hz, 1 H), 2.73 (d, J = 10.2 Hz, 1 H), 2.83 (d, J = 16.8 Hz, 1 H), 2.93 (dt, J = 12.2, 3.6 Hz, 1 H), 3.45 (d, J = 16.8 Hz, 1 H), 3.67-3.77 (m, 2 H), 3.87 (t, J = 4.7 Hz, 4 H), 4.36-4.48 (m, 6 H), 5.45-5.51 (m, 1 H), 7.11 (d, J = 5.3 Hz, 1 H), 7.25-7.33 (m, 2 H), 7.46 (d, J = 8.0 Hz, 1 H), 7.58-7.60 (m, 1 H), 8.22 (dd, J = 7.5, 1.0 Hz, 1 H) and 8.30 (bs, 1 H). This compound is not covered by the claims.

### Example 29 9-Ethyl-2-(1H-indol-4-yl)-6-morpholin-4-yl-8-[4-(tetrahydro-pyran-4-yl)-piperazin-1-ylmethyl]-9H-purine

Prepared by using Suzuki coupling method A. The title compound was obtained as a colourless oil (102 mg, 87 %).
[M + H]⁺ 531.2
¹H NMR (400 MHz, CDCl₃): δ 1.53 (t, J = 7.15 Hz, 3 H), 1.61 (m, 2 H), 1.80 (m, 2 H), 2.03-3.08 (m, 9 H), 3.36 (t, J = 11.7 Hz, 2 H), 3.76 (s, 2 H), 3.79-3.90 (m, 4 H), 4.01 (dd, J = 11.5, 4.1 Hz, 2 H), 4.35-4.46 (m, 6 H), 7.25-7.32 (m, 2 H), 7.46 (d, J = 8.0 Hz, 1 H), 7.59 (t, J = 2.5 Hz, 1 H), 8.21 (dd, J = 7.5, 1.0 Hz, 1 H) and 8.29 (bs, 1 H). This compound is not covered by the claims.

### Example 30 4-[9-Ethyl-2-(5-fluoro-1H-indol-4-yl)-6-morpholin-4-yl-9H-purin-8-ylmethyl]-6,6-dimethyl-piperazin-2-one

Prepared by using Suzuki coupling method C. The title compound was obtained as white solid (42 mg, 62 %).
[M + H]⁺ 507.2
¹H NMR (400 MHz, CDCl₃): δ 1.15 (s, 6 H), 1.40 (t, J = 7.1 Hz, 3 H), 2.94 (s, 2 H), 3.74 (t, J = 4.6 Hz, 4 H), 3.85 (s, 2 H), 4.23 (m, 4 H), 4.33 (q, J = 7.1 Hz, 2 H), 6.71 (d, J = 2.6 Hz, 1 H), 6.98 (dd, J = 11.1, 8.7 Hz, 1 H), 7.40-7.45 (m, 2 H), 7.80 (s, 1 H) and 11.21 (bs, 1 H). This compound is not covered by the claims.

### Example 31 4-[9-Ethyl-2-(1H-indol-4-yl)-6-morpholin-4-yl-9H-purin-8-ylmethyl]-6,6-dimethyl-piperazin-2-one

Prepared by using Suzuki coupling method B. The title compound was obtained as white solid (42 mg, 58 %).
[M + H]⁺ 489.2
¹H NMR (400 MHz, DMSO-d₆): δ 1.27 (s, 6 H), 1.56 (t, J = 7.1 Hz, 3 H), 2.60 (s, 2 H), 3.12 (s, 2 H), 3.86 (t, J = 4.7 Hz, 4 H), 3.89 (s, 2 H), 4.37 (t, J = 4.7 Hz, 4 H), 4.49 (q, J = 7.1 Hz, 2 H), 7.20 (t, J = 7.7 Hz, 1 H), 7.33 (d, J = 3.1 Hz, 1 H), 7.42 (dd, J = 3.5, 1.0 Hz, 1 H), 7.49 (dd, J = 7.7, 1.0 Hz, 1 H) and 8.06 (dd, J = 7.7, 1.0 Hz, 1 H). This compound is not covered by the claims.

### Example 32 8-(2,2-Dimethyl-morpholin-4-ylmethyl)-9-ethyl-2-(5-fluoro-1H-indol-4-yl)-6-morpholin-4-yl-9H-purine

Prepared by using Suzuki coupling method C. The title compound was obtained as white solid (35 mg, 40 %).
[M + H]⁺ 494.2
¹H NMR (400 MHz, CDCl₃): δ 1.23 (s, 6 H), 1.53 (t, J = 7.1 Hz, 3 H), 2.35 (m, 2 H), 2.47 (m, 2 H), 3.74 (m, 4 H), 3.79-3.88 (m, 4 H), 4.36 (m, 4 H), 4.46 (q, J = 7.1 Hz, 2 H), 6.95 (t, J = 2.6 Hz, 1 H), 7.05 (dd, J = 10.9, 8.7 Hz, 1 H), 7.28 (t, J = 2.6 Hz, 1 H), 7.35 (dd, J = 8.7, 3.7 Hz, 1 H) and 8.22 (bs, 1 H). This compound is not covered by the claims.

### Example 33 9-Ethyl-2-(5-fluoro-1H-indol-4-yl)-6-morpholin-4-yl-8-(3-morpholin-4-yl-azetidin-1-ylmethyl)-9H-purine

Prepared by using Suzuki coupling method D. The title compound was obtained as a white solid (38 mg, 37 %).
[M + H]⁺ 521.2 ¹H NMR (400 MHz, CDCl₃): δ 1.47 (t, J = 7.2 Hz, 3 H), 2.37 (m, 4 H), 3.04-3.23 (m, 3 H), 3.62 (m, 2 H), 3.74 (m, 4 H), 3.78-3.87 (m, 4 H), 3.92 (s, 2 H), 4.32-4.40 (m, 6 H), 6.94 (t, J = 2.5 Hz, 1 H), 7.04 (dd, J = 10.9, 8.7 Hz, 1 H), 7.28 (m, 1 H), 7.33-7.37 (m, 1 H) and 8.22 (bs, 1 H). This compound is not covered by the claims.

### Example 34 9-Ethyl-2-(1H-indol-4-yl)-6-morpholin-4-yl-8-(3-morpholin-4-yl-azetidin-1-ylmethyl)-9H-purine

Prepared by using Suzuki coupling method E. The title compound was obtained as a tan solid (58 mg, 57 %).
[M + H]⁺ 503.2
¹H NMR (400 MHz, CDCl₃): δ 1.51 (t, J = 7.2 Hz, 3 H), 2.38 (m, 4 H), 3.09 (m, 3 H), 3.66 (m, 2 H), 3.75 (m, 4 H), 3.89 (t, J = 4.7 Hz, 4 H), 3.95 (s, 2 H), 4.37-4.46 (m, 6 H), 7.27-7.34 (m, 2 H), 7.47 (d, J = 8.0 Hz, 1 H), 7.57-7.60 (m, 1 H) and 8.20-8.27 (m, 2 H). This compound is not covered by the claims.

### Example 35 9-Ethyl-2-(1H-indol-4-yl)-6-morpholin-4-yl-8-[4-(2,2,2-trifluoro-ethyl)-piperazin-1-ylmethyl]-9H-purine

Prepared by using Suzuki coupling method E. The title compound was obtained as a tan foam (94 mg, 38 %).
[M + H]⁺ 529.3
¹H NMR (400 MHz, CDCl₃): δ 1.52 (t, J = 7.1 Hz, 3 H), 2.59 (m, 4 H), 2.71 (m, 4 H), 2.95 (d, J = 9.4 Hz, 1 H), 3.01 (d, J = 9.4 Hz, 1 H), 3.76 (s, 2 H), 3.89 (t, J = 4.7 Hz, 4 H), 4.36-4.47 (m, 6 H), 7.29-7.34 (m, 2 H), 7.48 (d, J = 8.0 Hz, 1 H), 7.60 (s, 1 H) and 8.19-8.26 (m, 2 H). This compound is not covered by the claims.

### Example 36 9-Ethyl-2-(5-fluoro-1H-indol-4-yl)-6-morpholin-4-yl-8-[4-(2,2,2-trifluoro-ethyl)-piperazin-1-ylmethyl]-9H-purine

Prepared by using Suzuki coupling method D. The title compound was obtained as a cream foam (90 mg, 35 %).
[M + H]⁺ 547.3
¹H NMR (400 MHz, CDCl₃): δ 1.50 (t, J = 7.1 Hz, 3 H), 2.59 (m, 4 H), 2.71 (m, 4 H), 2.96 (d, J = 9.4 Hz, 1 H), 2.99 (d, J = 9.4 Hz, 1 H), 3.76 (s, 2 H), 3.85 (t, J = 4.7 Hz, 4 H), 4.32-4.44 (m, 6 H), 6.94 (m, 1 H), 7.04 (dd, J = 11.0, 8.8 Hz, 1 H), 7.27 (m, 1 H), 7.35 (ddd, J = 8.8, 3.9,0.9 Hz, 1 H) and 8.21 (bs, 1 H). This compound is not covered by the claims.

### Example 37 9-Ethyl-2-(1H-indol-4-yl)-6-morpholin-4-yl-8-(4-pyrazol-1-yl-piperidin-1-ylmethyl)-9H-purine

Prepared by using Suzuki coupling method B. The title compound was obtained as a white solid (120 mg, 99 %).
[M+H]⁺ 512.3
¹H NMR (400 MHz, CDCl₃): δ 1.50 (t, J = 7.1 Hz, 3 H), 2.02-2.13 (m, 2 H), 2.16 (d, J = 12.3 Hz, 2 H), 2.34 (dd, J = 12.7, 10.5 Hz, 2 H), 3.04 (d, J = 11.5 Hz, 2 H), 3.81 (s, 2 H), 3.89 (t, J = 4.7 Hz, 4 H), 4.18 (tt, J = 11.5,4.3 Hz, 1 H), 4.40 (t, J = 4.7 Hz, 4 H), 4.48 (q, J = 7.1 Hz, 2 H), 6.25 (t, J = 2.0 Hz, 1 H), 7.29 (d, J = 7.8 Hz, 1 H), 7.30-7.36 (m, 1 H), 7.43 (d, J = 2.3 Hz, 1 H), 7.47 (dd, J = 7.8, 1.0 Hz, 1 H), 7.52 (d, J = 1.8 Hz, 1 H), 7.60 (m, 1 H), 8.23 (dd, J = 7.5, 1.0 Hz, 1 H) and 8.26 (bs, 1 H). This compound is not covered by the claims.

### Example 38 9-Ethyl-2-(5-fluoro-1H-indol-4-yl)-6-morpholin-4-yl-8-(4-pyrazol-1-yl-piperidin-1-ylmethyl)-9H-purine

Prepared by using Suzuki coupling method C. The title compound was obtained as a white solid (88 mg, 71 %).
[M + H]⁺ 530.4
¹H NMR (400 MHz, CDCl₃): δ 1.51 (t, J = 7.1 Hz, 3 H), 2.02-2.11 (m, 2 H), 2.16 (d, J = 12.3 Hz, 2 H), 2.34 (t, J = 11.6 Hz, 2 H), 3.03 (d, J = 11.6 Hz, 2 H), 3.81 (s, 2 H), 3.85 (t, J = 4.7 Hz, 4 H), 4.11-4.22 (m, 1 H), 4.36 (m, 4 H), 4.42 (q, J = 7.1 Hz, 2 H), 6.25 (t, J = 2.1 Hz, 1 H), 6.93 (t, J = 2.5 Hz, 1 H), 7.02 (dd, J = 11.0, 8.8 Hz, 1 H), 7.25 (m, 1 H), 7.28-7.33 (m, 1 H), 7.43 (d, J = 2.3 Hz, 1 H), 7.52 (d, J = 1.8 Hz, 1 H) and 8.42 (bs, 1 H). This compound is not covered by the claims.

### Example 39 9-Ethyl-2-(5-fluoro-1H-indol-4-yl)-6-morpholin-4-yl-8-[4-(1H-pyrazol-3-yl)-piperidin-1-ylmethyl]-9H-purine

Prepared by using Suzuki coupling method C. The title compound was obtained as a tan solid (12 mg, 10 %).
[M + H]⁺ 530.4
¹H NMR (400 MHz, CDCl₃): δ 1.51 (t, J = 7.1 Hz, 3 H), 1.67-1.82 (m, 2 H), 2.00 (d, J = 13.1 Hz, 2 H), 2.28 (t, J = 11.5 Hz, 2 H), 2.69-2.78 (m, 1 H), 2.98 (d, J = 11.5 Hz, 2 H), 3.79 (s, 2 H), 3.85 (t, J = 4.7 Hz, 4 H), 4.36 (m, 4 H), 4.43 (q, J = 7.1 Hz, 2 H), 6.13 (d, J = 2.2 Hz, 1 H), 6.94-6.96 (m, 1 H), 7.04 (dd, J = 11.0, 8.8 Hz, 1 H), 7.28 (m, 1 H), 7.34 (ddd, J = 8.8, 3.8, 0.9 Hz, 1 H), 7.49 (d, J = 2.1 Hz, 1 H) and 8.21 (bs, 1 H). This compound is not covered by the claims.

### Example 40 9-Ethyl-2-(1H-indol-4-yl)-6-morpholin-4-yl-8-[4-(1H-pyrazol-3-yl)-piperidin-1-ylmethyl]-9H-purine

Prepared by using Suzuki coupling method B. The title compound was obtained as a tan solid (38 mg, 32 %).
[M + H]⁺ 512.3
¹H NMR (400 MHz, CDCl₃): δ 1.56 (t, J = 7.1 Hz, 3 H), 1.69-1.82 (m, 2 H), 2.00 (d, J = 13.2 Hz, 2 H), 2.24-2.33 (m, 2 H), 2.69-2.78 (m, 1 H), 2.98 (d, J = 11.3 Hz, 2 H), 3.78 (s, 2 H), 3.89 (t, J = 4.7 Hz, 4 H), 4.41 (t, J = 4.7 Hz, 4 H), 4.48 (q, J = 7.1 Hz, 2 H), 6.13 (d, J = 2.2 Hz, 1 H), 7.29 (d, J = 7.8 Hz, 1 H), 7.31 (m, 1 H), 7.48 (m, 2 H), 7.61 (m, 1 H) and 8.21-8.26 (m, 2 H). This compound is not covered by the claims.

### Example 41 1-[9-Ethyl-2-(5-fluoro-1H-indol-4-yl)-6-morpholin-4-yl-9H-purin-8-ylmethyl]-piperidine-4-carboxylic acid

To a suspension of 1-[9-ethyl-2-(5-fluoro-1*H*-indol-4-yl)-6-morpholin-4-yl-9*H*-purin-8-ylmethyl]-piperidine-4-carboxylic acid ethyl ester (30 mg, 0.056 mmol) in THF (2 mL) were added a aqueous solution of lithium hydroxide (1 M, 100 µL, 0.1 mmol) and IMS (1 ml). The resulting solution was stirred at RT for 4 days, then concentrated *in vacuo.* The resulting residue was co-evaporated repeatedly with MeOH, then triturated with diethyl ether. The resultant solid was collected by filtration and dried to give the title compound as a cream solid (20 mg, 70 %).
[M + H]⁺ 508.3
¹H NMR (400 MHz, DMSO-d₆): δ 1.42 (t, J = 7.1 Hz, 3 H), 1.44-1.56 (m, 2 H), 1.72 (m, 2 H), 1.85 (m, 1 H), 2.04 (t, J = 11.2 Hz, 2 H), 2.77 (d, J = 11.2 Hz, 2 H), 3.69-3.77 (m, 6 H), 4.16-4.34 (m, 6 H), 6.72 (t, J = 2.4 Hz, 1 H), 6.98 (dd, J = 11.1, 8.7 Hz, 1 H), 7.40-7.45 (m, 2 H) and 11.31 (bs, 1 H). This compound is not covered by the claims.

### Example 42 1-[9-Ethyl-2-(5-fluoro-1H-indol-4-yl)-6-morpholin-4-yl-9H-purin-8-ylmethyl]-4-methyl-piperidine-4-carboxylic acid amide

Prepared by using Suzuki coupling method C. The title compound was obtained as a white solid (50 mg, 44 %).
[M + H]⁺ 521.3
¹H NMR (400 MHz, CDCl₃): δ 1.23 (s, 3 H), 1.46-1.60 (m, 5 H), 2.02 (m, 2 H), 2.40-2.49 (m, 2 H), 2.63 (m, 2 H), 3.40 (m, 2 H), 3.74 (s, 2 H), 3.85 (t, J = 4.7 Hz, 4 H), 4.29-4.44 (m, 6 H), 6.86 (m, 1 H), 7.03 (dd, J = 11.0, 8.7 Hz, 1 H), 7.28 (m, 1 H), 7.37 (dd, J = 8.8, 3.8 Hz, 1 H) and 9.06 (bs, 1 H). This compound is not covered by the claims.

### Example 43 4-{1-[9-Ethyl-2-(5-(fluoro-1H-indol-4-yl)-6-morpholin-9-yl-9H-purin-8-ylmethyl]-piperidin-4-yl}-morpholin-3-one

Prepared by using Suzuki coupling method C. The title compound was obtained as a colourless oil (45 mg, 47 %).
[M + H]⁺ 563.3
¹H NMR (400 MHz, CDCl₃): δ 1.49 (t, J = 7.1 Hz, 3 H), 1.65-1.76 (m, 4 H), 2.25-2.34 (m, 2 H), 2.99 (m, 2 H), 3.28 (t, J = 5.0 Hz, 2 H), 3.75 (s, 2 H), 3.82-3.89 (m, 6 H), 4.18 (s, 2 H), 4.33-4.43 (m, 6 H), 4.47-4.58 (m, 1 H), 6.91-6.93 (m, 1 H), 7.02 (dd, J = 11.0, 8.8 Hz, 1 H), 7.25 (m, 1 H), 7.32 (ddd, J = 8.9, 4.0, 0.8 Hz, 1 H) and 8.44 (bs, 1 H). This compound is not covered by the claims.

### Example 44 4-{1-[9-Ethyl-2-(1H-indol-4-yl)-6-morpholin-4-yl-9H-purin-8-ylmethyl]-piperidin-4-yl}-morpholin-3-one

Prepared by using Suzuki coupling method B. The title compound was obtained as a white solid (80 mg, 92 %).
[M + H]⁺ 545.3
¹H NMR (400 MHz, CDCl₃): δ 1.51 (t, J = 7.1 Hz, 3 H), 1.61-1.76 (m, 4 H), 2.26 (td, J = 11.2, 3.4 Hz, 2 H), 2.95 (d, J = 11.2 Hz, 2 H), 3.23 (t, J = 5.0 Hz, 2 H), 3.72 (s, 2 H), 3.80-3.88 (m, 6 H), 4.15 (s, 2 H), 4.33-4.44 (m, 6 H), 4.43-4.54 (m, 1 H), 7.25 (m, 2 H), 7.41 (dt, J = 8.0, 0.9 Hz, 1 H), 7.56 (ddd, J = 3.2, 2.1, 0.9 Hz, 1 H), 8.19 (dd, J = 7.5, 1.0 Hz, 1 H) and 8.47 (bs, 1 H). This compound is not covered by the claims.

### Example 45 4-[9-Ethyl-2-(5-fluoro-1H-indol-4-yl)-6-morpholin-4-yl-9H-purin-8-ylmethyl]-1-isopropyl-piperazin-2-one

Prepared by using Suzuki coupling method C. The title compound was obtained as a pale yellow oil (36 mg, 16 %).
[M + H]⁺ 521.3
¹H NMR (400 MHz, CDCl₃): δ 1.13 (d, J = 6.9 Hz, 6 H), 1.47 (t, J = 7.2 Hz, 3 H), 2.76 (t, J = 5.3 Hz, 2 H), 3.17-3.25 (m, 2 H), 3.31 (s, 2 H), 3.79 (s, 2 H), 3.85 (t, J = 4.7 Hz, 4 H), 4.33-4.41 (m, 6 H), 4.85 (m, 1 H), 6.92 (dd, J = 3.0, 2.2 Hz, 1 H), 7.02 (dd, J = 11.0, 8.8 Hz, 1 H), 7.25 (m, 1 H), 7.29-7.34 (m, 1 H) and 8.50 (bs, 1 H). This compound is not covered by the claims.

### Example 46 4-[9-Ethyl-2-(1H-indol-4-yl)-6-morpholin-4-yl-9H-purin-8-ylmethyl]-1-isopropyl-piperazin-2-one

Prepared by using Suzuki coupling method B. The title compound was obtained as a pale yellow oil (73 mg, 27 %).
[M + H]⁺ 503.3
¹H NMR (400 MHz, CDCl₃): δ 1.12 (d, J = 6.8 Hz, 6 H), 1.52 (t, J = 7.2 Hz, 3 H), 2.75 (t, J = 5.3 Hz, 2 H), 3.13-3.22 (m, 2 H), 3.31 (s, 2 H), 3.78 (s, 2 H), 3.89 (t, J = 4.7 Hz, 4 H), 4.37-4.45 (m, 6 H), 4.81-4.93 (m, 1 H), 7.27 (d, J = 7.8 Hz, 1 H), 7.30 (m, 1 H), 7.45 (dt, J = 8.0, 1.0 Hz, 1 H), 7.59 (ddd, J = 3.3, 2.2, 1.0 Hz, 1 H), 8.23 (dd, J = 7.5, 1.0 Hz, 1 H) and 8.47 (bs, 1 H). This compound is not covered by the claims.

### Example 47 9-Ethyl-2-(5-fluoro-1H-indol-4-yl)-6-morpholin-4-yl-8-[4-(tetrahydropyran-4-yl)-piperazin-1-ylmethyl]-9H-purine

Prepared by using Suzuki coupling method H. The title compound was obtained as a white solid (42 mg, 35 %).
[M + H]⁺ 549.3
¹H NMR (400 MHz, CDCl₃): δ 1.50 (t, J = 7.1 Hz, 3 H), 1.59 (m, 2 H), 1.79 (m, 2 H), 2.14-2.93 (m, 9 H), 3.37 (t, J = 11.5 Hz, 2 H), 3.77 (s, 2 H), 3.85 (t, J = 4.7 Hz, 4 H), 4.03 (dd, J = 11.5, 4.1 Hz, 2 H), 4.31-4.44 (m, 6 H), 6.94 (t, J = 2.5 Hz, 1 H), 7.04 (dd, J = 10.9, 8.8 Hz, 1 H), 7.27 (m, 1 H), 7.32-7.37 (m, 1 H) and 8.24 (bs, 1 H). This compound is not covered by the claims.

### Example 48 8-[4-(1,1-Dioxo-hexahydro-1-thiopyran-4-yl)-piperazin-1-ylmethyl]-9-ethyl-2-(5-fluoro-1H-indol-4-yl)-6-morpholin-4-yl-9H-purine

Prepared by using Suzuki coupling method H. The title compound was obtained as a white solid (19 mg, 16 %).
[M + H]⁺ 597.2
¹H NMR (400 MHz, CDCl₃): δ 1.49 (t, J = 7.1 Hz, 3 H), 2.16 (m, 2 H), 2.21-2.31 (m, 2 H), 2.42-2.63 (m, 9 H), 2.85-2.94 (m, 2 H), 3.17-3.28 (m, 2 H), 3.77 (s, 2 H), 3.85 (t, J = 4.7 Hz, 4 H), 4.33-4.42 (m, 6 H), 6.93-6.95 (m, 1 H), 7.04 (dd, J = 11.0, 8.8 Hz, 1 H), 7.27 (d, J = 3.0 Hz, 1 H), 7.34 (ddd, J = 8.8, 3.8, 0.9 Hz, 1 H) and 8.26 (bs, 1 H). This compound is not covered by the claims.

### Example 49 8-[4-(1,1-Dioxo-hexahydro-1-thiopyran-4-yl)-piperazin-1-ylmethyl]-9-ethyl-2-(1H-indol-4-yl)-6-morpholin-4-yl-9H-purine

Prepared by using Suzuki coupling method I. The title compound was obtained as a white solid (16 mg, 14 %).
[M + H]⁺ 579.2
¹H NMR (400 MHz, CDCl₃): δ 1.54 (t, J = 7.2 Hz, 3 H), 2.16 (m, 2 H), 2.20-2.31 (m, 2 H), 2.28-2.67 (m, 9 H), 2.85-2.95 (m, 2 H), 3.18-3.28 (m, 2 H), 3.76 (s, 2 H), 3.89 (t, J = 4.7 Hz, 4 H), 4.37-4.47 (m, 6 H), 7.29 (d, J = 7.9 Hz, 1 H), 7.29-7.35 (m, 1 H), 7.48 (d, J = 7.9 Hz, 1 H), 7.60 (t, J = 2.5 Hz, 1 H), 8.23 (dd, J = 7.5, 1.0 Hz, 1 H) and 8.27 (bs, 1 H). This compound is not covered by the claims.

### Example 50 (R)-8-[9-Ethyl-2-(1H-indol-4-yl)-6-morpholin-4-yl-9H-purin-8-ylmethyl]-octahydro-pyrazino[2,1-c][1,4]oxazine

Prepared by using Suzuki coupling method B. The title compound was obtained as a white solid (82 mg, 63 %).
[M + H]⁺ 503.3
¹H NMR (400 MHz, CDCl₃): δ 1.55 (t, J = 7.1 Hz, 3 H), 1.98 (t, J = 10.6 Hz, 1 H), 2.28-2.51 (m, 4 H), 2.57-2.72 (m, 2 H), 2.80 (m, 2 H), 3.27 (t, J = 10.6 Hz, 1 H), 3.61-3.79 (m, 4 H), 3.80-3.91 (m, 5 H), 4.36-4.48 (m, 6 H), 7.28 (d, J = 7.9 Hz, 1 H), 7.31 (m, 1 H), 7.46 (d, J = 7.9 Hz, 1 H), 7.60 (m, 1 H), 8.23 (dd, J = 7.5, 1.0 Hz, 1 H) and 8.32 (bs, 1 H). This compound is not covered by the claims.

### Example 51 (R)-8-[9-Ethyl-2-(5-fluoro-1H-indol-4-yl)-6-morpholin-4-yl-9H-purin-8-ylmethyl]-octahydro-pyrazino[2,1-c][1,4]oxazine

Prepared by using Suzuki coupling method C. The title compound was obtained as a white solid (42 mg, 57 %).
[M + H]⁺ 521.3
¹H NMR (400 MHz, CDCl₃): δ 1.50 (t, J = 7.1 Hz, 3 H), 1.99 (m, 1 H), 2.40 (m, 4 H), 2.61-2.90 (m, 4 H), 3.28 (m, 1 H), 3.62-3.84 (m, 4 H), 3.81-3.88 (m, 5 H), 4.31-4.44 (m, 6 H), 6.94 (m, 1 H), 7.05 (dd, J = 11.0, 8.8 Hz, 1 H), 7.28 (t, J = 2.8 Hz, 1 H), 7.35 (ddd, J = 8.9, 3.9, 0.9 Hz, 1 H) and 8.22 (bs, 1 H). This compound is not covered by the claims.

### Example 52 (R)-8-[9-Ethyl-2-(5-fluoro-1H-indol-4-yl)-6-morpholin-4-yl-9H-purin-8-ylmethyl]-hexahydro-pyrazino[2,1-c][1,4]oxazin-4-one

Prepared by using Suzuki coupling method C. The title compound was obtained as a white solid (30 mg, 31 %).
[M + H]⁺ 535.3
¹H NMR (400 MHz, CDCl₃): δ 1.51 (t, J = 7.1 Hz, 3 H), 2.08 (t, J = 10.9 Hz, 1 H), 2.29 (td, J = 11.8, 3.2 Hz, 1 H), 2.76-2.87 (m, 2 H), 2.96 (d, J = 11.6 Hz, 1 H), 3.53 (dd, J = 11.8, 7.8 Hz, 1 H), 3.59-3.67 (m, 1 H), 3.75 (d, J = 13.5 Hz, 1 H), 3.82-3.87 (m, 5 H), 3.96 (dd, J = 11.8, 4.4 Hz, 1 H), 4.13 (d, J = 16.3 Hz, 1 H), 4.20 (d, J = 16.3 Hz, 1 H), 4.33-4.47 (m, 6 H), 4.62 (d, J = 13.5 Hz, 1 H), 6.93 (t, J = 2.5 Hz, 1 H), 7.04 (dd, J = 11.0, 8.8 Hz, 1 H), 7.23 (t, J = 2.8 Hz, 1H), 7.35 (dd, J = 8.8, 3.8 Hz, 1 H) and 8.26 (bs, 1 H). This compound is not covered by the claims.

### Example 53 8-(2,2-Dimethyl-morpholin-4-ylmethyl)-9-ethyl-2-(1H-indol-4-yl)-6-morpholin-4-yl-9H-purine

Prepared by using Suzuki coupling method B. The title compound was obtained as a tan solid (49 mg, 60 %).
[M + H]⁺ 476.3
¹H NMR (400 MHz, CDCl₃): δ 1.26 (s, 6 H), 1.54 (t, J = 7.2 Hz, 3 H), 2.34 (bs, 2 H), 2.46 (bs, 2 H), 3.71 (bs, 2 H), 3.75 (bs, 2 H), 3.89 (t, J = 4.8 Hz, 4 H), 4.34-4.43 (m, 4 H), 4.51 (q, J = 7.2 Hz, 2 H), 7.29 (d, J = 7.9 Hz, 1 H), 7.32 (m, 1 H), 7.48 (d, J = 7.9 Hz, 1 H), 7.60 (m, 1 H), 8.23 (dd, J = 7.5, 1.0 Hz, 1 H) and 8.27 (bs, 1 H). This compound is not covered by the claims.

### Example 54 8-[4-(1,1-Dioxothiomorpholin-4-yl)-piperidin-1-ylmethyl]-9-ethyl-2-(5-fluoro-1H-indol-4-yl)-6-morpholin-4-yl-9H-purine

Prepared by using Suzuki coupling method C. The title compound was obtained as a colourless oil (92 mg, 77 %).
[M + H]⁺ 597.3
¹H NMR (400 MHz, CDCl₃): δ 1.46-1.60 (m, 5 H), 1.75 (m, 2 H), 2.15 (m, 2 H), 2.52 (m, 1 H), 2.97 (m, 2 H), 3.01-3.06 (m, 8 H), 3.74 (s, 2 H), 3.84 (t, J = 4.7 Hz, 4 H), 4.33-4.43 (m, 6 H), 6.94 (m, 1 H), 6.98-7.07 (m, 1 H), 7.27 (m, 1 H), 7.34 (ddd, J = 9.0, 4.0, 0.8 Hz, 1 H) and 8.29 (bs, 1 H). This compound is not covered by the claims.

### Example 55 8-[4-(1,1-Dioxothiomorpholin-4-yl)-piperidin-1-ylmethyl]-9-ethyl-2-(1H-indol-4-yl)-6-morpholin-4-yl-9H-purine

Prepared by using Suzuki coupling method B. The title compound was obtained as a colourless oil (155 mg, 89 %).
[M + H]⁺ 579.3
¹H NMR (400 MHz, CDCl₃): δ 1.50-1.60 (m, 5 H), 1.74 (m, 2 H), 2.08-2.20 (m, 2 H), 2.46-2.56 (m, 1 H), 2.97 (m, 2 H), 3.00-3.07 (m, 8 H), 3.73 (s, 2 H), 3.89 (t, J = 4.7 Hz, 4 H), 4.32-4.47 (m, 6 H), 7.28 (d, J = 7.8 Hz, 1 H), 7.31-7.33 (m, 1 H), 7.47 (m, 1 H), 7.59 (m, 1 H), 8.23 (dd, J = 7.7, 0.9 Hz, 1 H) and 8.31 (bs, 1 H). This compound is not covered by the claims.

### Example 56 1-{1-[9-Ethyl-2-(1H-indol-4-yl)-6-morpholin-4-yl-9H-purin-8-ylmethyl]-piperidin-4-yl}-pyrrolidin-2-one

Prepared by using Suzuki coupling method B. The title compound was obtained as a pale yellow oil (32 mg, 24 %).
[M + H]⁺ 529.3
¹H NMR (400 MHz, CDCl₃): δ 1.54 (t, J = 7.1 Hz, 3 H), 1.62-1.76 (m, 4 H), 1.94-2.05 (m, 2 H), 2.23-2.31 (m, 2 H), 2.39 (t, J = 8.1 Hz, 2 H), 2.96 (m, 2 H), 3.34 (t, J = 7.0 Hz, 2 H), 3.74 (s, 2 H), 3.88 (t, J = 4.7 Hz, 4 H), 3.97-4.08 (m, 1 H), 4.36-4.48 (m, 6 H), 7.28 (d, J = 7.8 Hz, 1 H), 7.30 (m, 1 H), 7.45 (m, 1 H), 7.59 (m, 1 H), 8.18-8.25 (m, 1 H) and 8.44 (bs, 1 H). This compound is not covered by the claims.

### Example 57 8-[9-Ethyl-2-(5-fluoro-1H-indol-4-yl)-6-morpholin-4-yl-9H-purin-8-ylmethyl]-2,8-diaza-spiro[4.5]decan-1-one

Prepared by using Suzuki coupling method C. The title compound was obtained as a colourless oil (43 mg, 35 %).
[M + H]⁺ 533.3
¹H NMR (400 MHz, CDCl₃): δ 1.50 (t, J = 7.1 Hz, 3 H), 1.80 (m, 2 H), 1.92-2.02 (m, 2 H), 2.02-2.09 (m, 2 H), 2.26 (m, 2 H), 2.89 (m, 2 H), 3.32 (t, J = 6.9 Hz, 2 H), 3.77-3.89 (m, 6 H), 4.35 (m, 4 H), 4.42 (q, J = 7.1 Hz, 2 H), 5.80 (bs, 1 H), 6.94 (ddd, J = 3.4, 2.3, 0.9 Hz, 1 H), 7.03 (dd, J = 11.0, 8.8 Hz, 1 H), 7.27 (m, 1 H), 7.33 (ddd, J = 8.9, 3.9, 0.9 Hz, 1 H) and 8.36 (bs, 1 H).

### Example 58 7-[9-Ethyl-2-(5-fluoro-1H-indol-4-yl}-6-morpholin-4-yl-9H-purin-8-ylmethyl]-3-oxa-7,9-diaza-bicyclo[3.3.1]nonane

Prepared by using Suzuki coupling method C. The title compound was obtained as a cream solid (40 mg, 71 %).
[M + H]⁺ 507.2
¹H NMR (400 MHz, CDCl₃): δ 1.51 (t, J = 7.2 Hz, 3 H), 2.63 (d, J = 11.1 Hz, 2 H), 2.90 (bs, 2 H), 2.96 (d, J = 11.1 Hz, 2 H), 3.76 (s, 2 H), 3.76-3.93 (m, 8 H), 4.36 (m, 5 H), 4.58 (q, J = 7.2 Hz, 2 H), 6.97 (m, 1 H), 7.04 (dd, J = 11.0, 8.8 Hz, 1 H), 7.28 (m, 1 H), 7.35 (ddd, J = 8.8, 3.9, 0.9 Hz, 1 H) and 8.20 (bs, 1 H). This compound is not covered by the claims.

### Example 59 8-[9-Ethyl-2-(1H-indol-4-yl)-6-morpholin-4-yl-9H-purin-8-ylmethyl]-2,8-diaza-spiro[4.5]decan-1-one

Prepared by using Suzuki coupling method B. The title compound was obtained as a white solid (61 mg, 59 %)
[M+H]⁺ 515.3
¹H NMR (400 MHz, CDCl₃): δ 1.46 (m, 2 H), 1.56 (t, J = 7.1 Hz, 3 H), 1.96 (td, J = 12.6, 3.9 Hz, 2 H), 2.07 (t, J = 7.2 Hz, 2 H), 2.25 (t, J = 11.7 Hz, 2 H), 2.81-2.93 (m, 2 H), 3.33 (t, J = 7.2 Hz, 2 H), 3.78 (s, 2 H), 3.90 (t, J = 4.7 Hz, 4 H), 4.38 (t, J = 4.7 Hz, 4 H), 4.48 (q, J = 7.1 Hz, 2 H), 7.25-7.36 (m, 2 H), 7.47-7.55 (m, 2 H), 8.17 (m, 1 H) and 9.12 (bs, 1 H).

### Example 60 1'-[9-Ethyl-2-(5-fluoro-1H-indol-4-yl)-6-morpholin-4-yl-9H-purin-8-ylmethyl]-[1,4']bipiperidinyl-2-one

Prepared by using Suzuki coupling method C. The title compound was obtained as a white solid (29 mg, 30 %)
[M+H]⁺ 561.3
¹H NMR (400 MHz, CDCl₃): δ 1.50 (t, J = 7.1 Hz, 3 H), 1.62-1.82 (m, 8 H), 2.30 (dd, J = 12.5, 10.4 Hz, 2 H), 2.40 (m, 2 H), 2.96 (m, 2 H), 3.18 (m, 2 H), 3.75 (s, 2 H), 3.85 (t, J = 4.7 Hz, 4 H), 4.31-4.45 (m, 6 H), 4.53-4.63 (m, 1 H), 6.94 (m, I H); 7.04 (dd, J = 10.9, 8.8 Hz, 1 H), 7.27 (m, 1 H), 7.31-7.36 (m, 1 H) and 8.28 (bs, 1 H). This compound is not covered by the claims.

### Example 61 1'-[9-Ethyl-2-(1H-indol-4-yl)-6-morpholin-4-yl-9H-purin-8-ylmethyl]-[1,4']bipiperidinyl-2-one

Prepared by using Suzuki coupling method B. The title compound was obtained as a white solid (33 mg, 44 %)
[M+H]⁺ 543.3
¹H NMR (400 MHz, CDCl₃): δ 1.53-1.82 (m, 11 H), 2.30 (dd, J = 12.5, 10.4 Hz, 2 H), 2.40 (m, 2 H), 2.96 (m, 2 H), 3.18 (m, 2 H), 3.75 (s, 2 H), 3.89 (t, J = 4.7 Hz, 4 H), 4.37-4.48 (m, 6 H), 4.53-4.62 (m, 1 H), 7.27-7.34 (m, 2 H), 7.47 (d, J = 8.0 Hz, 1 H), 7.60 (m, 1 H), 8.22 (m, 1 H) and 8.27 (bs, 1 H). This compound is not covered by the claims.

### Example 62 1-{1-[9-Ethyl-2-(5-fluoro-1H-indol-4-yl)-6-morpholin-4-yl-9H-purin-8-ylmethyl]-piperidin-4-yl}-pyrrolidin-2-one

Prepared by using Suzuki coupling method C. The title compound was obtained as a white solid (19 mg, 18 %)
[M+H]⁺ 547.4
¹H NMR (400 MHz, CDCl₃): δ 1.50 (t, J = 7.1 Hz, 3 H), 1.64-1.75 (m, 4 H), 1.95-2.05 (m, 2 H), 2.23-2.33 (m, 2 H), 2.39 (t, J = 8.1 Hz, 2 H), 2.96 (m, 2 H), 3.35 (t, J = 7.0 Hz, 2 H), 3.75 (s, 2 H), 3.85 (t, J = 4.7 Hz, 4 H), 3.98-4.07 (m, 1 H), 4.32-4.45 (m, 6 H), 6.95 (m, 1 H), 7.05 (dd, J = 11.0, 8.8 Hz, 1 H), 7.28 (m, 1 H), 7.35 (m, 1 H) and 8.22 (bs, 1 H). This compound is not covered by the claims.

### Example 63 2-{1-[9-Ethyl-2-(5-fluoro-1H-indol-4-yl)-6-morpholin-4-yl-9H-purin-8-ylmethyl]-azetidin-3-ylamino}-2-methyl-propionamide

Prepared by using Suzuki coupling method B. The title compound was obtained as a colourless oil (45 mg, 56 %)
[M+H]⁺ 536.4
¹H NMR (400 MHz, CDCl₃): δ 1.29 (s, 6 H), 1.45 (t, J = 7.2 Hz, 3 H), 2.99 (m, 2 H), 3.52 (m, 1 H), 3.79 (t, J = 7.0 Hz, 2 H), 3.85 (t, J = 4.8 Hz, 4 H), 3.88 (s, 2 H), 4.29-4.38 (m, 6 H), 5.38 (m, 1 H), 6.89-6.95 (m, 1 H), 6.97 (m, 1 H), 7.03 (dd, J = 11.0, 8.8 Hz, 1 H), 7.26 (t, J = 2.9 Hz, 1 H), 7.33 (m, 1 H) and 8.38 (bs, 1 H). This compound is not covered by the claims.

### Example 64 2-{1-[9-Ethyl-2-(1H-indol-4-yl)-6-morpholin-4-yl-9H-purin-8-ylmethyl]-azetidin-3-ylamino}-2-methyl-propionamide

Prepared by using Suzuki coupling method B. The title compound was obtained as a pale yellow oil (26 mg, 36 %)
[M+H]⁺ 518.3
¹H NMR (400 MHz, CDCl₃): δ 1.28 (s, 6 H), 1.51 (t, J = 7.2 Hz, 3 H), 2.86 (m, 2 H), 3.48 (m, 1 H), 3.69-3.77 (m, 2 H), 3.82 (s, 2 H), 3.89 (t, J = 4.7 Hz, 4 H), 4.36-4.44 (m, 6 H), 5.28 (bs, 1 H), 6.99 (bs, 1 H), 7.26-7.33 (m, 2 H), 7.46 (d, J = 8.0 Hz, 1 H), 7.59 (m, 1 H), 8.22 (dd, J = 8.5, 0.9 Hz, 1 H) and 8.32 (bs, 1 H). This compound is not covered by the claims.

### Example 65 2-{(S)-1-[9-Ethyl-2-(5-fluoro-1H-indol-4-yl)-6-morpholin-4-yl-9H-purin-8-ylmethyl]-pyrrolidin-3-ylamino}-2-methyl-propionamide

Prepared by using Suzuki coupling method B. The title compound was obtained as a pale yellow solid (31 mg, 50 %)
[M+H]⁺ 550.3
¹H NMR (400 MHz, CDCl₃): δ 1.31 (s, 3 H), 1.32 (s, 3 H), 1.47 (t, J = 7.2 Hz, 3 H), 1.48-1.60 (m, 2H), 2.17-2.28 (m, 1 H), 2.41 (dd, J = 9.3, 5.4 Hz, 1 H), 2.55-2.64 (m, 1 H), 2.72 (td, J = 8.8, 5.4 Hz, 1 H), 2.85 (dd, J = 9.3, 6.8 Hz, 1 H), 3.31 (m, 1 H), 3.79-3.91 (m, 6 H), 4.38 (m, 6 H), 5.29 (bs, 1 H), 6.94 (m, 1 H), 6.97-7.06 (m, 1 H), 7.14 (bs, 1 H), 7.26 (m, 1 H), 7.33 (m, 1 H) and 8.35 (bs, 1 H). This compound is not covered by the claims.

### Example 66 2-({1-[9-Ethyl-2-(5-fluoro-1H-indol-4-yl)-6-morpholin-4-yl-9H-purin-8-ylmethyl]-azetidin-3-yl}-methyl-amino)-2-methyl-propionamide

Prepared by using Suzuki coupling method B. The title compound was obtained as an off-white solid (57 mg, 31 %)
[M+H]⁺ 550.2
¹H NMR (400 MHz, CDCl₃): δ 1.18 (s, 6 H), 1.48 (t, J = 7.2 Hz, 3 H), 2.21 (s, 3 H), 3.17 (m, 2 H), 3.47-3.55 (m, 3 H), 3.85 (m, 6 H), 4.33-4.42 (m, 6 H), 5.23 (m, 1 H), 6.94 (m, 1 H), 6.99-7.07 (m, 2 H), 7.27 (t, J = 2.8 Hz, 1 H), 7.35 (m, 1 H) and 8.25 (bs, 1 H). This compound is not covered by the claims.

### Example 67 2-{4-[2-(5-Fluoro-1H-indol-4-yl)-9-methyl-6-morpholin-4-yl-9H-purin-8-ylmethyl]-piperazin-1-yl)-isobutyramide

Prepared by using Suzuki coupling method C. The title compound was obtained as a white solid (189 mg, 81 %).
[M+H]⁺ 536.1
¹H NMR (400 MHz, CDCl₃): δ 1.22 (s, 6 H), 2.56 (m, 8 H), 3.77 (s, 2 H), 3.85 (t, J = 4.8 Hz, 4 H), 3.90 (s, 3 H), 4.36 (m, 4 H), 5.19 (d, J = 5.3 Hz, 1 H), 6.96 (m, 1 H), 7.05 (dd, J = 11.0, 8.7 Hz, 1 H), 7.11 (d, J = 5.4 Hz, 1 H), 7.29 (m, 1 H), 7.35 (ddd, J = 8.9, 4.2, 0.9 Hz, 1 H) and 8.22 (bs, 1 H). This compound is not covered by the claims.

### Example 68 2-{4-[2-(1H-Indol-4-yl)-9-methyl-6-morpholin-4-yl-9H-purin-8-ylmethyl]-piperazin-1-yl}-isobutyramide

Prepared by using Suzuki coupling method C. The title compound was obtained as a white solid (75 mg, 70 %).
[M+H]⁺ 518.2
¹H NMR (400 MHz, DMSO-d₆): δ 1.24 (s, 6 H), 2.57 (m, 8 H), 3.77 (s, 2 H), 3.89 (t, J = 4.7 Hz, 4 H), 3.95 (s, 3 H), 4.40 (t, J = 4.7 Hz, 4 H), 5.19 (d, J = 5.2 Hz, 1 H), 7.12 (m, 1 H), 7.27-7.34 (m, 2 H), 7.48 (d, J = 8.0 Hz, 1 H), 7.62 (t, J = 2.5 Hz, 1 H), 8.23 (dd, J = 7.5, 1.0 Hz, 1 H) and 8.26 (bs, 1 H). This compound is not covered by the claims.

### Example 69 (R)-8-[2-(1H-Indol-4-yl)-6-morpholin-4-yl-9H-purin-8-ylmethyl]-octahydro-pyrazino[2,1-c][1,4]oxazine

Prepared by using Suzuki coupling method C. The title compound was obtained as a white solid. (35 mg, 29 %).
[M+H]⁺ 475.1
¹H NMR (400 MHz, DMSO-d₆): δ 1.80 (t, J = 10.4 Hz, 1 H), 2.14-2.36 (m, 3 H), 2.56-2.64 (m, 2 H), 2.66 (d, J = 9.0 Hz, 1 H), 2.78 (d, J = 9.0 Hz, 1 H), 3.06 (t, J = 10.4 Hz, 1 H), 3.47 (td, J = 11.4, 2.4 Hz, 1 H), 3.56-3.64 (m, 1 H), 3.66 (d, J = 4.5 Hz, 2 H), 3.71 (d, J = 10.4 Hz, 2 H), 3.79 (t, J = 4.6 Hz, 4 H), 4.28 (m, 4 H), 7.17 (t, J = 7.7 Hz, 1 H), 7.39-7.48 (m, 3 H), 8.05 (dd, J = 8.5, 1.1 Hz, 1 H), 11.16 (s, 1 H) and 12.91 (bs, 1 H). This compound is not covered by the claims.

### Example 70 2-{4-[2-(5-Fluoro-1H-indol-4-yl)-6-morpholin-4-yl-9H-purin-8-ylmethyl]-piperazin-1-yl}-isobutyramide

Prepared by using Suzuki coupling method A followed by THP-deprotection. The title compound was obtained as a white solid (17 mg, 24 %).
[M + H]⁺ 522.13
¹H NMR (400 MHz, DMSO-d₆): δ 1.07 (s, 6 H), 2.45 (m, 4 H), 2.52 (m, 4 H), 3.68 (s, 2 H), 3.74 (t, J = 4.6 Hz, 4 H), 4.22 (m, 4 H), 6.68 (t, J = 2.4 Hz, 1 H), 6.92 (d, J = 3.5 Hz, 1 H), 6.98 (dd, J = 11.1, 8.7 Hz, 1 H), 7.05 (d, J = 3.5 Hz, 1 H), 7.39-7.44 (m, 2 H), 11.20 (s, 1 H) and 13.00 (bs, 1 H). This compound is not covered by the claims.

### Example 71 2-{4-[2-(1H-Indol-4-yl)-6-morpholin-4-yl-9H-purin-8-ylmethyl]-piperazin-1-yl}-isobutyramide

Prepared by using Suzuki coupling method B followed by THP-deprotection. The title compound was obtained as a white solid (16 mg, 15 %).
[M + H]⁺ 408.2
¹H NMR (400 MHz, DMSO-d₆): δ 1.06 (s, 6 H), 2.46 (m, 4 H), 2.53 (m, 4 H), 3.68 (s, 2 H), 3.73-3.82 (m, 4 H), 4.29 (m, 4 H), 6.93 (d, J = 3.5 Hz, 1 H), 7.06 (d, J = 3.5 Hz, 1 H), 7.17 (t, J = 7.7 Hz, 1 H), 7.40-7.45 (m, 2 H), 7.47 (d, J = 8.0 Hz, 1 H), 8.05 (d, J = 7.5 Hz, 1 H), 11.16 (s, 1 H) and 12.89 (bs, 1 H). This compound is not covered by the claims.

### Example 72 2-({1-[9-Ethyl-2-(5-fluoro-1H-indol-4-yl)-6-morpholin-4-yl-9H-purin-8-ylmethyl]-azetidin-3-yl}-methyl-amino)-2-methyl-propionamide

2-{[1-(2-Chloro-9-ethyl-6-morpholin-4-yl-9*H*-purin-8-ylmethyl)-azetidin-3-yl]-methyl-amino}-2-methyl-propionamide, was reacted under Suzuki coupling conditions (Method B). The crude reaction mixture was loaded onto an Isolute® SCX-2 cartridge, washed with MeOH then eluted with 2 M NH₃ in MeOH. The resulting residue was then purified by column chromatography to give the desired product as an off-white solid (57 mg, 31 %).
[M+H]⁺ 550.2
¹H NMR (400 MHz, CDCl₃): δ 1.17 (s, 6 H), 1.48 (t, J = 7.2 Hz, 3 H), 2.21 (s, 3 H), 3.17 (t, J = 5.6 Hz, 2 H), 3.47-3.55 (m, 3 H), 3.85 (m, 6 H), 4.33-4.42 (m, 6 H), 5.23 (d, J = 5.0 Hz, 1 H), 6.94 (m, 1 H), 7.04 (m, 2 H), 7.27 (t, J = 2.8 Hz, 1 H), 7.35 (m, 1 H) and 8.25 (bs, 1 H). This compound is not covered by the claims.

### Example 73 2-({1-[9-Ethyl-2-(1H-indol-4-yl)-6-morpholin-4-yl-9H-purin-8-ylmethyl]-azetidin-3-yl}-methyl-amino)-2-methyl-propionamide

2-{[1-(2-Chloro-9-ethyl-6-morpholin-4-yl-9*H*-purin-8-ylmethyl)-azetidin-3-yl]-methyl-amino}-2-methyl-propionamide, was reacted under Suzuki coupling conditions (Method B). The reaction mixture was loaded onto an Isolute® SCX-2 cartridge, washed with MeOH then eluted with 2 M NH₃ in MeOH. The resulting residue was then purified by column chromatography to give the desired product as an off-white solid (145 mg, 86 %).
[M+H]⁺ 532.2
¹H NMR (400 MHz, CDCl₃): 8 1.18 (s, 6 H), 1.53 (t, J = 7.2 Hz, 3 H), 2.20 (s, 3 H), 3.10-3.23 (m, 2 H), 3.45-3.58 (m, 3 H), 3.85 (s, 2 H), 3.89 (t, J = 4.8 Hz, 4 H), 4.37-4.46 (m, 6 H), 5.21 (d, J = 5.1 Hz, 1 H), 7.01 (t, J = 5.2 Hz, 1 H), 7.27-7.34 (m, 2 H), 7.44-7.49 (m, 1 H), 7.60 (m, 1 H), 8.22 (dd, J = 7.5, 1.0 Hz, 1 H) and 8.28 (bs, 1 H). This compound is not covered by the claims.

### Example 74 2-{4-[2-(5-Fluoro-1H-indol-4-yl)-9-{2-hydroxy-ethyl)-6-morpholin-4-yl-9H-purin-8-ylmethyl]-piperazin-1-yl}-isobutyramide

Prepared by Suzuki coupling Method B. The resulting residue was then purified by column chromatography to give the desired product as an off-white solid (60 mg, 51 %).
[M+H]⁺ 566.2
¹H NMR (400 MHz, CDCl₃): δ 1.23 (s, 6 H), 2.56 (m, 4 H), 2.65 (m, 4 H), 3.75 (s, 2 H), 3.85 (t, J = 4.7 Hz, 4 H), 4.02 (t, J = 4.3 Hz, 2 H), 4.37 (m, 4 H), 4.47 (t, J = 4.3 Hz, 2 H), 5.25-5.31 (m, 1 H), 6.07 (bs, 1 H), 6.91 (t, J = 2.5 Hz, 1 H), 6.98-7.09 (m, 2 H), 7.28 (t, J = 2.8 Hz, 1 H), 7.35 (dd, J = 8.8, 3.8 Hz, 1 H) and 8.28 (bs, 1 H). This compound is not covered by the claims.

### Example 75 {1-[2-(1H-Indol-4-yl)-6-morpholin-4-yl-9H-purin-8-ylmethyl]-piperidin-4-yl}-dimethyl-amine

Prepared from {1-[2-chloro-6-morpholin-4-yl-9-(tetrahydro-pyran-2-yl)-9*H*-purin-8-ylmethyl]piperidin-4-yl}-dimethyl-amine and 4-indole boronic acid pinacol ester instead of the boronic acid using Suzuki Method G to give {1-[2-(1*H*-indol-4-yl)-6-morpholin-4-yl-9-(tetrahydro-pyran-2-yl)-9H-purin-8-ylmethyl]-piperidin-4-yl}dimethyl-amine (106 mg).
[M + H]⁺ 545.3

This was stirred with 2M aqueous HCl (15 mL) and MeOH (1 mL) for 12 hours. The mixture was then diluted with CH₂Cl₂ and the pH of the aqueous layer adjusted to 10 with 1M aqeous Na₂CO₃. The aqueous layer was extracted with CH₂Cl₂ and the combined organic layers dried (MgSO₄). The solvent was evaporated and trituration of the residue in CH₂Cl₂-petrol gave the title compound as an off-white solid (30 mg).
¹H NMR (400 MHz, CDCl₃) 1.58 (m, 1H); 1.78 (m, 2H); 2.11 (m, 4H); 2.27 (s, 6H); 2.81 (m, 2H); 3.58 (s, 2H); 3.91 (m, 4H); 4.42 (m, 4H); 7.33 (m, 2H); 7.50 (m, 2H); 8.13 (d, 1H); 8.35 (brs, 1H)
[M + H]⁺ 461. This compound is not covered by the claims.

### Example 76 {1-[2-(5-Fluoro-1H-indol-4-yl)-6-morpholin-4-yl-9H-purin-8-ylmethyl]-piperidin-4-yl}-dimethyl-amine

Prepared from {1-[2-chloro-6-morpholin-4-yl-9-(tetrahydro-pyran-2-yl)-9*H*-purin-8-ylmethyl]piperidin-4-yl}-dimethyl-amine using Suzuki Method G to give {1-[2-(1H-Indol-4-yl)-6-morpholin-4-yl-9-(tetrahydro-pyran-2-yl)-9H-purin-8-ylmethyl]-piperidin-4-yl}-dimethyl-amine as a yellow oil (251 mg).
[M + H]⁺ 563

This was treated as described for {1-[2-(1*H*-indol-4-yl)-6-morpholin-4-yl-9*H*-purin-8-ylmethyl]-piperidin-4-yl}-dimethyl-amine followed by chromatography to give the title compound as a white solid (20 mg).
¹H NMR (400 MHz, CDCl₃) 1.75 (m, 4H); 2.00 (m, 3H); 2.28 (s, 6H); 2.77 (m, 2H); 3.43 (s, 2H); 3.85 (m, 4H); 4.35 (m, 4H); 6.89 (m, 1H); 7.08 (m, 1H); 7.22 (m, 1H); 7.39 (m, 1H); 8.65 (brs, 1H); 11.5 (brs, 1H).
[M + H]⁺ 480.3. This compound is not covered by the claims.

### Example 77 3-{1-[9-Ethyl-2-(5-fluoro-1H-indol-4-yl)-6-morpholin-4-yl-9H-purin-8-ylmethyl]-piperidin-4-yl}-oxazolidin-2-one

Prepared using Suzuki Method G to give the title compund as a white solid (53 mg).
¹H NMR (400 MHz, CDCl₃) 1.52 (m, 3H); 1.69 (m, 2H); 1.82 (m, 2H); 2.29 (m, 2H); 3.00 (m, 2H); 3.55 (m, 2H); 3.80 (m, 4H); 4.33-4.45 (m, 8H); 6.96 (m, 1H); 7.07 (m, 1H); 7.29 (m, 1H); 7.37 (m, 1H); 8.23 (br s, 1H)
[M + H]⁺ 549.2. This compound is not covered by the claims.

### Example 78 3-{1-[9-Ethyl-2-(1H-indol-4-yl)-6-morpholin-4-yl-9H-purin-8-ylmethyl]-piperidin-4-yl}-oxazolidin-2-one

Prepared using Suzuki Method G to give the title compund as a white solid (79 mg).
¹H NMR (400 MHz, CDCl₃) 1.57 (t, 3H); 1.66-1.83 (m, 4H); 2.27 (m, 2H); 3.00 (m, 2H); 3.53 (m, 2H); 3.76-3.84 (m, 3H); 3.92 (m, 4H); 4.33-4.49 (m, 8H); 7.34 (m, 2H); 7.51(m, 1H); 7.63 (m, 1H); 8.26 (d, 1H); 8.29 (br s, 1H).
[M + H]⁺ 531.2. This compound is not covered by the claims.

### Example 79 1-[9-Ethyl-2-(1H-indol-4-yl)-6-morpholin-4-yl-9H-purin-8-ylmethyl]-4-morpholin-4-yl-piperidine-4-carboxylic acid amide

The title compound was prepared using Suzuki coupling Method G to give an off-white foam (55 mg).
δ_{H}(400 MHz, CDCl₃) 1.56 (m, 3H), 1.89 (m, 4H), 2.60 (m, 6H), 2.83 (m, 2H), 3.72 (t, 4H), 3.78 (s, 2H), 3.91 (t, 4H), 4.42 (m, 4H), 4.45 (q, 2H), 5.25 (br s, 1H), 6.42 (br s, 1H), 7.32 (m, 2H), 7.50 (d, 1H), 7.63 (m, 1H), 8.25 (m, 2H).
[M + H]⁺ 574.25. This compound is not covered by the claims.

### Example 80 1-[9-Ethyl-2-(5-fluoro-1H-indol-4-yl)-6-morpholin-4-yl-9H-purin-8-ylmethyl]-4-morpholin-4-yl-piperidine-4-carboxylic acid

The title compound was prepared using Suzuki coupling Method G to give an off-white solid (41 mg).
δ_{H}(400 MHz, CDCl₃) 1.52 (t, 3H), 1.88 (t, 4H), 2.57 (m, 2H), 2.61 (t, 4H), 2.83 (m, 2H), 3.49 (t, 4H), 3.78 (s, 2H), 3.87 (t, 4H), 4.38 (m, 4H), 4.42 (q, 2H), 5.25 (br s, 1H), 6.43 (br s, 1H), 6.97 (m, 1H), 7.07 (m, 1H), 7.27 (m, 1H), 7.36 (dd, 1H), 8.22 (br s, 1H).
[M + H]⁺ 592.21. This compound is not covered by the claims.

### Example 81 N-{1-[9-Ethyl-2-(1H-indol-4-yl)-6-morpholin-4-yl-9H-purin-8-ylmethyl]-piperidin-4-yl}-N-methyl-methanesulfonamide

The title compound was prepared using Suzuki coupling Method G to give a white solid (35 mg).
δ_{H} (400 MHz, CDCl₃) 1.57 (m, 3H), 1.77 (m, 4H), 2.28 (t, 2H), 2.82 (s, 3H), 2.86 (s, 3H), 3.01 (m, 2H), 3.77 (s, 2H), 3.81 (m, 1 H), 3.91 (t, 4H), 4.42 (m, 4H), 4.40 (q, 2H), 7.32 (m, 2H), 7.50 (d, 1H), 7.63 (m, 1H), 8.26 (m, 2H).
[M + H]⁺ 553.21. This compound is not covered by the claims.

### Example 82 N-{1-[9-Ethyl-2-(5-fluoro-1H-indol-4-yl)-6-morpholin-4-yl-9H-purin-8-ylmethyl]-piperidin-4-yl}-N-methyl-methanesulfonamide

The title compound was prepared using Suzuki coupling Method G to give a white solid (94 mg).
δ_{H} (400 MHz, CDCl₃) 1.54 (t, 3H), 1.78 (m, 4H), 2.28 (t, 2H), 2.82 (s, 3H), 2.86 (s, 3H), 3.01 (m, 2H), 3.77 (s, 2H), 3.81 (m, 1H), 3.87 (t, 4H), 4.41 (m, 6H), 6.97 (m, 1H), 7.07 (m, 1H), 7.30 (m, 1H), 7.37 (m, 1H), 8.21 (br s, 1H).
[M + H]⁺ 571.81. This compound is not covered by the claims.

### Biological evaluation and Pharmaceutical formulations

### Example 83 Biological Testing

Compounds of the invention, prepared as described in the preceding Examples, were submitted to the following biological assay:

### (i) PI3K Biochemical Screening

Compound inhibition of PI3K was determined in a radiometric assay using purified, recombinant enzyme and ATP at a concentration of luM. All compounds were serially diluted in 100% DMSO. The kinase reaction was incubated for 1 hour at room temperature, and the reaction was terminated by the addition of PBS. IC₅₀ values were subsequently determined using sigmoidal dose-response curve fit (variable slope). All of the compounds tested had an IC₅₀ against PI3K of 50µM or less. Typically the IC₅₀ against the p110δ isoform of PI3K was less than 500nM.

### Example 84 Tablet composition

Tablets, each weighing 0.15 g and containing 25 mg of a compound of the invention were manufactured as follows:
Composition for 10,000 tablets
Compound of the invention (250 g)
Lactose (800 g)
Corn starch (415g)
Talc powder (30 g)
Magnesium stearate (5 g)

The compound of the invention, lactose and half of the corn starch were mixed. The mixture was then forced through a sieve 0.5 mm mesh size. Corn starch (10 g) is suspended in warm water (90 ml). The resulting paste was used to granulate the powder. The granulate was dried and broken up into small fragments on a sieve of 1.4 mm mesh size. The remaining quantity of starch, talc and magnesium was added, carefully mixed and processed into tablets.

### Example 85 Injectable Formulation

| | |
|---|---|
| Compound of the invention | 200mg |
| Hydrochloric Acid Solution 0.1M or | |
| Sodium Hydroxide Solution 0.1M q.s. to pH | 4.0 to 7.0 |
| Sterile water q.s. to | 10 ml |

The compound of the invention was dissolved in most of the water (35°-40°C) and the pH adjusted to between 4.0 and 7.0 with the hydrochloric acid or the sodium hydroxide as appropriate. The batch was then made up to volume with water and filtered through a sterile micropore filter into a sterile 10 ml amber glass vial (type 1) and sealed with sterile closures and overseals.

### Example 86 Intramuscular Injection

| | |
|---|---|
| Compound of the invention | 200 mg |
| Benzyl Alcohol | 0.10 g |
| Glycofurol 75 | 1.45 g |
| Water for injection q.s to | 3.00 ml |

The compound of the invention was dissolved in the glycofurol. The benzyl alcohol was then added and dissolved, and water added to 3 ml. The mixture was then filtered through a sterile micropore filter and sealed in sterile 3 ml glass vials (type 1).

### Example 87 Syrup Formulation

| | |
|---|---|
| Compound of invention | 250 mg |
| Sorbitol Solution | 1.50 g |
| Glycerol | 2.00 g |
| Sodium benzoate | 0.005 g |
| Flavour | 0.0125 ml |
| Purified Water q.s. to | 5.00 ml |

The compound of the invention was dissolved in a mixture of the glycerol and most of the purified water. An aqueous solution of the sodium benzoate was then added to the solution, followed by addition of the sorbital solution and finally the flavour. The volume was made up with purified water and mixed well.

## Claims

1. A compound which is a purine of formula (Ia) or (Ib): 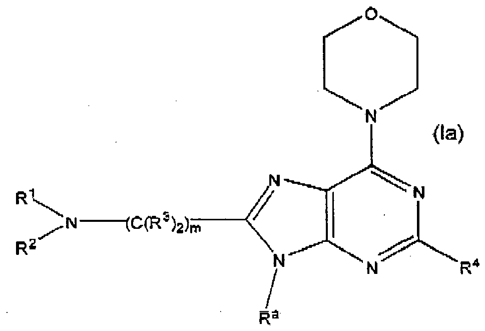 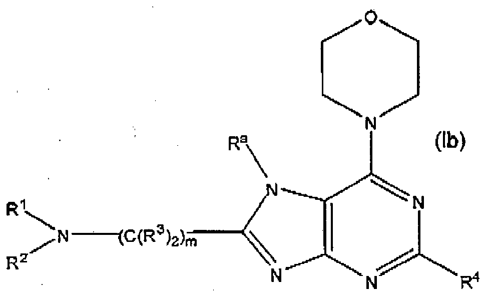 wherein
R¹ and R² form, together with the N atom to which they are attached, a group of the following formula (IIa): 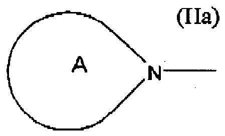
in which A is a group of formula (IIb): 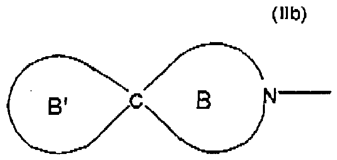
wherein
ring B is a 4- to 7-membered saturated N-containing heterocyclic ring which includes 0 or 1 additional heteroatoms selected from N, S and O and
ring B' is a 3- to 12- membered saturated carbocyclic ring, a 5- to 7-membered saturated O-containing heterocyclic ring or a 4- to 7-membered saturated N-containing heterocyclic ring as defined above, each of B and B' being unsubstituted or substituted;
m is 0, 1 or 2;
R³ is H or C₁-C₆ alkyl;
R^{a} is selected from R, C(O)OR, C(O)NR₂, halo(C₁-C₆)alkyl, SO₂R, SO₂NR₂,, wherein each R is independently H or C₁-C₆ alkyl which is unsubstituted or substituted; and
R⁴ is an indole group which is unsubstituted or substituted;
or a pharmaceutically acceptable salt thereof.

2. A compound according to claim 1 wherein R⁴ is an indole group which is unsubstituted or substituted by a group selected from CN, halo, -C(O)NR₂, halo(C₁-C₆)alkyl, -SO₂R, -SO₂NR₂, and a 5-membered heteroaryl group containing 1, 2, 3 or 4 heteroatoms selected from O, N and S, wherein R is H or C₁-C₆ alkyl.

3. A compound according to claim 1 which is selected from:
8-[9-Ethyl-2-(5-fluoro-1*H*-indol-4-yl)-6-morpholin-4-yl-9*H*-purin-8-ylmethyl]-2,8-diaza-spiro[4.5]decan-3-one;
8-[9-Ethyl-2-(1*H*-indol-4-yl)-6-morpholin-4-yl-9*H*-purin-8-ylmethyl]-2,8-diaza-spiro [4.5]decan-3-one;
9-[9-Ethyl-2-(5-fluoro-1*H*-indol-4-yl)-6-morpholin-4-yl-9*H*-purin-8-ylmethyl]-1-oxa-4,9-diaza-spiro[5.5]undecan-3-one;
9-[9-Ethyl-2-(1*H*-indol-4-yl)-6-morpholin-4-yl-9*H*-purin-8-ylmethyl]-1-oxa-4,9-diaza-spiro[5.5]undecan-3-one;
8-[9-Ethyl-2-(5-fluoro-1*H*-indol-4-yl)-6-morpholin-4-yl-9*H*-purin-8-ylmethyl]-2,8-diaza-spiro[4.5]decan-1-one;
8-[9-Ethyl-2-(1*H*-indol-4-yl)-6-morpholin-4-yl-9*H*-purin-8-ylmethyl]-2,8-diaza-spiro[4.5]decan-1-one;
and the pharmaceutically acceptable salts thereof.

4. A pharmaceutical composition which comprises a pharmaceutically acceptable carrier or diluent and, as an active ingredient, a compound as defined in any one of claims 1 to 3.

5. A compound as defined in any one of claims 1 to 3 for use in a method of medical treatment of the human or animal body by therapy.

6. A compound as defined in any one of claims 1 to 3 for treating a disease or disorder arising from abnormal cell growth, function or behaviour associated with PI3 kinase.

7. A compound as claimed in claim 6 wherein the disease or disorder is selected from cancer, immune disorders, cardiovascular disease, viral infection, inflammation, metabolism/endocrine function disorders and neurological disorders.

8. Use of a compound as defined in any one of claims 1 to 3 in the manufacture of a medicament for treating a disease or disorder arising from abnormal cell growth, function or behaviour associated with PI3 kinase.

9. Use according to claim 8 wherein the medicament is for treating cancer, immune disorders, cardiovascular disease, viral infection, inflammation, metabolism/endocrine function disorders and neurological disorders.

## Patentansprüche

1. Eine Verbindung, welche ein Purin der Formel (Ia) oder (Ib) ist: 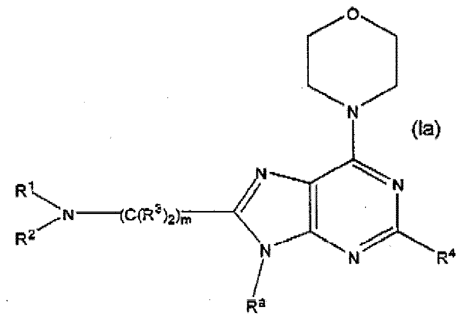 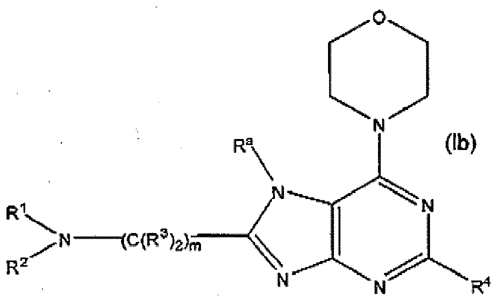 wobei
R¹ und R² zusammen mit dem N-Atom, an das sie gebunden sind, einen Rest der folgenden Formel (IIa): 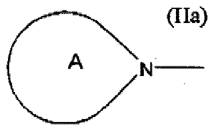
bilden, in dem A ein Rest der Formel (IIb): 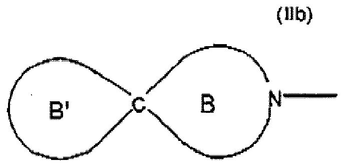
ist, wobei
Ring B ein 4- bis 7-gliedriger, gesättigter N-enthaltender, heterocyclischer Ring ist, der 0 oder 1 zusätzliche(s) Heteroatom(e), ausgewählt aus N, S und O, enthält und
Ring B' ein 3- bis 12-gliedriger, gesättigter, carbocyclischer Ring, ein 5- bis 7-gliedriger, gesättigter O-enthaltender, heterocyclischer Ring oder ein 4- bis 7-gliedriger, gesättigter N-enthaltender, heterocyclischer Ring wie vorstehend definiert ist, wobei jeder aus B und B' unsubstituiert oder substituiert ist;
m gleich 0, 1 oder 2 ist;
R³ gleich H oder C₁-C₆-Alkyl ist;
R^{a} ausgewählt ist aus R, C(O)OR, C(O)NR₂, Halogen(C₁-C₆)alkyl, SO₂R, SO₂NR₂, wobei jedes R unabhängig H oder C₁-C₆-Alkyl ist, welches unsubstituiert oder substituiert ist; und
R⁴ ein Indolrest ist, welcher unsubstituiert oder substituiert ist;
oder ein pharmazeutisch verträgliches Salz davon.

2. Eine Verbindung nach Anspruch 1, wobei R⁴ ein Indolrest ist, welcher unsubstituiert oder mit einem Rest, ausgewählt aus CN, Halogen, -C(O)NR₂, Halogen(C₁-C₆)alkyl, -SO₂R, -SO₂NR₂ und einem 5-gliedrigen Heteroarylrest, enthaltend 1, 2, 3 oder 4 Heteroatome, ausgewählt aus O, N und S, wobei R gleich H oder C₁-C₆-Alkyl ist, substituiert ist.

3. Eine Verbindung nach Anspruch 1, welche ausgewählt ist aus:
8-[9-Ethyl-2-(5-fluor-1*H*-indol-4-yl)-6-morpholin-4-yl-9*H*-purin-8-ylmethyl]-2,8-diaza-spiro[4.5]decan-3-on;
8-[9-Ethyl-2-(1*H*-indol-4-yl)-6-morpholin-4-yl-9*H*-purin-8-ylmethyl]-2,8-diaza-spiro[4.5]decan-3-on;
9-[9-Ethyl-2-(5-fluor-1*H*-indol-4-yl)-6-morpholin-4-yl-9*H*-purin-8-ylmethyl]-1-oxa-4,9-diaza-spiro[5.5]undecan-3-on;
9-[9-Ethyl-2-(1*H*-indol-4-yl)-6-morpholin-4-yl-9*H*-purin-8-ylmethyl]-1-oxa-4,9-diaza-spiro[5.5]undecan-3-on;
8-[9-Ethyl-2-(5-fluor-1*H*-indol-4-yl)-6-morpholin-4-yl-9*H*-purin-8-ylmethyl]-2,8-diaza-spiro[4.5]decan-1-on;
8-[9-Ethyl-2-(1*H*-indol-4-yl)-6-morpholin-4-yl-9*H*-purin-8-ylmethyl]-2,8-diaza-spiro[4.5]decan-1-on:
und den pharmazeutisch verträglichen Salzen davon.

4. Ein Arzneimittel, welches einen pharmazeutisch verträglichen Träger oder ein pharmazeutisch verträgliches Verdünnungsmittel und, als einen Wirkstoff, eine Verbindung wie in einem der Ansprüche 1 bis 3 definiert umfasst.

5. Eine Verbindung wie in einem der Ansprüche 1 bis 3 definiert zur Verwendung bei einem Verfahren der medizinischen Behandlung des menschlichen oder tierischen Körpers durch Therapie.

6. Eine Verbindung wie in einem der Ansprüche 1 bis 3 definiert zur Behandlung einer Erkrankung oder Störung, welche aufgrund von anormalem Zellwachstum, anormaler Funktion oder Verhaltensweise, die mit PI3-Kinase in Zusammenhang stehen, entsteht.

7. Eine Verbindung wie in Anspruch 6 beansprucht, wobei die Erkrankung oder Störung aus Krebs, Immunstörungen, kardiovaskulärer Erkrankung, Virusinfektion, Entzündung, Stoffwechsel-/endokrinen Funktionsstörungen und neurologischen Störungen ausgewählt ist.

8. Verwendung einer Verbindung wie in einem der Ansprüche 1 bis 3 definiert bei der Herstellung eines Medikaments zur Behandlung einer Erkrankung oder Störung, welche aufgrund von anormalem Zellwachstum, anormaler Funktion oder Verhaltensweise, die mit PI3-Kinase in Zusammenhang stehen, entsteht.

9. Verwendung nach Anspruch 8, wobei das Medikament zur Behandlung von Krebs, Immunstörungen, kardiovaskulärer Erkrankung, Virusinfektion, Entzündung, Stoffwechsel-/endokrinen Funktionsstörungen und neurologischen Störungen bestimmt ist.

## Revendications

1. Composé qui est une purine de formule (Ia) ou (Ib) : 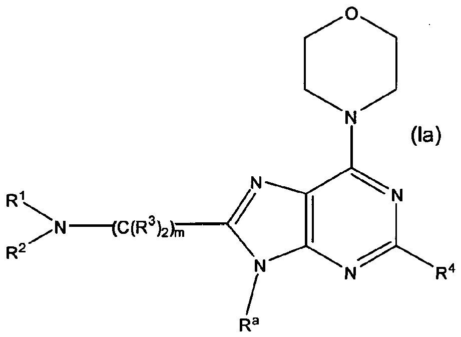 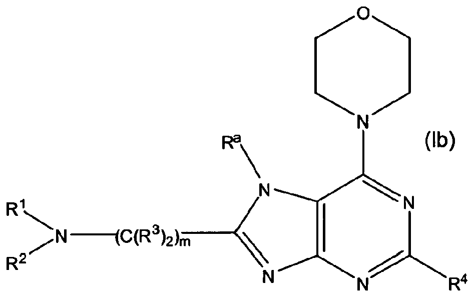 formules dans lesquelles :
R¹ et R² forment, conjointement avec l'atome de N auquel ils sont fixés, un groupe répondant à la formule (IIa) suivante : 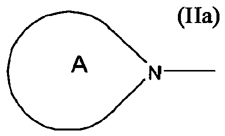
dans laquelle A représente un groupe de formule (IIb) : 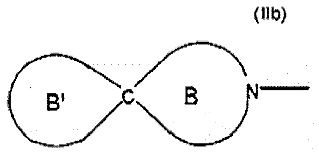
dans laquelle :
le noyau B est un noyau hétérocyclique contenant N saturé tétra- à heptagonal qui comprend 0 ou 1 hétéroatome supplémentaire choisi entre N, S et 0, et
le noyau B' est un noyau carbocyclique saturé tri- à dodécagonal, un noyau hétérocyclique contenant 0 saturé penta- à heptagonal ou un noyau hétérocyclique contenant N saturé tétra- à heptagonal répondant à la définition précitée, chacun de B et B' étant non substitué ou substitué ;
m est égal à 0, 1 ou 2 ;
R³ représente H ou un groupe alkyle en C₁ à C₆ ;
R^{a} est choisi entre des groupes R, C(O)OR, C(O)NR₂, halogéno-alkyle en C₁ à C₆, SO₂R, SO₂NR₂, dans lesquels chaque groupe R représente indépendamment H ou un groupe alkyle C₁ à C₆ qui est non substitué ou substitué ; et
R⁴ représente un groupe indole qui est non substitué ou substitué ;
ou un de ses sels pharmaceutiquement acceptables.

2. Composé suivant la revendication 1, dans lequel R⁴ représente un groupe indole qui est non substitué ou substitué avec un groupe choisi entre des groupes CN, halogéno, -C(O)NR₂, halogénoalkyle en C₁ à C₆, -SO₂R, -SO₂NR₂, et un groupe hétéroaryle pentagonal contenant 1, 2, 3 ou 4 hétéroatomes choisis entre 0, N et S, dans lesquels R représente H ou un groupe alkyle en C₁ à C₆.

3. Composé suivant la revendication 1, qui est choisi entre :
la 8-[9-éthyl-2-(5-fluoro-1*H*-indole-4-yl)-6-morpholine-4-yl-9*H*-purine-8-ylméthyl]-2,8-diazaspiro[4.5]décane-3-one ;
la 8-[9-éthyl-2-(1*H*-indole-4-yl)-6-morpholine-4-yl-9*H*-purine-8-ylméthyl]-2,8-diazaspiro[4.5]décane-3-one ;
la 9-[9-éthyl-2-(5-fluoro-1*H*-indole-4-yl)-6-morpholine-4-yl-9*H*-purine-8-ylméthyl]-1-oxa-4,9-diazaspiro[5.5]undécane-3-one ;
la 9-[9-éthyl-2-(1H-indole-4-yl)-6-morpholine-4-yl-9H-purine-8-ylméthyl]-1-oxa-4,9-diazaspiro[5.5]undécane-3-one ;
la 8-[9-éthyl-2-(5-fluoro-1*H*-indole-4-yl)-6-morpholine-4-yl-9*H*-purine-8-ylméthyl]-2,8-diazaspiro[4.5]décane-1-one ;
la 8-[9-éthyl-2-(1*H*-indole-4-yl)-6-morpholine-4-yl-9*H*-purine-8-ylméthyl]-2,8-diazaspiro[4.5]décane-3-one ;
et ses sels pharmaceutiquement acceptables.

4. Composition pharmaceutique qui comprend un support ou diluant pharmaceutiquement acceptable et, comme ingrédient actif, un composé tel que défini dans l'une quelconque des revendications 1 à 3.

5. Composé tel que défini dans l'une quelconque des revendications 1 à 3, pour une utilisation dans un procédé de traitement médical de l'organisme humain ou animal par thérapie.

6. Composé tel que défini dans l'une quelconque des revendications 1 à 3, pour traiter une maladie ou un trouble provenant d'une croissance, d'une fonction ou d'un comportement cellulaire, anormal, associé à la kinase PI3.

7. Composé suivant la revendication 6, la maladie ou le trouble étant choisi entre le cancer, des troubles immunitaires, une maladie cardiovasculaire, une infection virale, une inflammation, des troubles de la fonction métabolique/endocrinienne et des troubles neurologiques.

8. Utilisation d'un composé tel que défini dans l'une quelconque des revendications 1 à 3, dans la production d'un médicament pour traiter une maladie ou un trouble provenant d'une croissance, d'une fonction ou d'un comportement cellulaire, anormal, associé à la kinase PI3.

9. Utilisation suivant la revendication 8, dans laquelle le médicament sert à traiter le cancer, des troubles immunitaires, une maladie cardiovasculaire, une infection virale, une inflammation, des troubles de la fonction métabolique/endocrinienne et des troubles neurologiques.
